# EUROPEAN PATENT APPLICATION

(11) **EP 2 641 901 A1**
(43) Date of publication of application: **25.09.2013**
(21) Application number: 12160780.8
(22) Date of filing: 22.03.2012
(51) Int. Cl.: C07D 215/40, C07D 401/12, A61K 31/4709, A01N 43/42

(54) **Novel microbiocides**

(71) Applicant: Syngenta Participations AG, 4058 Basel (CH)
(72) Inventor: The designation of the inventor has not yet been filed
(74) Representative: Herrmann, Jörg

(57) **Abstract**

The invention relates to compounds of formula I 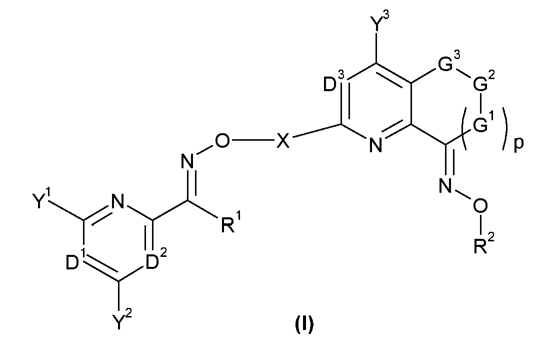 wherein R¹, R², X, Y¹, Y², Y³, D¹, D², D³, G¹, G², G³ and p are as defined in the claims. The invention further provides intermediates used in the preparation of these compounds, to compositions which comprise these compounds and to their use in agriculture or horticulture for controlling or preventing infestation of plants by phytopathogenic microorganisms, preferably fungi.

## Description

The present invention relates to novel microbiocidally active, in particular fungicidally active, cyclic bisoxime derivatives. It further relates to intermediates used in the preparation of these compounds, to compositions which comprise these compounds and to their use in agriculture or horticulture for controlling or preventing infestation of plants by phytopathogenic microorganisms, preferably fungi.

Fungicidally active bisoximes are described in WO08074418.

Surprisingly, it has been found that novel cyclic bisoxime derivatives have microbiocidal activity. The present invention accordingly relates to bisoxime derivatives of formula (I) 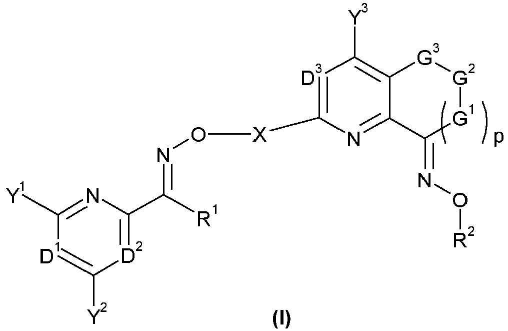 wherein
D¹ represents N or C-Y⁴;
D² represents N or C-Y⁵;
D³ represents N or C-Y⁶;
wherein both D¹ and D² cannot be N;
Y¹, Y², Y³, Y⁴, Y⁵ and Y⁶ independently of one another represent hydrogen, halogen, CN, NO₂, C₁-C₈ alkyl, C₁-C₄-alkoxy-C₁-C₄-alkyl, C₁-C₄-alkoxy-C₁-C₄-alkoxy-C₁-C₄-alkyl, C₃-C₈ cycloalkyl, C₂-C₈ alkenyl, C₂-C₈ alkynyl, phenyl, pyridyl, pyrimidinyl, OR³, COR⁴, SH, C₁-C₈ alkylthio, C₁-C₈ alkylsulphinyl, C₁-C₈ alkylsulphonyl, N(R⁵)₂, CO₂R³, O(CO)R⁴, CON(R⁵)₂, NR⁵COR⁴ or CR⁴N-OR³, wherein the alkyl, cycloalkyl, alkenyl, alkynyl, phenyl, pyrimidinyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from O, S, N and N(R⁵), providing that the heterocycle does not contain adjacent oxygen atoms, adjacent sulphur atoms, or adjacent sulphur and oxygen atoms, and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
R¹ represents hydrogen, halogen, CN, OH, SH, C₁-C₈ alkylthio, C₁-C₈ alkylsulphinyl, C₁-C₈ alkylsulphonyl, NH₂, C₁-C₁₀ alkyl, C₃-C₈ cycloalkyl, C₂-C₈ alkenyl, C₂-C₈ alkynyl, (R³O)carbonyl(C₁-C₄-alkyl), phenyl or pyridyl, wherein the alkyl, cycloalkyl, alkenyl, alkynyl, phenyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₃-C₆ cycloalkyl and a 5- or 6-membered heterocycle containing one to three heteroatoms independently selected from O, S and N, providing that the heterocycle does not contain adjacent oxygen atoms, adjacent sulphur atoms, or adjacent sulphur and oxygen atoms;
or if D² is C-Y⁵ then R¹ and Y⁵ together may be -(G⁴)_{q}-G⁵-G⁶-;
R² represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
each R³ independently of one another represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄-haloalkyl, C₁-C₄ alkoxy, C₁-C₄ haloalkoxy and C₁-C₄-alkoxy-C₁-C₄-alkyl;
each R⁴ independently of one another represents hydrogen, C₁-C₈ alkyl, C₃-C₈ cycloalkyl, C₂-C₈ alkenyl, C₂-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy.;
each R⁵ independently of one another represents hydrogen, OH, C₁-C₈ alkyl, C₁-C₈ alkoxy, C₁-C₈-alkoxy-C₁-C₄-alkyl, C₃-C₈ alkenyl, C₃-C₈ alkynyl, COR⁴ or phenyl wherein the alkyl, alkoxy, alkenyl, alkynyl and phenyl are optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkyoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5, B-6, B-7 or B-8: 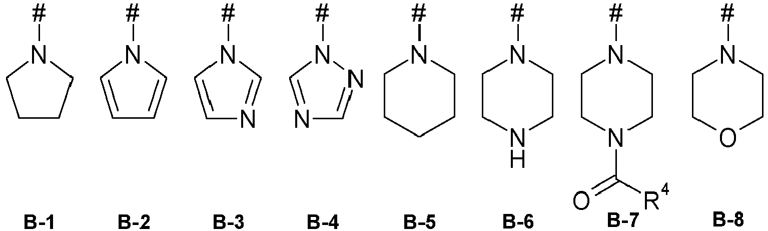 wherein the cycle formed is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
X represents X-2, X-3, X-4 or X-5: 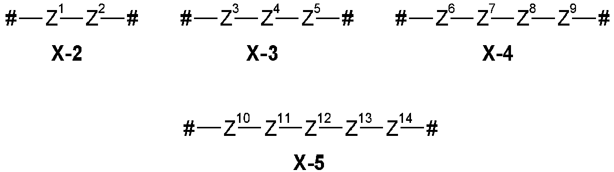
Z¹, Z², Z³, Z⁵, Z⁶, Z⁷, Z⁸, Z⁹, Z¹⁰, Z¹¹, Z¹³ and Z¹⁴ independently of one another represent CR⁶R⁷, C=CR⁸R⁹ or C=O;
Z⁴ and Z¹² independently of one another represent C=CR⁸R⁹, CR¹⁰R¹¹, SiR¹²R¹³ or C=O;
or in each case two adjacent radicals Z⁴ and Z⁵ or Z⁷ and Z⁸ or Z⁸ and Z⁹ or Z¹¹ and Z¹² or Z¹² and Z¹³ or Z¹³ and Z¹⁴ may together represent a group selected from CR¹⁴=CR¹⁵- and -C≡C-, wherein X-4 or X-5 may not contain more than one such group;
each R⁶ and R⁷ independently of one another represent hydrogen, halogen, OH, C₁-C₄ alkyl, C₁-C₄ haloalkyl, C₃-C₆ cycloalkyl, C₃-C₆ halocycloalkyl, C₁-C₄ alkylthio, C₁-C₄ alkylsulphinyl, C₁-C₄ alkylsulphonyl, phenyl or CN, wherein the phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R⁶ and R⁷ together with the carbon atom to which they are attached may form a C₃-C₆ cycloalkyl group or a C₃-C₆ halocycloalkyl group;
each R⁸ and R⁹ independently of one another represent hydrogen, halogen, C₁-C₄ alkyl or C₁-C₄ haloalkyl;
each R¹⁰, R¹¹, R¹² and R¹³ independently of one another represent hydrogen, halogen, OH, C₁-C₄ alkyl, C₁-C₄ haloalkyl, C₃-C₆ cycloalkyl, C₃-C₆ halocycloalkyl, phenyl or CN, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R¹⁰ and R¹¹ together with the carbon atom to which they are attached may form a C₃-C₆ cycloalkyl group or a C₃-C₆ halocycloalkyl group;
wherein the groupings X-2, X-3, X-4 and X-5 may contain at most one ring which contains either only one of the radicals Z¹ to Z¹⁴ or two radicals Z¹ to Z¹⁴ or three radicals Z¹ to Z¹⁴ or four radicals Z¹ to Z¹⁴ as ring members; and wherein radicals Z¹, Z², Z³, Z⁵, Z⁶, Z⁹, Z¹⁰ and Z¹⁴ are not substituted by OH;
G¹, G², G⁴ and G⁵ independently of one another represent -C(R¹⁶R¹⁷)-;
G³ and G⁶ independently of one another represent -C(R¹⁶R¹⁷)-, O, N(R¹⁸) or S;
or G¹ and G², or G² and G³, or G¹ and G¹, or G⁴ and G⁵, or G⁵ and G⁶, or G⁴ and G⁴ together represent -CR¹⁶=CR¹⁷-_{;}
each R¹⁶ and R¹⁷ independently of one another represent hydrogen, halogen, C₁-C₄ alkyl, C₁-C₄ haloalkyl, C₁-C₄ alkoxy or C₁-C₄ haloalkoxy;
R¹⁸ represents hydrogen, OH, C₁-C₄ alkyl, C₁-C₄ alkoxy, C₃-C₆ cycloalkyl, C₁-C₈ alkylcarbonyl or C₁-C₈ haloalkylcarbonyl;
p and q are each independently 1 or 2;
or a salt or an N-oxide thereof.

Halogen, either as a lone substituent or in combination with another substituent (e.g. haloalkyl) is generally fluorine, chlorine, bromine or iodine, and usually fluorine, chlorine or bromine.

Each alkyl moiety (including the alkyl moiety of alkoxy, alkylthio, etc.) is a straight or branched chain and, depending on the number of carbon atoms it contains, is, for example, methyl, ethyl, *n*-propyl, *n*-butyl, *n*-pentyl, *n*-hexyl, *iso*-propyl, *sec*-butyl, *iso*-butyl, *tert*-butyl, *neo*-pentyl, *n-*heptyl or 1,3-dimethylbutyl, and usually methyl or ethyl.

The alkenyl and alkynyl groups can be mono- or di-unsaturated and are examples thereof are derived from the above mentioned alkyl groups.

Haloalkyl moieties are alkyl moieties which are substituted by one or more of the same or different halogen atoms and are, for example, monofluoromethyl, difluoromethyl, trifluoromethyl, monochloromethyl, dichloromethyl, trichloromethyl, 2,2,2-trifluoroethyl, 2,2-difluoroethyl, 2-fluoroethyl, 1,1-difluoroethyl, 1-fluoroethyl, 2-chloroethyl, pentafluoroethyl, 1,1-difluoro-2,2,2-trichloroethyl, 2,2,3,3-tetrafluoroethyl and 2,2,2-trichloroethyl, and typically trichloromethyl, difluorochloromethyl, difluoromethyl, trifluoromethyl and dichlorofluoromethyl. Alkoxy is, for example, methoxy, ethoxy, propoxy, *iso*-propoxy, *n*-butoxy, *iso*-butoxy, *sec*-butoxy and *tert*-butoxy, and usually methoxy or ethoxy.

Haloalkoxy is, for example, fluoromethoxy, difluoromethoxy, trifluoromethoxy, 2,2,2-trifluoro-ethoxy, 1,1,2,2-tetrafluoroethoxy, 2-fluoroethoxy, 2-chloroethoxy, 2,2-difluoroethoxy and 2,2,2-trichloroethoxy, and usually difluoromethoxy, 2-chloroethoxy and trifluoromethoxy.

Alkylthio is, for example, methylthio, ethylthio, propylthio, *iso*-propylthio, *n*-butylthio, *iso*-butylthio, *sec*-butylthio or *tert*-butylthio, and usually methylthio or ethylthio.

Alkylsulphonyl is, for example, methylsulphonyl, ethylsulphonyl, propylsulphonyl, *iso*propylsulphonyl, *n*-butylsulphonyl, *iso*-butylsulphonyl, *sec*-butylsulphonyl or *tert*-butylsulphonyl, and usually methylsulphonyl or ethylsulphonyl.

Alkylsulphinyl is, for example, methylsulphinyl, ethylsulphinyl, propylsulphinyl, isopropylsulphinyl, *n*-butylsulphinyl, *iso*-butylsulphinyl, *sec*-butylsulphinyl or *tert*-butylsulphinyl, and usually methylsulphinyl or ethylsulphinyl Cycloalkyl may be saturated or partially unsaturated, preferably fully saturated, and is, for example, cyclopropyl, cyclobutyl, cyclopentyl or cyclohexyl.

Alkoxyalkyl is, for example, methoxymethyl, methoxyethyl, ethoxymethyl, ethoxyethyl, *n-*propoxymethyl, *n*-propoxyethyl, *iso*-propoxymethyl or *iso*-propoxyethyl.

Aryl includes phenyl, naphthyl, anthracyl, fluorenyl and indanyl, but is usually phenyl. Carbocycle includes cycloalkyl groups and aryl groups.

Heterocycloalkyl is a non-aromatic ring that may be saturated or partially unsaturated, preferably fully saturated, containing carbon atoms as ring members and at least one heteroatom selected from O, S and N as ring members. Examples include oxiranyl, oxetanyl, tetrahydrofuranyl, tetrahydropyranyl, 1,3-dioxolanyl, 1,4-dioxanyl, aziridinyl, azetidinyl, pyrrolidinyl, piperidinyl, oxazinanyl, morpholinyl, thiomorpholinyl, imidazolidinyl, pyrazolidinyl and piperazinyl, preferably morpholinyl, pyrrolidinyl, piperdinyl and piperazinyl, more preferably morpholinyl and pyrollidinyl.

Heteroaryl is, for example, a monovalent monocyclic or bicyclic aromatic hydrocarbon radical. Examples of monocyclic groups include pyridyl, pyridazinyl, pyrimidinyl, pyrazinyl, pyrrolyl, pyrazolyl, imidazolyl, triazolyl, tetrazolyl, furanyl, thiophenyl, oxazolyl, isoxazolyl, oxadiazolyl, thiazolyl, isothiazolyl, and thiadiazolyl. Examples of bicyclic groups include quinolinyl, cinnolinyl, quinoxalinyl, benzimidazolyl, benzothiophenyl, and benzothiadiazolyl. Monocyclic heteroaryl groups are preferred, preferably pyridyl, pyrrolyl, imidazolyl and triazolyl, e.g. 1,2,4 triazolyl, pyridyl and imidazolyl being most preferred.

The terms "heterocycle" and "heterocyclic ring" are used interchangeably and are defined to include heterocycloalkyl and heteroaryl groups. Any reference herein to a heterocycle or heterocyclic ring preferably refers to the specific examples given under the definition of heteroaryl and heterocycloalkyl above, and are preferably morpholinyl, pyrrolidinyl, piperdinyl, piperazinyl pyridyl, pyrrolyl, imidazolyl and triazolyl, e.g. 1,2,4 triazolyl, more preferably morpholinyl, pyrollidinyl, pyridyl and imidazolyl.

Where a moiety is indicated as being (optionally) substituted, e.g. alkyl, this includes those moieties where they are part of a larger group, e.g. the alkyl in the alkylthio group. Where a moiety is indicated as being optionally substituted by one or more other groups, preferably there are one to five optional substituents, more preferably one to three optional substituents.

The following substituents definitions, including preferred definitions, may be combined in any combination:
D¹ represents N or C-Y⁴;
D² represents N or C-Y⁵;
D³ represents N or C-Y⁶;
wherein both D¹ and D² cannot be N.

In one group of compounds, D¹ represents C-Y⁴, D² represents N and D³ represents C-Y⁶.

In one group of compounds, D¹ represents C-Y⁴, D² represents C-Y⁵ and D³ represents N.

In one group of compounds, D¹ represents N, D² represents C-Y⁵ and D³ represents C-Y⁶.

In one group of compounds, D¹ represents N, D² represents C-Y⁵ and D³ represents N.

In one group of compounds, D¹ represents C-Y⁴, D² represents N and D³ represents N.
Y¹, Y², Y³, Y⁴, Y⁵ and Y⁶ independently of one another represent hydrogen, halogen, CN, NO₂, C₁-C₈ alkyl, C₁-C₄-alkoxy-C₁-C₄-alkyl, C₁-C₄-alkoxy-C₁-C₄-alkoxy-C₁-C₄-alkyl, C₃-C₈ cycloalkyl, C₂-C₈ alkenyl, C₂-C₈ alkynyl, phenyl, pyridyl, pyrimidinyl, OR³, COR⁴, SH, C₁-C₈ alkylthio, C₁-C₈ alkylsulphinyl, C₁-C₈ alkylsulphonyl, N(R⁵)₂, CO₂R³, O(CO)R⁴, CON(R⁵)₂, NR⁵COR⁴ or CR⁴N-OR³, wherein the alkyl, cycloalkyl, alkenyl, alkynyl, phenyl, pyrimidinyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from O, S, N and N(R⁵), providing that the heterocycle does not contain adjacent oxygen atoms, adjacent sulphur atoms, or adjacent sulphur and oxygen atoms, and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy and C₁-C₄-alkoxy-C₁-C₄-alkyl.

Preferably, Y¹, Y², Y³, Y⁴, Y⁵ and Y⁶ independently of one represent hydrogen, halogen, OH, CN, C₁-C₈ alkyl, C₁-C₈ haloalkyl, C₁-C₈ alkoxy, C₁-C₈ haloalkoxy, C₁-C₈ alkylthio, C₃-C₈ cycloalkyl, phenyl, pyridyl, N(R⁵)₂, or NR⁵COR⁴, wherein phenyl and pyridyl are optionally substituted by one or more groups independently selected from halogen, CN, C₁-C₄ alkyl, C₁-C₄ haloalkyl, C₁-C₄ alkoxy and C₁-C₄ haloalkoxy;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from N and N(R⁵)₂ and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy; wherein each R⁴ independently represents C₁-C₈ alkyl or C₁-C₈ haloalkyl;
wherein each R⁵ independently of one another represents hydrogen, C₁-C₈ alkyl, COR⁴ or phenyl, wherein the phenyl is optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5;
wherein the cycle formed is optionally substituted by one or more groups independently selected from halogen, methyl and halomethyl.

More preferably, Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₃-C₆ cycloalkyl, C₁-C₄ alkylthio, N(R⁵)₂, NR⁵COR⁴ or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
wherein each R⁴ independently represents C₁-C₄ alkyl or C₁-C₄ haloalkyl;
wherein each R⁵ independently of one another represents hydrogen, C₁-C₈ alkyl or phenyl, wherein phenyl is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5 wherein the cycle formed is optionally substituted by one or more independently selected from halogen, methyl and halomethyl.

Even more preferably Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₃-C₆ cycloalkyl, C₁-C₄ alkylthio, N(R⁵)₂, NR⁵COR⁴ or phenyl, wherein phenyl is optionally substituted by one or more groups independently selected selected from halogen, methyl, CN, methoxy, halomethyl and halomethoxy;
wherein each R⁴ independently represents C₁-C₄ alkyl or C₁-C₄ haloalkyl;
wherein each R⁵ independently of one another represents hydrogen, C₁-C₈ alkyl or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy.

Yet more preferably, Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₃-C₆ cycloalkyl, C₁-C₄ alkylthio, N(R⁵)₂, NR⁵COR⁴ or phenyl, wherein phenyl is optionally substituted by one or more groups independently selected selected from halogen, methyl, CN, methoxy, halomethyl and halomethoxy;
wherein each R⁴ independently represents C₁-C₄ alkyl or C₁-C₄ haloalkyl;
wherein each R⁵ independently of one another represents hydrogen, C₁-C₈ alkyl or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy.

Even more preferably, Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy or C₁-C₄ haloalkoxy.

R¹ represents hydrogen, halogen, CN, OH, SH, C₁-C₈ alkylthio, C₁-C₈ alkylsulphinyl, C₁-C₈ alkylsulphonyl, NH₂, C₁-C₁₀ alkyl, C₃-C₈ cycloalkyl, C₂-C₈ alkenyl, C₂-C₈ alkynyl, (R³O)carbonyl(C₁-C₄-alkyl), phenyl or pyridyl, wherein the alkyl, cycloalkyl, alkenyl, alkynyl, phenyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₃-C₆ cycloalkyl and a 5- or 6-membered heterocycle containing one to three heteroatoms independently selected from O, S and N, providing that the heterocycle does not contain adjacent oxygen atoms, adjacent sulphur atoms, or adjacent sulphur and oxygen atoms;
or if D² is C-Y⁵ then Y⁵ and R¹ together may be -(G⁴)_{q}-G⁵-G⁶-.

Preferably R¹ represents hydrogen, C₁-C₈ alkyl, C₃-C₈ cycloalkyl, phenyl or pyridyl, wherein the alkyl, cycloalkyl, phenyl and pyridyl are optionally substituted by one or more groups, e.g. one to five groups, independently selected from halogen, CN, C₁-C₄ alkyl, C₁-C₄ haloalkyl, OH, C₁-C₄ alkoxy, C₁-C₄ haloalkoxy and C₃-C₆ cycloalkyl.

More preferably R¹ represents hydrogen, C₁-C₄ alkyl, phenyl or pyridyl, wherein alkyl is optionally substituted by one or more groups, e.g. one to five groups, independently selected from halogen, OH, C₁-C₄ alkoxy and C₁-C₄ haloalkoxy, and wherein phenyl and pyridyl are optionally substituted by one or more groups, e.g. one to five groups, independently selected from halogen, CN, C₁-C₄ alkyl, C₁-C₄ haloalkyl, OH, C₁-C₄ alkoxy, C₁-C₄ haloalkoxy and C₃-C₆ cycloalkyl. Even more preferably R¹ represents hydrogen, C₁-C₄ alkyl, phenyl or pyridin-2-yl, wherein the phenyl and pyridin-2-yl are optionally substituted by one or more groups, e.g. one to five groups, independently selected from halogen, CN, methyl, halomethyl, methoxy and halomethoxy.

Yet more preferably, R¹ represents C₁-C₄ alkyl.

In another group of compounds R¹ represents hydrogen, C₁-C₄ alkyl, C₂-C₄ alkenyl, phenyl or pyridyl, wherein the alkyl, alkenyl, phenyl and pyridyl are optionally substituted by one or more groups independently selected from C₁-C₄ alkyl, C₁-C₄ haloalkyl, halogen, CN, C₁-C₄ alkoxy and C₁-C₄ haloalkoxy.

In another preferred group of compounds, R¹ represents hydrogen, halogen, CN, OH, SH, C₁-C₈ alkylthio, C₁-C₈ alkylsulphinyl, C₁-C₈ alkylsulphonyl, NH₂, C₁-C₁₀ alkyl, C₃-C₈ cycloalkyl, C₂-C₈ alkenyl, C₂-C₈ alkynyl, (C₁-C₄ alkyloxycarbonyl) C₁-C₄ alkyl, (C₁-C₄ alkyl)O₂C, phenyl or pyridyl, wherein the alkyl, cycloalkyl, alkenyl, alkynyl, phenyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₃-C₆ cycloalkyl and a 5- or 6-membered heterocycle containing one to three heteroatoms independently selected from O, S and N, providing that the heterocycle does not contain adjacent oxygen atoms, adjacent sulphur atoms, or adjacent sulphur and oxygen atoms.

In another preferred group of compounds, R¹ represents hydrogen, (C₁-C₄ alkyl)O₂C, C₁-C₁₀ alkyl, phenyl or pyridyl, wherein the alkyl, phenyl and pyridyl are optionally substituted by one or more groups independently selected from halogen, CN, C₁-C₄ alkyl, C₁-C₄ haloalkyl, C₃-C₆ cycloalkyl and a 5- or 6-membered heterocycle containing one to three nitrogen atoms.

In a further preferred group of compounds, D² is C-Y⁵ and R¹ and Y⁵ together may be -(G⁴)_{q}-G⁵-G⁶-.

R² represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy.

Preferably, R² represents hydrogen, C₁-C₈ alkyl, C₁-C₈ haloalkyl, C₃-C₈ cycloalkyl, C₃-C₈ halocycloalkyl, C₃-C₈ alkenyl, C₃-C₈ haloalkenyl, C₃-C₈ alkynyl, C₃-C₈ haloalkynyl, benzyl or pyridyl, wherein the, benzyl and pyridyl are optionally substituted by one or more groups, e.g. one to five groups, independently selected from halogen, CN, C₁-C₄ alkyl, C₁-C₄ haloalkyl, C₁-C₄ alkoxy and C₁-C₄ haloalkoxy.

More preferably, R² represents C₁-C₄ alkyl, C₃-C₆ alkenyl, C₃-C₆ alkynyl or benzyl, wherein the alkyl, alkenyl, alkynyl and benzyl are optionally substituted by one or more groups, e.g. one to five groups, independently selected from halogen, CN, C₁-C₄ alkoxy and C₁-C₄ haloalkoxy.

More preferably, R² represents hydrogen, C₁-C₄ alkyl, or C₁-C₄ haloalkyl.

In one group of compounds R² represents hydrogen, C₁-C₄ alkyl, C₃-C₆ alkenyl, C₃-C₆ alkynyl or benzyl, wherein the alkyl, alkenyl, alkynyl and benzyl are optionally substituted by one or ore groups, e.g. one to five groups, independently selected from C₁-C₄ alkyl, C₁-C₄ haloalkyl, halogen, CN, C₁-C₄ alkoxy and C₁-C₄ haloalkoxy.

Each R³ independently of one another represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups, e.g. one to five groups, independently selected from halogen, CN, NH₂, NO₂, OH, C₁-C₄ alkyl, C₁-C₄ haloalkyl, C₁-C₄-alkoxy, C₁-C₄-haloalkoxy and C₁-C₄-alkoxy-C₁-C₄-alkyl.

Each R⁴ independently of one another represents hydrogen, C₁-C₈ alkyl, C₃-C₈ cycloalkyl, C₂-C₈ alkenyl, C₂-C₈ alkynyl, benzyl, phenyl or pyridyl, wherein the alkyl, cycloalkyl, alkenyl, alkynyl, phenyl, benzyl and pyridyl are optionally substituted by one or more groups, e.g. one to five groups, independently selected from halogen, CN, NH₂, NO₂, OH, C₁-C₄ alkyl, C₁-C₄ haloalkyl, C₁-C₄ alkoxy and C₁-C₄ haloalkoxy.

Preferably each R⁴ independently of one another represent C₁-C₈ alkyl or C₁-C₈ haloalkyl.

More preferably each R⁴ independently of one another represent C₁-C₄ alkyl or C₁-C₄ haloalkyl.

Each R⁵ independently of one another represents hydrogen, OH, C₁-C₈ alkyl, C₁-C₈ alkoxy, C₁-C₈-alkoxy-C₁-C₄-alkyl, C₃-C₈ alkenyl, C₃-C₈ alkynyl, COR⁴ or phenyl, wherein the alkyl, alkoxy, alkenyl, alkynyl and phenyl are optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5, B-6, B-7 or B-8: 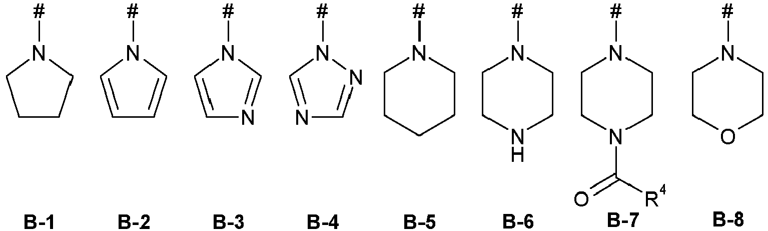
wherein the cycle formed is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy.

Preferably, each R⁵ independently of one another represents hydrogen, C₁-C₈ alkyl, COR⁴ or phenyl, wherein the phenyl is optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5;
wherein the cycle formed is optionally substituted by one or more groups independently selected from halogen, methyl and halomethyl.

More preferably, each R⁵ independently of one another represents hydrogen, C₁-C₈ alkyl or phenyl, wherein phenyl is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy; wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkyoxy.

More preferably still, each R⁵ independently of one another represents hydrogen, C₁-C₈ alkyl or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkyoxy.

X represents X-2, X-3, X-4 or X-5:

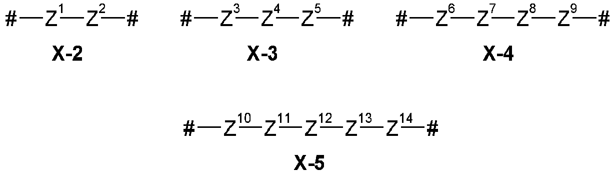

Preferably X represents X-3 or X-5. More preferably X represents X-3.
Z¹, Z², Z³, Z⁵, Z⁶, Z⁷, Z⁸, Z⁹, Z¹⁰, Z¹¹, Z¹³ and Z¹⁴ independently of one another represent CR⁶R⁷, C=CR⁸R⁹or C=O;
or in each case two adjacent radicals Z⁴ and Z⁵ or Z⁷ and Z⁸ or Z⁸ and Z⁹ or Z¹¹ and Z¹² or Z¹² and Z¹³ or Z¹³ and Z¹⁴ may together represent a group selected from CR¹⁴=CR¹⁵- and -C≡C-, wherein X-4 or X-5 may not contain more than one such group.

Preferably Z¹, Z², Z³, Z⁵, Z⁶, Z⁷, Z⁸, Z⁹, Z¹⁰, Z¹¹, Z¹³ and Z¹⁴ independently of one another represent methylene, halomethylene, CH(CH₃) or C(CH₃)₂.

More preferably Z¹, Z², Z³, Z⁵, Z⁶, Z⁷ Z⁸, Z⁹ Z¹⁰, Z¹¹, Z¹³ and Z¹⁴ represent methylene or halomethylene.

Most preferably Z¹, Z², Z³, Z⁵, Z⁶, Z⁷, Z⁸, Z⁹, Z¹⁰, Z¹¹ Z¹³ and Z¹⁴ represent methylene.

Z⁴ and Z¹² independently of one another represent C=CR⁸R⁹, CR¹⁰R¹¹, SiR¹²R¹³ or C=O. Preferably Z⁴ and Z¹² independently of one another represent C=CR⁸R⁹, CR¹⁰R¹¹ or SiR¹²R¹³. More preferably Z⁴ and Z¹² independently of one another represent C=CR⁸R⁹ or CR¹⁰R¹¹ .

Most preferably Z⁴ and Z¹² independently of one another represent CR¹⁰R¹¹.

Each R⁶ and R⁷ independently of one another represent hydrogen, halogen, OH, C₁-C₄ alkyl, C₁-C₄ haloalkyl, C₃-C₆ cycloalkyl, C₃-C₆ halocycloalkyl, C₁-C₄ alkylthio, C₁-C₄ alkylsulphinyl, C₁-C₄ alkylsulphonyl, phenyl or CN, wherein the phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkoxy.; or R⁶ and R⁷ together with the carbon atom to which they are attached may form a C₃-C₆ cycloalkyl group or a C₃-C₆ halocycloalkyl group.

Each R⁸ and R⁹ independently of one another represent hydrogen, halogen, C₁-C₄ alkyl or C₁-C₄ haloalkyl.

Preferably each R⁸ and R⁹ independently of one another represent hydrogen, halogen, methyl or halomethyl.

Each R¹⁰, R¹¹, R¹² and R¹³ independently of one another represent hydrogen, halogen, OH, C₁-C₄ alkyl, C₁-C₄ haloalkyl, C₃-C₆ cycloalkyl, C₃-C₆ halocycloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkoxy; or R¹⁰ and R¹¹ together with the carbon atom to which they are attached may form a C₃-C₆ cycloalkyl group or a C₃-C₆ halocycloalkyl group.

Preferably, each R¹⁰, R¹¹, R¹² and R¹³ independently of one another represent hydrogen, halogen, OH, C₁-C₄ alkyl, C₁-C₄ haloalkyl, C₃-C₆ cycloalkyl, phenyl or CN, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy; or R¹⁰ and R¹¹ together with the carbon atom to which they are attached may form a C₃-C₆ cycloalkyl group or a C₃-C₆ halocycloalkyl group.

More preferably, each R¹⁰, R¹¹, R¹² and R¹³ independently of one another represent hydrogen, halogen, OH, C₁-C₄ alkyl, C₁-C₄ haloalkyl, phenyl or CN wherein the phenyl is optionally substituted by one or more groups independently selected from halogen, CN, methyl, halomethyl, methoxy and halomethoxy; or R⁸ and R⁹ together with the carbon atom to which they are attached may form a C₃-C₆ cycloalkyl group optionally substituted by halogen.

More preferably still, each R¹⁰, R¹¹, R¹² and R¹³ independently of one another represent hydrogen, halogen, C₁-C₄ alkyl, C₁-C₄ haloalkyl or phenyl, wherein the phenyl is optionally substituted by one or more groups independently selected from halogen, CN, methyl, halomethyl, methoxy and halomethoxy; or R⁸ and R⁹ together with the carbon atom to which they are attached may form a C₃-C₆ cycloalkyl group optionally substituted by halogen.

Most preferably, R¹⁰, R¹¹, R¹² and R¹³ independently of one another represent hydrogen, halogen, C₁-C₄ alkyl or C₁-C₄ haloalkyl.
G¹, G², G⁴ and G⁵ independently of one another represent -C(R¹⁶R¹⁷)-;
G³ and G⁶ independently of one another represent -C(R¹⁶R¹⁷)-, O, N(R¹⁸) or S;
or G¹ and G², or G² and G³, or G¹ and G¹, or G⁴ and G⁵, or G⁵ and G⁶, or G⁴ and G⁴ together represent -CR¹⁶=CR¹⁷-. For the avoidance of doubt, when p is 2, G¹ and G¹ as well as G² and G³ may represent CR¹⁶=CR¹⁷ such that the ring contains two double bond moieties. Likewise, when q is 2, G⁴ and G⁴ as well as G⁵ and G⁶ may represent CR¹⁶=CR¹⁷.

Preferably G¹, G², G⁴ and G⁵ independently of one another represent -C(R¹⁶R¹⁷)- ;
G³ and G⁶ independently of one another represent -C(R¹⁶R¹⁷)-, O, N(R¹⁸) or S; or G¹ and G², or G² and G³, or G¹ and G¹, or G⁴ and G⁵, or G⁵ and G⁶, or G⁴ and G⁴ together represent -CR¹⁶=CR¹⁷-.

More preferably, G¹, G², G⁴ and G⁵ independently of one another represent -C(R¹⁶R¹⁷)-; G³ and G⁶ independently of one another represent -C(R¹⁶R¹⁷)-, O, N(R¹⁸) or S.

More preferably again, G¹, G², G³, G⁴, G⁵ and G⁶ independently of one another represent - C(R¹⁶R¹⁷)-.

Even more preferably G¹, G², G³, G⁴, G⁵ and G⁶ represent methylene.

Each R¹⁶ and R¹⁷ independently of one another represent hydrogen, halogen, C₁-C₄ alkyl, C₁-C₄ haloalkyl, C₁-C₄ alkoxy or C₁-C₄ haloalkoxy.

R¹⁸ represents hydrogen, OH, C₁-C₄ alkyl, C₁-C₄ alkoxy, C₃-C₆-cycloalkyl, C₁-C₈ alkylcarbonyl or C₁-C₈ haloalkylcarbonyl.

p and q are each independently of one another 1 or 2.

Preferably p and q are 1.

In one group of compounds, D¹ represents N or C-Y⁴;
D² represents N or C-Y⁵;
D³ represents N or C-Y⁶;
wherein both D¹ and D² cannot be N;
Y¹, Y², Y³, Y⁴, Y⁵ and Y⁶ independently of one another represent hydrogen, halogen, CN, NO₂, C₁-C₈ alkyl, C₁-C₄-alkoxy-C₁-C₄-alkyl, C₁-C₄-alkoxy-C₁-C₄-alkoxy-C₁-C₄-alkyl, C₃-C₈ cycloalkyl, C₂-C₈ alkenyl, C₂-C₈ alkynyl, phenyl, pyridyl, pyrimidinyl, OR³, COR⁴, SH, C₁-C₈ alkylthio, C₁-C₈ alkylsulphinyl, C₁-C₈ alkylsulphonyl, N(R⁵)2, CO₂R³, O(CO)R⁴, CON(R⁵)₂, NR⁵COR⁴ or CR⁴N-OR³, wherein the alkyl, cycloalkyl, alkenyl, alkynyl, phenyl, pyrimidinyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from O, S, N and N(R⁵), providing that the heterocycle does not contain adjacent oxygen atoms, adjacent sulphur atoms or adjacent sulphur and oxygen atoms, and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
R¹ represents hydrogen, halogen, CN, OH, SH, C₁-C₈ alkylthio, C₁-C₈ alkylsulphinyl, C₁-C₈ alkylsulphonyl, NH₂, C₁-C₁₀ alkyl, C₃-C₈ cycloalkyl, C₂-C₈ alkenyl, C₂-C₈ alkynyl, (R³O)carbonyl(C₁-C₄-alkyl), phenyl or pyridyl, wherein the alkyl, cycloalkyl, alkenyl, alkynyl, phenyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkyoxy, C₃-C₆ cycloalkyl and a 5- or 6-membered heterocycle containing one to three heteroatoms independently selected from O, S and N, providing that the heterocycle does not contain adjacent oxygen atoms, adjacent sulphur atoms, or adjacent sulphur and oxygen atoms;
or if D² is C-Y⁵ then R¹ and Y⁵ together may be -(G⁴)_{q}-G⁵-G⁶-;
R² represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
each R³ independently of one another represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄-haloalkyl, C₁-C₄ alkoxy, C₁-C₄ haloalkoxy and C₁-C₄-alkoxy-C₁-C₄-alkyl;
each R⁴ independently of one another represents hydrogen, C₁-C₈ alkyl, C₃-C₈ cycloalkyl, C₂-C₈ alkenyl, C₂-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
each R⁵ independently of one another represents hydrogen, OH, C₁-C₈ alkyl, C₁-C₈ alkoxy, C₁-C₈-alkoxy-C₁-C₄-alkyl, C₃-C₈ alkenyl, C₃-C₈ alkynyl, COR⁴ or phenyl, wherein the alkyl, alkoxy, alkenyl, alkynyl and phenyl are optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5, B-6, B-7 or B-8;
wherein the cycle formed is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
X represents X-2, X-3, X-4 or X-5: 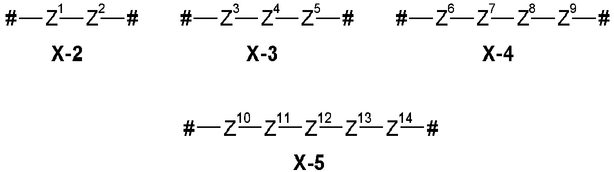
Z¹, Z², Z³, Z⁵, Z⁶, Z⁷, Z⁸, Z⁹, Z¹⁰, Z¹¹, Z¹³ and Z¹⁴ independently of one another represent CR⁶R⁷, C=CR⁸R⁹ or C=O;
or in each case two adjacent radicals Z⁴ and Z⁵ or Z⁷ and Z⁸ or Z⁸ and Z⁹ or Z¹¹ and Z¹² or Z¹² and Z¹³ or Z¹³ and Z¹⁴ may together represent a group selected from CR¹⁴=CR¹⁴- and -C≡C-, wherein X-4 or X-5 may not contain more than one such group;
Z⁴ and Z¹² independently of one another represent C=CR⁸R⁹, CR¹⁰R¹¹, SiR¹²R¹³ or C=O;
each R⁶ and R⁷ independently of one another represent hydrogen, halogen, OH, C₁-C₄ alkyl, C₁-C₄ haloalkyl, C₃-C₆ cycloalkyl, C₃-C₆ halocycloalkyl, C₁-C₄ alkylthio, C₁-C₄ alkylsulphinyl, C₁-C₄ alkylsulphonyl, phenyl or CN, wherein the phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R⁶ and R⁷ together with the carbon atom to which they are attached may form a C₃-C₆ cycloalkyl group or a C₃-C₆ halocycloalkyl group;
each R⁸ and R⁹ independently of one another represent hydrogen, halogen, C₁-C₄ alkyl or C₁-C₄ haloalkyl;
each R¹⁰, R¹¹, R¹² and R¹³ independently of one another represent hydrogen, halogen, OH, C₁-C₄ alkyl, C₁-C₄ haloalkyl, C₃-C₆ cycloalkyl, C₃-C₆ halocycloalkyl, phenyl or CN, wherein phenyl is optionally substituted by one or more groups independently selected from halogen, CN, C1-C₄ alkyl, C₁-C₄ haloalkyl, C₁-C₄ alkoxy and C₁-C₄ haloalkoxy;
or R¹⁰ and R¹¹ together with the carbon atom to which they are attached may form a C₃-C₆ cycloalkyl group or a C₃-C₆ halocycloalkyl group;
wherein the groupings X-2, X-3, X-4 and X-5 may contain at most one ring which contains either only one of the radicals Z¹ to Z¹⁴ or two radicals Z¹ to Z¹⁴ or three radicals Z¹ to Z¹⁴ or four radicals Z¹ to Z¹⁴ as ring members; and wherein radicals Z¹, Z², Z³, Z⁵, Z⁶, Z⁹, Z¹⁰ and Z¹⁴ are not substituted by OH;
G¹, G², G⁴ and G⁵ independently of one another represent -C(R¹⁶R¹⁷)-;
G³ and G⁶ independently of one another represent -C(R¹⁶R¹⁷)-, O, N(R¹⁸) or S;
or G¹ and G², or G² and G³, or G¹ and G¹, or G⁴ and G⁵, or G⁵ and G⁶, or G⁴ and G⁴ together represent -CR¹⁶=CR¹⁷-;
each R¹⁶ and R¹⁷ independently of one another represent hydrogen, halogen, C₁-C₄ alkyl, C₁-C₄ haloalkyl, C₁-C₄ alkoxy or C₁-C₄ haloalkoxy;
R¹⁸ represents hydrogen, OH, C₁-C₄ alkyl, C₁-C₄ alkoxy, C₃-C₆ cycloalkyl, C₁-C₈ alkylcarbonyl or C₁-C₈ haloalkylcarbonyl; and
p and q are each independently 0, 1 or 2.

In another group of compounds, Y¹, Y², Y³, Y⁴, Y⁵ and Y⁶ independently of one represent hydrogen, halogen, OH, CN, C₁-C₈ alkyl, C₁-C₈ haloalkyl, C₁-C₈ alkoxy, C₁-C₈ haloalkoxy, C₁-C₈ alkylthio, C₃-C₈ cycloalkyl, phenyl, pyridyl, N(R⁵)₂, or NR⁵COR⁴, wherein phenyl and pyridyl are optionally substituted by one or more groups independently selected from halogen, CN, C₁-C₄ alkyl, C₁-C₄ haloalkyl, C₁-C₄ alkoxy and C₁-C₄ haloalkoxy;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from N and N(R⁵)₂ and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy; R¹ represents hydrogen, C₁-C₈ alkyl, C₃-C₈ cycloalkyl, phenyl or pyridyl, wherein the alkyl, cycloalkyl, phenyl and pyridyl are optionally substituted by one or more groups independently selected from halogen, CN, C₁-C₄ alkyl, C₁-C₄ haloalkyl, OH, C₁-C₄ alkoxy, C₁-C₄ haloalkoxy and C₃-C₆ cycloalkyl;
R² represents C₁-C₄ alkyl, C₁-C₄ haloalkyl, C₃-C₈ cycloalkyl, C₃-C₈ halocycloalkyl, C₃-C₈ alkenyl, C₃-C₈ haloalkenyl, C₃-C₈ alkynyl, C₃-C₈ haloalkynyl, benzyl or pyridyl, wherein the, benzyl and pyridyl are optionally substituted by one or more groups, e.g. one to five groups, independently selected from halogen, CN, C₁-C₄ alkyl, C₁-C₄ haloalkyl, C₁-C₄ alkoxy and C₁-C₄ haloalkoxy;
each R⁴ independently represents C₁-C₈ alkyl or C₁-C₈ haloalkyl;
each R⁵ independently of one another represents hydrogen, C₁-C₈ alkyl, COR⁴ or phenyl, wherein the phenyl is optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5;
wherein the cycle formed is optionally substituted by one or more groups independently selected from halogen, methyl and halomethyl;
X represents X-3;
Z³ and Z⁵ independently of one another represent methylene or halomethylene;
Z⁴ represent C=C_{R}⁸_{R}⁹, or CR¹⁰R¹¹;
each R⁸ and R⁹ independently of one another represent hydrogen, halogen, methyl or halomethyl;
each R¹⁰and R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴ , G⁵ and G⁶ independently of one another represent -C(R¹⁶R¹⁷)-;
p and q are each independently 1 or 2.

In another group of compounds, Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₁-C₄ alkylthio, C₃-C₆ cycloalkyl, N(R⁵)₂, NR⁵COR⁴ or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
R¹ represents hydrogen, C₁-C₄ alkyl, phenyl or pyridyl, wherein alkyl is optionally substituted by one or more groups independently selected from halogen, OH, C₁-C₄ alkoxy and C₁-C₄ haloalkoxy, and wherein phenyl and pyridyl are optionally substituted by one or more groups independently selected from halogen, CN, C₁-C₄ alkyl, C₁-C₄ haloalkyl, OH, C₁-C₄ alkoxy, C₁-C₄ haloalkoxy and C₃-C₆ cycloalkyl;
R² represents C₁-C₄ alkyl, C₃-C₆ alkenyl, C₃-C₆ alkynyl or benzyl, wherein the alkyl, alkenyl, alkynyl and benzyl are optionally substituted by one or more groups, e.g. one to five groups, independently selected from halogen, CN, C₁-C₄ alkoxy and C₁-C₄ haloalkoxy;
each R⁴ independently represents C₁-C₄ alkyl or C₁-C₄ haloalkyl;
each R⁵ independently of one another represents hydrogen, C₁-C₈ alkyl or phenyl, wherein phenyl is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5 wherein the cycle formed is optionally substituted by one or more independently selected from halogen, methyl and halomethyl;
X represents X-3;
Z³ and Z⁵ represent methylene;
Z⁴ represents C=CR⁸R⁹ or CR¹⁰R¹¹;
each R⁸ and R⁹ independently of one another represent hydrogen, halogen, methyl or halomethyl;
each R¹⁰ and R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴, G⁵ and G⁶ represent methylene;
p and q are each independently 1 or 2.

In a further group of compounds, Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₁-C₄ alkylthio, C₃-C₆ cycloalkyl, N(R⁵)₂, NR⁵COR⁴ or phenyl, wherein phenyl is optionally substituted by one or more groups independently selected selected from halogen, methyl, CN, methoxy, halomethyl and halomethoxy;
R¹ represents hydrogen, C₁-C₄ alkyl, phenyl or pyridin-2-yl wherein phenyl and pyridin-2-yl are optionally substituted by one or more groups independently selected from halogen, CN, methyl, halomethyl, methoxy and halomethoxy;
R² represents hydrogen, C₁-C₈ alkyl, or C₁-C₈ haloalkyl;
each R⁴ independently represents C₁-C₄ alkyl or C₁-C₄ haloalkyl;
each R⁵ independently of one another represents hydrogen, C₁-C₈ alkyl or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
X represents X-3;
Z³ and Z⁵ represent methylene;
Z⁴ represents C=CR⁸R⁹ or CR¹⁰R¹¹;
each R⁸ and R⁹ independently of one another represent hydrogen, halogen, methyl or halomethyl;
each R¹⁰ and R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴, G⁵ and G⁶ represent methylene;
p and q are each independently 1 or 2.

In one group of compounds, p is 1 and -G¹-G²-G³- represent -CH₂-CH₂-CH₂-.

In another group of compounds, R¹ and Y⁵ together represent -CH₂-CH₂-CH₂-.

Certain intermediates that can be used to prepare compounds of formula (I) are novel and as such also form part of the present invention.

Accordingly, in a further aspect the invention provides a compound of formula (IV) 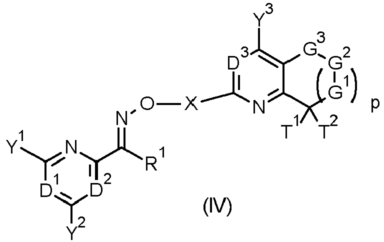 wherein D¹, D², D³, p, X, Y¹, Y², Y³ and R¹ are as defined herein for compounds of formula (I) and T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl; or a salt or N-oxide thereof.

The preferred definitions of R¹, X, G¹, G², G³, D¹, D², D³, Y¹, Y², Y³ and p defined in respect of compounds of formula (I) also apply to compounds of formula (IV).

One preferred group of compounds of formula (IV) are the compounds of formula (IVa), that is a compound of formula (IV) wherein X is X-3, X-4 or X-5, wherein Z⁴ and Z⁵, Z⁸ and Z⁹ or Z¹³ and Z¹⁴ together form -C≡C- and Z³, Z⁶, Z⁷, Z¹⁰, Z¹¹ and Z¹² are as defined herein for compounds of formula (I).

Another preferred group of compounds of formula (IV) are the compounds of formula (IVb), that is a compound of formula (IV) wherein X is X-3, X-4 or X-5, wherein Z⁴, Z⁵, Z⁸, Z⁹, Z¹³ and Z¹⁴ are methylene and Z³, Z⁶, Z⁷, Z¹⁰, Z¹¹ and Z¹² are as defined herein for compounds of formula (I).

In a further aspect the invention provides a compound of formula (VII) 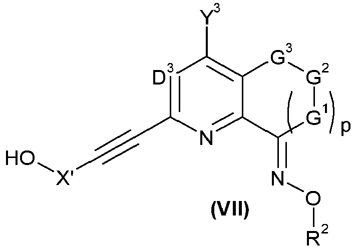 wherein X' represents X'-1, X'-2 or X'-3 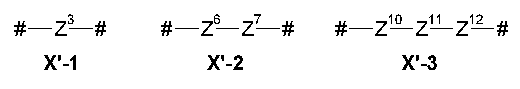 wherein Z³, Z⁴, Z⁶, Z⁷, Z¹⁰, Z¹¹ and Z¹² are as defined herein for a compound of formula (I) and D³, Y³, R², G¹, G², G³ and p are as defined herein for compounds of formula (I); or a salt or N-oxide thereof.

The preferred definitions of R², G¹, G², G³, D³, Y³ and p defined in respect of compounds of formula (I) also apply to compounds of formula (VII).

In a further aspect the invention provides a compound of formula (XI) 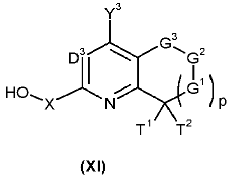 wherein and T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and X, D³, Y³, G¹, G², G³ and p are as defined herein for compounds of formula (I); or a salt or N-oxide thereof.

The preferred definitions of X, D³, Y³, G¹, G², G³ and p defined in respect of compounds of formula (I) also apply to compounds of formula (XI).

One preferred group of compounds of formula (XI) are the compounds of formula (XIa), that is a compound of formula (XI) wherein X is X-3, X-4 or X-5, wherein Z⁴ and Z⁵, Z⁸ and Z⁹ or Z¹³ and Z¹⁴ together form -C≡C- and Z³, Z⁶, Z⁷, Z¹⁰, Z¹¹ and Z¹² are as defined herein for compounds of formula (I).

In a further aspect the invention provides a compound of formula (XII) 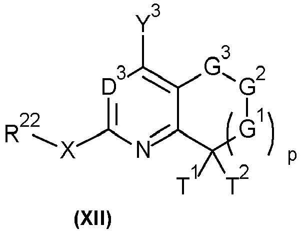 wherein R²² is a halogen or a sulfonic acid ester group; T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and X, D³, Y³, G¹, G², G³ and p are as defined herein for compounds of formula (I);
or a salt or N-oxide thereof.

The preferred definitions of X, D³, Y³, G¹, G², G³ and p defined in respect of compounds of formula (I) also apply to compounds of formula (XII).

In a preferred group of compounds of formula (XII), R¹⁸ is chlorine, bromine, iodine, mesylate, tosylate, triflate, phenylsulfonic acid ester, nitro-phenylsulfonic acid ester, or nonafluorobutylsulfonic acid ester.

One preferred group of compounds of formula (XII) are the compounds of formula (XIIa), that is a compound of formula (XII) wherein X is X-3, X-4 or X-5, wherein Z⁴ and Z⁵, Z⁸ and Z⁹ or Z¹³ and Z¹⁴ together form -C≡C- and Z³, Z⁶, Z⁷, Z¹⁰, Z¹¹ and Z¹² are as defined herein for compounds of formula (I).

In a further aspect the invention provides a compound of formula (XVI) 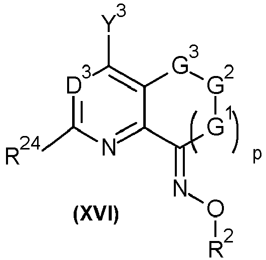 wherein R²⁴ is Cl, Br, I or a sulfonate and R², D³, Y³, G¹, G², G³ and p are as defined herein for compounds of formula (I);
or a salt or N-oxide thereof.

The preferred definitions of R², D³, Y³, G¹, G², G³ and p defined in respect of compounds of formula (I) also apply to compounds of formula (XVI).

The compounds of formula (I) may exist as different geometric or optical isomers or in different tautomeric forms. These may be separated and isolated by well-known (usually chromatographic) techniques, and all such isomers and tautomers and mixtures thereof in all proportions as well as isotopic forms, such as deuterated compounds, are part of the present invention. In particular, the carbon-nitrogen double bonds of the compound of formula (I) allow the four cis/trans isomers shown below:

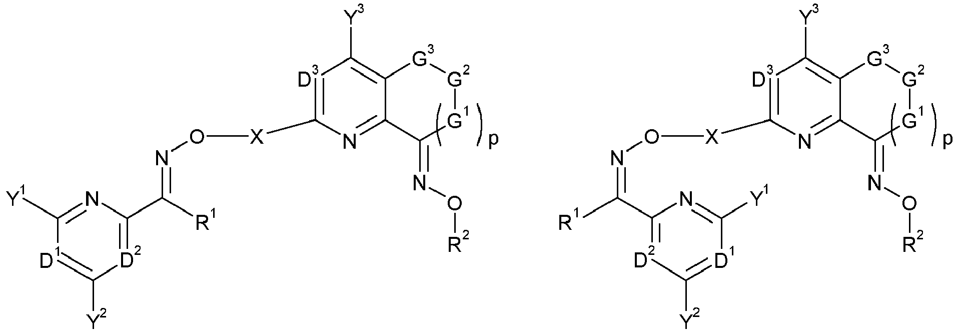

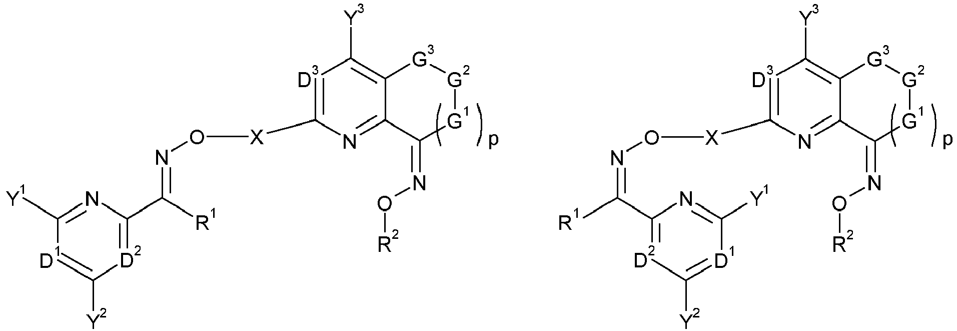

The present invention includes each of these isomers. The invention may provide a compound of formula (I) as just one of these isomers or as a mixture of one or more isomers in any ratio. Likewise, the invention also includes the corresponding isomers of the intermediates described herein, e.g. compounds (II), (IV) and (VIII). In addition, where a reaction scheme depicts synthesis of one geometric isomer, the scheme also includes synthesis of the other geometric isomers where possible.

The compounds in tables 1 to 5 illustrate compounds of formula (I).

Table X represents Table 1 (when X is 1), Table 2 (when X is 2), Table 3 (when X is 3), Table 4 (when X is 4), Table 5 (when X is 5).

**Table 1: This table discloses compounds 1.01 to 1.182 of the formula (I-I)**

| | **Y¹** | **D¹** | **Y²** | **D²** | **R¹** |
|---|---|---|---|---|---|
| X.001 | H | C-H | H | C-H | CH₃ |
| X.002 | H | N | H | C-H | CH₃ |
| X.003 | H | C-H | H | N | CH₃ |
| X.004 | CH₃ | C-H | H | C-H | H |
| X.005 | CH₃ | C-H | H | C-H | CH₃ |
| X.006 | CH₃ | C-H | H | C-H | CH₂CH₃ |
| X.007 | CH₃ | C-H | H | C-H | phenyl |
| X.008 | CH₃ | C-H | CH₃ | C-H | CH₃ |
| X.009 | CH₃ | C-H | CH₂CH₃ | C-H | CH₃ |
| X.010 | CH₃ | C-H | cyclopropyl | C-H | CH₃ |
| X.011 | CH₃ | C-H | CH₃-C≡ C | C-H | CH₃ |
| X.012 | CH₃ | C-H | H-C≡ C | C-H | CH₃ |
| X.013 | CH₃ | C-H | O-CH₃ | C-H | CH₃ |
| X.014 | CH₃ | C-H | S-CH₃ | C-H | CH₃ |
| X.015 | CH₃ | C-H | Cl | C-H | CH₃ |
| X.016 | CH₃ | C-H | N- (CH₂CH₃)₂ | C-H | CH₃ |
| X.017 | CH₃ | N | CH₃ | C-H | CH₃ |
| X.018 | CH₃ | N | CH₂CH₃ | C-H | CH₃ |
| X.019 | CH₃ | N | cyclopropyl | C-H | CH₃ |
| X.020 | CH₃ | N | CH₃-C≡ C | C-H | CH₃ |
| X.021 | CH₃ | N | H-C≡ C | C-H | CH₃ |
| X.022 | CH₃ | N | O-CH₃ | C-H | CH₃ |
| X.023 | CH₃ | N | S-CH₃ | C-H | CH₃ |
| X.024 | CH₃ | N | Cl | C-H | CH₃ |
| X.025 | CH₃ | N | N- (CH₂CH₃)₂ | C-H | CH₃ |
| X.026 | CH₃ | C-H | CH₃ | N | CH₃ |
| X.027 | CH₃ | C-H | CH₂CH₃ | N | CH₃ |
| X.028 | CH₃ | C-H | cyclopropyl | N | CH₃ |
| X.029 | CH₃ | C-H | CH₃-C≡ C | N | CH₃ |
| X.030 | CH₃ | C-H | H-C≡ C | N | CH₃ |
| X.031 | CH₃ | C-H | O-CH₃ | N | CH₃ |
| X.032 | CH₃ | C-H | S-CH₃ | N | CH₃ |
| X.033 | CH₃ | C-H | Cl | N | CH₃ |
| X.034 | CH₃ | C-H | N- (CH₂CH₃)₂ | N | CH₃ |
| X.035 | O-CH₃ | C-H | CH₃ | C-H | CH₃ |
| X.036 | O-CH₃ | C-H | CH₂CH₃ | C-H | CH₃ |
| X.037 | O-CH₃ | C-H | cyclopropyl | C-H | CH₃ |
| X.038 | O-CH₃ | C-H | CH₃-C≡ C | C-H | CH₃ |
| X.039 | O-CH₃ | C-H | H-C≡ C | C-H | CH₃ |
| X.040 | O-CH₃ | C-H | O-CH₃ | C-H | CH₃ |
| X.041 | O-CH₃ | C-H | S-CH₃ | C-H | CH₃ |
| X.042 | O-CH₃ | C-H | Cl | C-H | CH₃ |
| X.043 | O-CH₃ | C-H | N- (CH₂CH₃)₂ | C-H | CH₃ |
| X.044 | O-CH₃ | N | CH₃ | C-H | CH₃ |
| X.045 | O-CH₃ | N | CH₂CH₃ | C-H | CH₃ |
| X.046 | O-CH₃ | N | cyclopropyl | C-H | CH₃ |
| X.047 | O-CH₃ | N | CH₃-C≡ C | C-H | CH₃ |
| X.048 | O-CH₃ | N | H-C≡ C | C-H | CH₃ |
| X.049 | O-CH₃ | N | O-CH₃ | C-H | CH₃ |
| X.050 | O-CH₃ | N | S-CH₃ | C-H | CH₃ |
| X.051 | O-CH₃ | N | Cl | C-H | CH₃ |
| X.052 | O-CH₃ | N | N- (CH₂CH₃)₂ | C-H | CH₃ |
| X.053 | O-CH₃ | C-H | CH₃ | N | CH₃ |
| X.054 | O-CH₃ | C-H | CH₂CH₃ | N | CH₃ |
| X.055 | O-CH₃ | C-H | cyclopropyl | N | CH₃ |
| X.056 | O-CH₃ | C-H | CH₃-C≡ C | N | CH₃ |
| X.057 | O-CH₃ | C-H | H-C≡ C | N | CH₃ |
| X.058 | O-CH₃ | C-H | O-CH₃ | N | CH₃ |
| X.059 | O-CH₃ | C-H | S-CH₃ | N | CH₃ |
| X.060 | O-CH₃ | C-H | Cl | N | CH₃ |
| X.061 | O-CH₃ | C-H | N- (CH₂CH₃)₂ | N | CH₃ |
| X.062 | S-CH₃ | C-H | CH₃ | C-H | CH₃ |
| X.063 | S-CH₃ | C-H | CH₂CH₃ | C-H | CH₃ |
| X.064 | S-CH₃ | C-H | cyclopropyl | C-H | CH₃ |
| X.065 | S-CH₃ | C-H | CH₃-C≡ C | C-H | CH₃ |
| X.066 | S-CH₃ | C-H | H-C≡ C | C-H | CH₃ |
| X.067 | S-CH₃ | C-H | O-CH₃ | C-H | CH₃ |
| X.068 | S-CH₃ | C-H | S-CH₃ | C-H | CH₃ |
| X.069 | S-CH₃ | C-H | Cl | C-H | CH₃ |
| X.070 | S-CH₃ | C-H | N- (CH₂CH₃)₂ | C-H | CH₃ |
| X.071 | S-CH₃ | N | CH₃ | C-H | CH₃ |
| X.072 | S-CH₃ | N | CH₂CH₃ | C-H | CH₃ |
| X.073 | S-CH₃ | N | cyclopropyl | C-H | CH₃ |
| X.074 | S-CH₃ | N | CH₃-C≡ C | C-H | CH₃ |
| X.075 | S-CH₃ | N | H-C≡ C | C-H | CH₃ |
| X.076 | S-CH₃ | N | O-CH₃ | C-H | CH₃ |
| X.077 | S-CH₃ | N | S-CH₃ | C-H | CH₃ |
| X.078 | S-CH₃ | N | Cl | C-H | CH₃ |
| X.079 | S-CH₃ | N | N- (CH₂CH₃)₂ | C-H | CH₃ |
| X.080 | S-CH₃ | C-H | CH₃ | N | CH₃ |
| X.081 | S-CH₃ | C-H | CH₂CH₃ | N | CH₃ |
| X.082 | S-CH₃ | C-H | cyclopropyl | N | CH₃ |
| X.083 | S-CH₃ | C-H | CH₃-C≡ C | N | CH₃ |
| X.084 | S-CH₃ | C-H | H-C≡ C | N | CH₃ |
| X.085 | S-CH₃ | C-H | O-CH₃ | N | CH₃ |
| X.086 | S-CH₃ | C-H | S-CH₃ | N | CH₃ |
| X.087 | S-CH₃ | C-H | Cl | N | CH₃ |
| X.088 | S-CH₃ | C-H | N- (CH₂CH₃)₂ | N | CH₃ |
| X.089 | CH₃ | C-H | CH₃ | C-CH₂-CH₂-CH₂ | |
| X.090 | CH₃ | C-H | CH₂CH₃ | C-CH₂-CH₂-CH₂ | |
| X.091 | CH₃ | C-H | cyclopropyl | C-CH₂-CH₂-CH₂ | |
| X.092 | CH₃ | C-H | CH₃-C≡ C | C-CH₂-CH₂-CH₂ | |
| X.093 | CH₃ | C-H | H-C≡ C | C-CH₂-CH₂-CH₂ | |
| X.094 | CH₃ | C-H | O-CH₃ | C-CH₂-CH₂-CH₂ | |
| X.095 | CH₃ | C-H | S-CH₃ | C-CH₂-CH₂-CH₂ | |
| X.096 | CH₃ | C-H | Cl | C-CH₂-CH₂-CH₂ | |
| X.097 | CH₃ | C-H | N- (CH₂CH₃)₂ | C-CH₂-CH₂-CH₂ | |
| X.098 | CH₃ | N | CH₃ | C-CH₂-CH₂-CH₂ | |
| X.099 | CH₃ | N | CH₂CH₃ | C-CH₂-CH₂-CH₂ | |
| X.100 | CH₃ | N | cyclopropyl | C-CH₂-CH₂-CH₂ | |
| X.101 | CH₃ | N | CH₃-C≡ C | C-CH₂-CH₂-CH₂ | |
| X.102 | CH₃ | N | H-C≡ C | C-CH₂-CH₂-CH₂ | |
| X.103 | CH₃ | N | O-CH₃ | C-CH₂-CH₂-CH₂ | |
| X.104 | CH₃ | N | S-CH₃ | C-CH₂-CH₂-CH₂ | |
| X.105 | CH₃ | N | Cl | C-CH₂-CH₂-CH₂ | |
| X.106 | CH₃ | N | N- (CH₂CH₃)₂ | C-CH₂-CH₂-CH₂ | |
| X.107 | O-CH₃ | C-H | CH₃ | C-CH₂-CH₂-CH₂ | |
| X.108 | O-CH₃ | C-H | CH₂CH₃ | C-CH₂-CH₂-CH₂ | |
| X.109 | O-CH₃ | C-H | cyclopropyl | C-CH₂-CH₂-CH₂ | |
| X.110 | O-CH₃ | C-H | CH₃-C≡C | C-CH₂-CH₂-CH₂ | |
| X.111 | O-CH₃ | C-H | H-C≡C | C-CH₂-CH₂-CH₂ | |
| X.112 | O-CH₃ | C-H | O-CH₃ | C-CH₂-CH₂-CH₂ | |
| X.113 | O-CH₃ | C-H | S-CH₃ | C-CH₂-CH₂-CH₂ | |
| X.114 | O-CH₃ | C-H | Cl | C-CH₂-CH₂-CH₂ | |
| X.115 | O-CH₃ | C-H | N- (CH₂CH₃)₂ | C-CH₂-CH₂-CH₂ | |
| X.116 | O-CH₃ | N | CH₃ | C-CH₂-CH₂-CH₂ | |
| X.117 | O-CH₃ | N | CH₂CH₃ | C-CH₂-CH₂-CH₂ | |
| X.118 | O-CH₃ | N | cyclopropyl | C-CH₂-CH₂-CH₂ | |
| X.119 | O-CH₃ | N | CH₃-C≡C | C-CH₂-CH₂-CH₂ | |
| X.120 | O-CH₃ | N | H-C≡ C | C-CH₂-CH₂-CH₂ | |
| X.121 | O-CH₃ | N | O-CH₃ | C-CH₂-CH₂-CH₂ | |
| X.122 | O-CH₃ | N | S-CH₃ | C-CH₂-CH₂-CH₂ | |
| X.123 | O-CH₃ | N | Cl | C-CH₂-CH₂-CH₂ | |
| X.124 | O-CH₃ | N | N- (CH₂CH₃)₂ | C-CH₂-CH₂-CH₂ | |
| X.125 | S-CH₃ | C-H | CH₃ | C-CH₂-CH₂-CH₂ | |
| X.126 | S-CH₃ | C-H | CH₂CH₃ | C-CH₂-CH₂-CH₂ | |
| X.127 | S-CH₃ | C-H | cyclopropyl | C-CH₂-CH₂-CH₂ | |
| X.128 | S-CH₃ | C-H | CH₃-C≡ C | C-CH₂-CH₂-CH₂ | |
| X.129 | S-CH₃ | C-H | H-C≡ C | C-CH₂-CH₂-CH₂ | |
| X.130 | S-CH₃ | C-H | O-CH₃ | C-CH₂-CH₂-CH₂ | |
| X.131 | S-CH₃ | C-H | S-CH₃ | C-CH₂-CH₂-CH₂ | |
| X.132 | S-CH₃ | C-H | Cl | C-CH₂-CH₂-CH₂ | |
| X.133 | S-CH₃ | C-H | N- (CH₂CH₃)₂ | C-CH₂-CH₂-CH₂ | |
| X.134 | S-CH₃ | N | CH₃ | C-CH₂-CH₂-CH₂ | |
| X.135 | S-CH₃ | N | CH₂CH₃ | C-CH₂-CH₂-CH₂ | |
| X.136 | S-CH₃ | N | cyclopropyl | C-CH₂-CH₂-CH₂ | |
| X.137 | S-CH₃ | N | CH₃-C≡ C | C-CH₂-CH₂-CH₂ | |
| X.138 | S-CH₃ | N | H-C≡ C | C-CH₂-CH₂-CH₂ | |
| X.139 | S-CH₃ | N | O-CH₃ | C-CH₂-CH₂-CH₂ | |
| X.140 | O-CH₃ | N | S-CH₃ | C-CH₂-CH₂-CH₂ | |
| X.141 | O-CH₃ | N | Cl | C-CH₂-CH₂-CH₂ | |
| X.142 | O-CH₃ | N | N- (CH₂CH₃)₂ | C-CH₂-CH₂-CH₂ | |
| X.143 | CH₂CH₃ | C-H | H | C-CH₂-CH₂-CH₂ | |
| X.144 | CH₂CH₃ | N | H | C-CH₂-CH₂-CH₂ | |
| X.145 | CH₃-C≡ C | C-H | H | C-CH₂-CH₂-CH₂ | |
| X.146 | CH₃-C≡ C | N | H | C-CH₂-CH₂-CH₂ | |
| X.147 | cyclopropyl | C-H | H | C-CH₂-CH₂-CH₂ | |
| X.148 | cyclopropyl | N | H | C-CH₂-CH₂-CH₂ | |
| X.149 | phenyl | C-H | H | C-CH₂-CH₂-CH₂ | |
| X.150 | phenyl | N | H | C-CH₂-CH₂-CH₂ | |
| X.151 | 2-CH₃-phenyl | C-H | H | C-CH₂-CH₂-CH₂ | |
| X.152 | 2-CH₃-phenyl | N | H | C-CH₂-CH₂-CH₂ | |
| X.153 | Cl | C-H | H | C-CH₂-CH₂-CH₂ | |
| X.154 | Cl | N | H | C-CH₂-CH₂-CH₂ | |
| X.155 | F | C-H | H | C-CH₂-CH₂-CH₂ | |
| X.156 | F | N | H | C-CH₂-CH₂-CH₂ | |
| X.157 | Br | C-H | H | C-CH₂-CH₂-CH₂ | |
| X.158 | Br | N | H | C-CH₂-CH₂-CH₂ | |
| X.159 | CH₂CH₃ | C-H | H | C-H | CH₃ |
| X.160 | CH₂CH₃ | N | H | C-H | CH₃ |
| X.161 | CH₂CH₃ | C-H | H | N | CH₃ |
| X.162 | CH₃-C≡ C | C-H | H | C-H | CH₃ |
| X.163 | CH₃-C≡ C | N | H | C-H | CH₃ |
| X.164 | CH₃-C≡ C | C-H | H | N | CH₃ |
| X.165 | cyclopropyl | C-H | H | C-H | CH₃ |
| X.166 | cyclopropyl | N | H | C-H | CH₃ |
| X.167 | cyclopropyl | C-H | H | N | CH₃ |
| X.168 | phenyl | C-H | H | C-H | CH₃ |
| X.169 | phenyl | N | H | C-H | CH₃ |
| X.170 | phenyl | C-H | H | N | CH₃ |
| X.171 | 2-CH₃-phenyl | C-H | H | C-H | CH₃ |
| X.172 | 2-CH₃-phenyl | N | H | C-H | CH₃ |
| X.173 | 2-CH₃-phenyl | C-H | H | N | CH₃ |
| X.174 | Cl | C-H | H | C-H | CH₃ |
| X.175 | Cl | N | H | C-H | CH₃ |
| X.176 | Cl | C-H | H | N | CH₃ |
| X.177 | F | C-H | H | C-H | CH₃ |
| X.178 | F | N | H | C-H | CH₃ |
| X.179 | F | C-H | H | N | CH₃ |
| X.180 | Br | C-H | H | C-H | CH₃ |
| X.181 | Br | N | H | C-H | CH₃ |
| X.182 | Br | C-H | H | N | CH₃ |

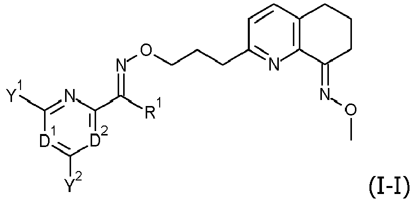

Wherein Y¹, Y², D¹, D² and R¹ have the specific meanings given in the table.

Table 2: This table discloses compounds 1.01 to 1.182 of the formula (I-II)

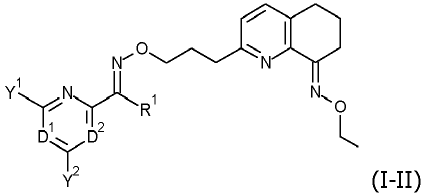

Wherein Y¹, Y², D¹, D² and R¹ have the specific meanings given in the table.

Table 3: This table discloses compounds 1.01 to 1.182 of the formula (I-III)

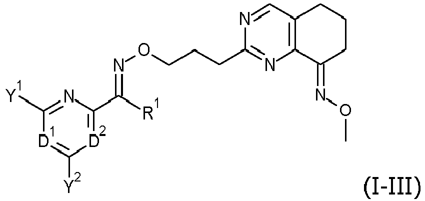

Wherein Y¹, Y², D¹, D² and R¹ have the specific meanings given in the table.

Table 4: This table discloses compounds 1.01 to 1.182 of the formula (I-IV)

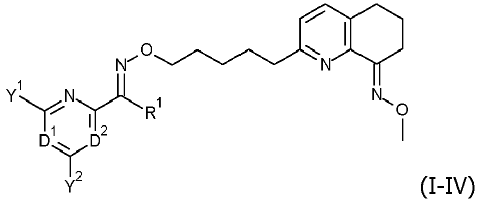

Wherein Y¹, Y², D¹, D² and R¹ have the specific meanings given in the table.

Table 5: This table discloses compounds 1.01 to 1.182 of the formula (I-V)

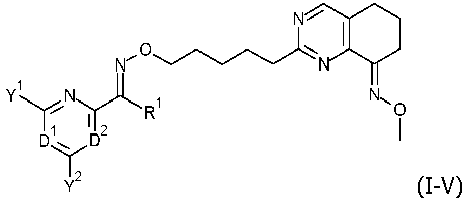

Wherein Y¹, Y², D¹, D² and R¹ have the specific meanings given in the table.

The compounds in Tables 1 to 5 include all isomers, tautomers and mixtures thereof, including the cis/trans isomers shown above.

The compounds of the invention may be made by a variety of methods, illustrated in schemes 1-5 and 8. Schemes 6-7 and 9-17 illustrate methods of obtaining important intermediates in the synthesis of the compounds of the invention.The compounds depicted in the schemes also indicate any isomers and tautomers, in particular the geometric isomers arising from the oxime and oxime ether moieties. 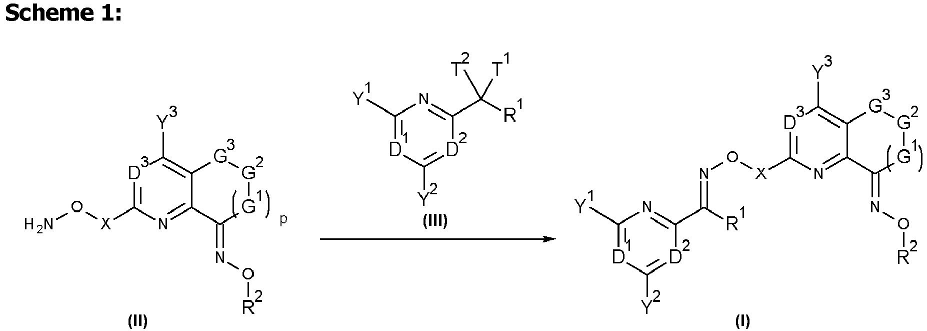 1) Compounds of formula (I) may be prepared by reacting a compound of formula (II) wherein R², G¹, G², G³, Y⁶, D³ and p are as described herein for compounds of formula (I) with a compound of formula (III), wherein T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and R¹, Y¹, Y³, D¹, D², are as defined herein for compounds of formula (I), as seen in **scheme 1.**
A general description of condensation reactions is given below, and typical reaction conditions for this type of reaction may be found in Journal of Organic Chemistry, 52(22), 4978-84; 1987; Chemical & Pharmaceutical Bulletin, 51(2), 138-151; 2003; Organic Letters, 10(2), 285-288; 2008; Journal of the American Chemical Society, 130(12), 4196-4201; 2008; Chemistry & Biology, 9(1), 113-129; 2002; Organic Preparations and Procedures International, 32(2), 153-159; 2000; Scientia Pharmaceutica, 66(1), 9-21; 1998, Journal of Medicinal Chemistry, 49(17), 5177-5186; 2006, Journal of Agricultural and Food Chemistry, 38(3), 839-44; 1990; Tetrahedron: Asymmetry, 8(2), 253-263; 1997; Journal of Medicinal Chemistry, 44(21), 3339-3342; 2001; Bioorganic & Medicinal Chemistry Letters, 12(3), 341-344; 2002; US 2007032470; WO 07/058504; Journal of Organic Chemistry, 73(5), 2007-2010; 2008; Bioorganic & Medicinal Chemistry Letters, 19(10), 2683-2687; 2009; and Bioorganic & Medicinal Chemistry Letters, 19(10), 2654-2660; 2009. 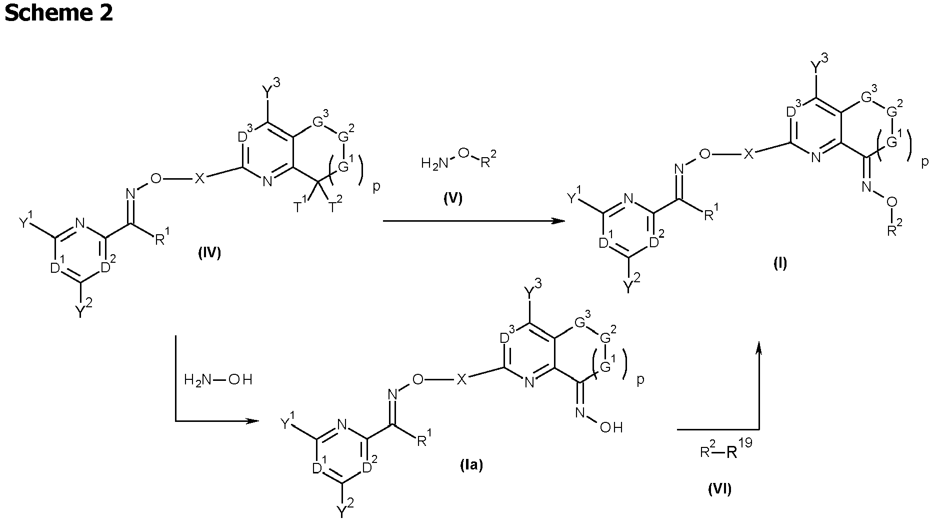 2) Alternatively, as seen in **scheme 2,** compounds of formula (I) may be prepared by reacting a compound of formula (IV), wherein D¹, D², D³, p, X, Y¹, Y², Y³ and R¹ are as defined herein for compounds of formula (I) and T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl, and a compound of formula (V), wherein R² is as defined herein for compounds of formula (I) under conditions described in procedure 1.
3) Alternatively, as seen in **scheme 2,** compounds of formula (I) may be prepared by reacting a compound of formula (Ia), that is a compound of formula (I) wherein R² is hydrogen, and a compound of formula (VI), wherein R² is as defined herein for compounds of formula (I) and R¹⁹ is a halogen, in particular chlorine, bromine or iodine, or a sulfonic acid ester group, such as mesylate, tosylate, triflate, phenylsulfonic acid ester, nitro-phenylsulfonic acid ester, or nonafluorobutylsulfonic acid ester.
Typical reaction conditions for alkylation reactions such as this may be found below. These are further illustrated in Chinese Journal of Chemistry, 27(1), 33-42; 2009; WO 09/049846; Journal of Antibiotics, 61(10), 603-614; 2008; Bioorganic & Medicinal Chemistry Letters, 18(24), 6471-6475; 2008; Journal of Medicinal Chemistry, 51(15), 4601-4608; 2008; WO 06/123145, Archiv der Pharmazie (Weinheim, Germany), 340(4), 202-208; 2007; Synthetic Communications, 37(7), 1155-1165; 2007; Russian Journal of Organic Chemistry, 42(5), 735-738; 2006; Bioinorganic Chemistry and Applications, 1(3-4), 299-308; 2003; Synthetic Communications, 28(14), 2621-2633; 1998; Synthetic Communications, 19(18), 3129-38; 1989.
4) Oximes of formula (Ia) may be obtained by a condensation reaction, whereby a compound of formula (IV) is reacted with hydroxylamine, or, alternatively, with a salt of hydroxylamine. A more detailed description of condensation reactions is given below.
Related references include the following: Journal of Heterocyclic Chemistry, 46(1), 116-118; 2009; Journal of Medicinal Chemistry, 20(5), 718-21; 1977; Journal of Organic Chemistry, 73(11), 4017-4026; 2008; EJEAFChe, Electronic Journal of Environmental, Agricultural and Food Chemistry, 5(5), 1515-1521; 2006; Advanced Synthesis & Catalysis, 346(13-15), 1798-1811; 2004. 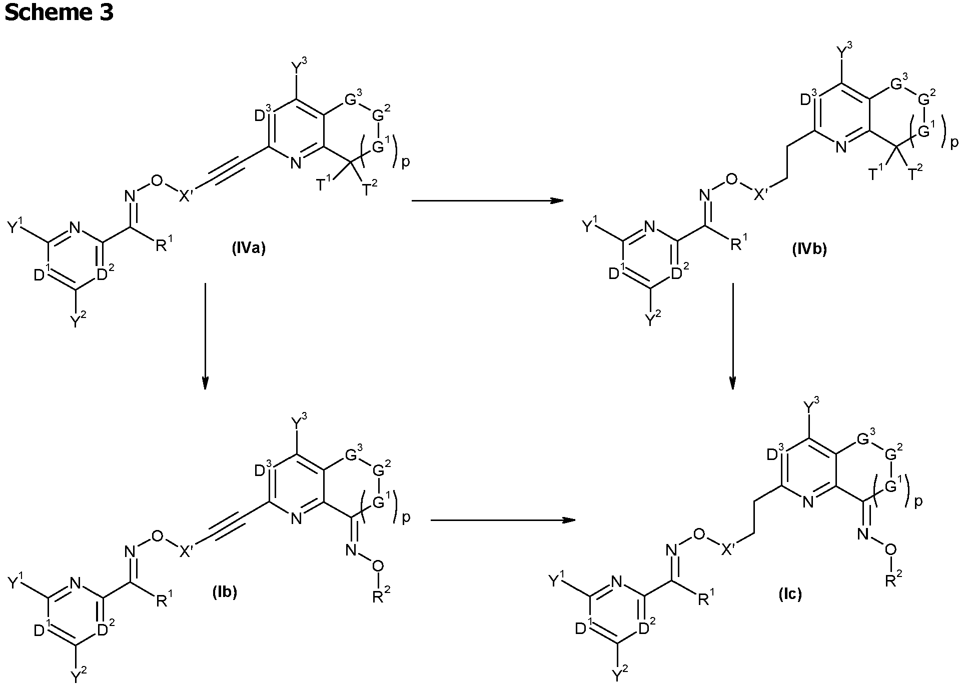 5) Alternatively, as seen in **scheme 3,** compounds of formula (Ic), that is a compound of formula (I) wherein X is X-3, X-4 or X-5, wherein Z⁴, Z⁵, Z⁸, Z⁹, Z¹³ and Z¹⁴ are methylene and Z³, Z⁶, Z⁷, Z¹⁰, Z¹¹ and Z¹² (represented in the scheme collectively as X') are as defined herein for compounds of formula (I), may be prepared by catalytic hydrogenation from compounds of formula (Ib), that is a compound of formula (I) wherein X is X-3, X-4 or X-5, and wherein Z⁴ and Z⁵, Z⁸ and Z⁹ or Z¹³ and Z¹⁴ together form -C=C- and Z³, Z⁶, Z⁷, Z¹⁰, Z¹¹ and Z¹² (represented in the scheme collectively as X') are as defined herein for compounds of formula (I)) in the presence of a metal catalyst, for example palladium, nickel or platinum. The reaction is usually carried out in the presence of a solvent under a hydrogen atmosphere. In some cases it is necessary to apply pressure in the range of 1 to 100 bar. Suitable solvents for such reactions are alcohols, such as methanol or ethanol, cyclic ethers, such as dioxane or tetrahydrofuran or esters like ethyl acetate. The reaction is usually carried out at a reaction temperature ranging from 0°C to the boiling point of the solvent. Examples for the hydrogenation in the presence of a nickel catalyst can be found in Journal of Organometallic Chemistry, 333(2), 139-53; 1987. Examples for the hydrogenation in the presence of a palladium catalyst can be found in Tetrahedron, 63(26), 6015-6034; 2007 or in Bioorganic & Medicinal Chemistry, 9(11), 2863-2870; 2001. Examples for the hydrogenation in the presence of a platinum catalyst can be found in Journal of Organic Chemistry, 53(2), 386-90; 1988 or in Journal of Medicinal Chemistry, 32(8), 1820-35; 1989
6) Compounds of formula (Ib) may be obtained by condensation reaction from compounds of formula (IVa), that is a compound of formula (IV) wherein X is X-3, X-4 or X-5, wherein Z⁴ and Z⁵, Z⁸ and Z⁹ or Z¹³ and Z¹⁴ together form -C=C- and Z³, Z⁶, Z⁷, Z¹⁰, Z¹¹ and Z¹² (represented in the scheme collectively as X') are as defined herein for compounds of formula (I). The reaction can be carried out analogously to procedure 2 or procedures 3 and 4 as shown in **Scheme 2.**
7) Alternatively, as seen in **scheme 3,** compounds of formula (Ic) may be obtained from compounds of formula (IVb), that is a compound of formula (IV) wherein X is X-3, X-4 or X-5, wherein Z⁴, Z⁵, Z⁸, Z⁹, Z¹³ and Z¹⁴ are methylene and Z³, Z⁶, Z⁷ Z¹⁰, Z¹¹ and Z¹² (represented in the scheme collectively as X') are as defined herein for compounds of formula (I). The reaction can be carried out analogously to procedure 2 or procedures 3 and 4 as shown in **Scheme 2.**
8) Compounds of formula (IVb) may be obtained from compounds of (IVa) by catalytic hydrogenation. The reaction can be carried out analogously to procedure 5 as shown in **Scheme 3.** 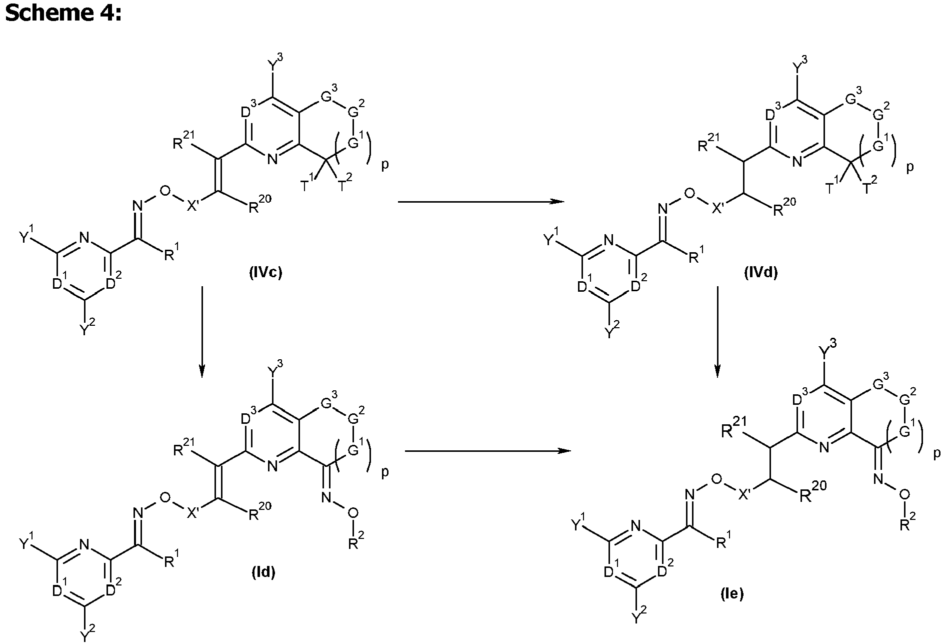 9) Alternatively, as seen in **scheme 4,** compounds of formula (Ie), that is a compound of formula I wherein X is X-3, X-4 or X-5 and wherein Z³, Z⁶, Z⁷, Z¹⁰, Z¹¹ and Z¹² are as defined herein for compounds of formula (I) (depicted in the scheme as X'); CHR²⁰ represents Z⁴, Z⁸ or Z¹³; CHR²¹ represents Z⁵, Z⁹ or Z¹⁴; R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy, may be prepared by catalytic hydrogenation from compound (Id), that is a compound of formula I wherein X is X-3, X-4 or X-5 and wherein Z³, Z⁶, Z⁷, Z¹⁰, Z¹¹ and Z¹² are as defined herein for compounds of formula (I) (depicted in the scheme as X'); R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy in the presence of a metal catalyst like for example palladium, nickel or platinum. The reaction is usually carried out in the presence of a solvent under a hydrogen atmosphere. In some cases it is necessary to apply pressure in the range of 1 to 100 bar. Suitable solvents for such reactions are alcohols, such as methanol or ethanol, cyclic ethers, such as dioxane or tetrahydrofuran or esters like ethyl acetate. The reaction is usually carried out at a reaction temperature ranging from 0°C to the boiling point of the solvent.
Examples for the hydrogenation in the presence of a nickel catalyst can be found in Journal of Organic Chemistry, 69(6), 1959-1966; 2004. Examples for the hydrogenation in the presence of a palladium catalyst can be found in Journal of Organic Chemistry, 74(16), 6072-6076; 2009. Examples for hydrogenation in the presence of a platinum catalyst can be found in Organometallics, 5(2), 348-55; 1986.
10) Compounds of formula (Id) may be obtained by condensation reaction from compounds of formula (IVc), that is a compound of formula (IV) wherein X is X-3, X-4 or X-5 and wherein X is X-3, X-4 or X-5 and wherein Z³, Z⁶, Z⁷, Z¹⁰, Z¹¹ and Z¹² are as defined herein for compounds of formula (I) (depicted in the scheme as X'); CHR²⁰ represents Z⁴, Z⁸ or Z¹³; CHR²¹ represents Z⁵, Z⁹ or Z¹⁴; R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy. The reaction can be carried out analogously to procedure 2 or procedures 3 and 4 as shown in **Scheme 2.**
11) Alternatively, as seen in **scheme 4,** compounds of formula (Ie) may be obtained from compounds of formula (IVd) that is a compound of formula IV wherein X is X-3, X-4 or X-5 and wherein Z³, Z⁶, Z⁷, Z¹⁰, Z¹¹ and Z¹² are as defined herein for compounds of formula (I) (depicted in the scheme as X'); CHR²⁰ represents Z⁴, Z⁸ or Z¹³; CHR²¹ represents Z⁵, Z⁹ or Z¹⁴; R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy. The reaction can be carried out analogously to procedure 2 or procedures 3 and 4 as shown in **Scheme 2.**
12) Compounds of formula (IVd) may be obtained from compounds of (IVc) by catalytic hydrogenation. The reaction can be carried out analogously to procedure 9 as shown in **Scheme 4.** 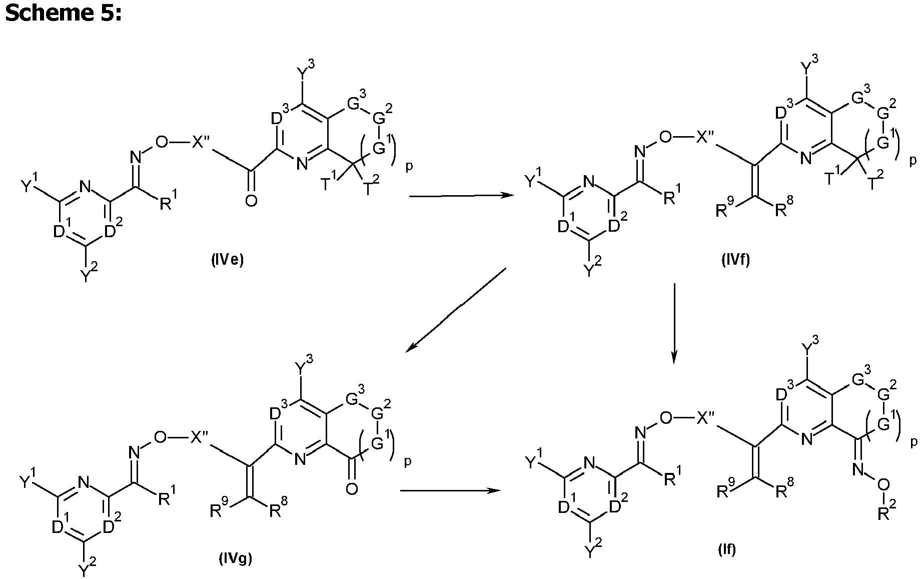 13) Alternatively, as seen in **scheme 5,** compounds of formula (If), that is a compound of formula (I) wherein Z² Z⁵, Z⁹ or Z¹⁴ represent C=CR⁸R⁹ (Z¹, Z³, Z⁴, Z⁶ Z⁷, Z⁸, Z¹⁰, Z¹¹, Z¹² and Z¹³ are depicted by X" in the scheme) may be obtained from compounds of formula (IVf), that is a compound of formula (I) wherein Z², Z⁵, Z⁹ or Z¹⁴ represent C=CR⁶R⁷ (Z¹, Z³, Z⁴, Z⁶, Z⁷, Z⁸, Z¹⁰, Z¹¹, Z¹² and Z¹³ are depicted by X" in the scheme) . The reaction can be carried out analogously to procedures procedure 2 or procedures 3 and 4 as shown in **Scheme 2.** In certain cases it may be appropriate to deprotect the ketone function first to obtain compounds of formula (IVg), that is a compound of formula (IVf) wherein T¹ and T² together with the carbon they are attached to form a carbonyl group.
14) Compounds of formula (IVf) may be obtained from compounds of (IVe), that is a compound of formula (IV) wherein Z², Z⁵, Z⁹ or Z¹⁴ represent C=O (Z¹, Z³, Z⁴ Z⁶, Z⁷ Z⁸, Z¹⁰, Z¹¹, Z¹² and Z¹³ are depicted by X" in the scheme). This can be done using one of several techniques well known to the person skilled in the art, including Wittig reaction or condensation reactions. The Wittig reaction comprises the reaction between an aldehyde or a ketone, for instance the ketone of formula (XIII), and a phosphorous ylide. Phosphorous ylides are usually prepared by treatment of a phosphonium salt with a base and phosphonium salts are usually prepared from a triarylphosphine and an alkyl halide. Several improvements and modification of the Wittig reaction are known and are described in the literature well known to the person skilled in the art, for example in March's Advanced Organic Chemistry: Reaction, Mechanisms and Structure, Sixth Edition, 2007 and references therein. Specific reaction conditions may be found in Journal of the American Chemical Society, 131(34), 12344-12353; 2009; Journal of Medicinal Chemistry, 51(22), 7193-7204; 2008 or Journal of Organic Chemistry, 74(11), 4166-4176; 2009. 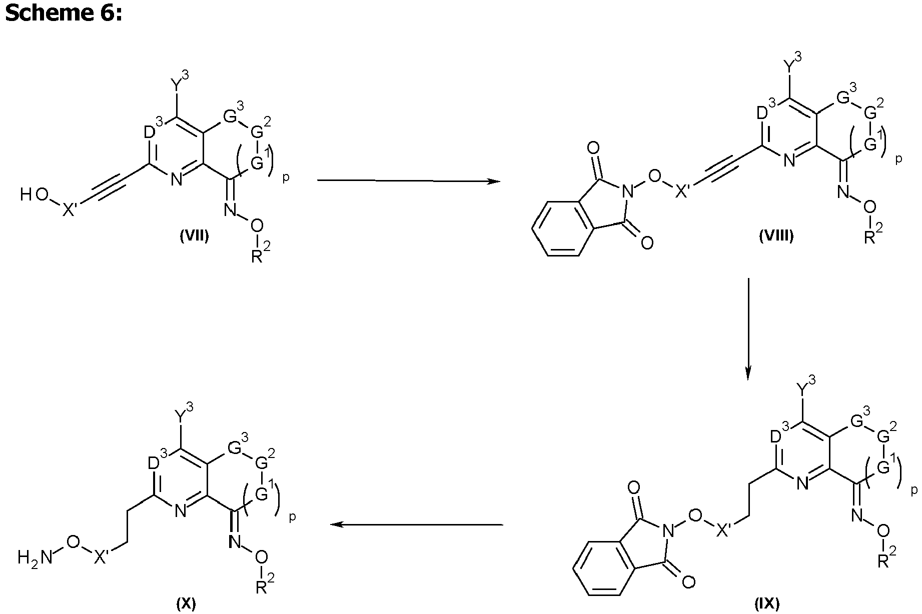 15) Compounds of formula (X) wherein X' represents X'-1, X'-2 or X'-3 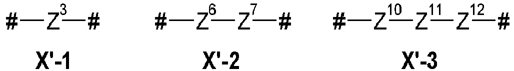 and wherein Z³ Z⁴, Z⁶, Z⁷, Z¹⁰, Z¹¹, Z¹², D³, Y³, R², G¹, G², G³ and p are as defined herein for a compound of formula (I) may be obtained from compounds of (IX) wherein X' represents X'-1, X'-2 or X'-3 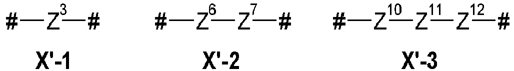 and wherein Z³, Z⁴, Z⁶, Z⁷, Z¹⁰, Z¹¹, Z¹², D³, Y³, R², G¹, G², G³ and p are as defined herein for a compound of formula (I) by cleavage of the phthalimide protecting group. Examples for such deprotections can be found in Greene, T. W., Wuts, P. G. N., Protective Groups in Organic Synthesis, John Wiley & Sons, Inc, 2006.
16) Compounds of formula (IX) may be obtained by catalytic hydrogenation from compounds of formula (VIII) wherein X' represents X'-1, X'-2 or X'-3 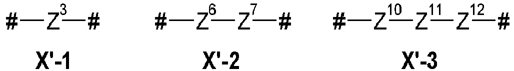 and wherein Z³, Z⁴, Z⁶, Z⁷, Z¹⁰, Z¹¹, Z¹², D³, Y³, R², G¹, G², G³ and p are as defined herein for a compound of formula (I). The reaction may be carried out analogously as shown in **Scheme 3** for procedure 5.
17) Compounds of formula (VIII) may be prepared from compounds of formula (VII) wherein X' represents X'-1, X'-2 or X'-3 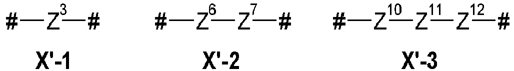 and wherein Z³, Z⁴, Z⁶, Z⁷, Z¹⁰, Z¹¹, Z¹², D³, Y³, R², G¹, G², G³ and p are as defined herein for a compound of formula (I) by a Mitsunobu reaction. The Mitsunobu reaction comprises the substitution of primary or secondary alcohols with nucleophiles, for example N-hydroxyphthalimide, as seen in **Scheme 6,** in the presence of a dialkyl azodicarboxylate and a trialkyl- or triaryl phosphine. Several improvements and modifications of the Mitsunobu reaction are known and are described in the literature and are well known to the person skilled in the art, for example in March's Advanced Organic Chemistry: Reaction, Mechanisms and Structure, Sixth Edition, 2007 and references therein. Specific reaction conditions may be found in Organic Preparations and Procedures International, 26(1), 111-13; 1994; Organic Letters, 11(9), 2019-2022; 2009; Tetrahedron Letters, 48(4), 647-650; 2007 or Journal of Organic Chemistry, 70(17), 6995-6998; 2005 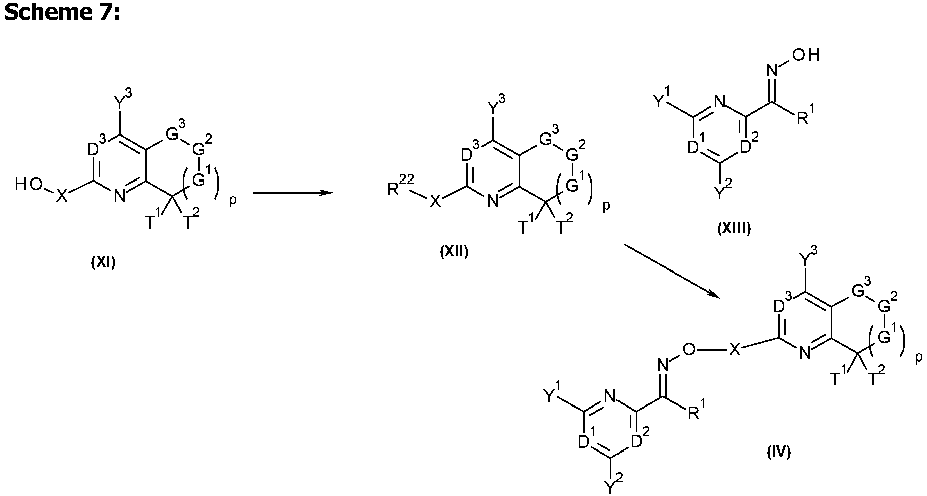 (18) Compounds of formula (IV) may be prepared by reacting compounds of formula (XII) wherein T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl, R²² is a halogen, in particular chlorine, bromine or iodine, or a sulfonic acid ester group, such as mesylate, tosylate, triflate, phenylsulfonic acid ester, nitro-phenylsulfonic acid ester, or nonafluorobutylsulfonic acid ester and D³, X, Y³, G¹, G², G³ and p are as defined herein for compounds of formula (I), with a compound of formula (XIII) wherein D¹, D², Y¹, Y² and R¹ are as defined herein for compounds of formula (I). The alkylation reaction can be carried out analogously to procedure 3 as shown in **Scheme 1.**
(19) Compounds of formula (XII) can be prepared from compounds of formula (XI), wherein D³, X, Y³, G¹, G², G³ and p are as defined herein for compounds of formula (I) and T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl. Such transformations can be affected using a number of conditions well known to the person skilled in the art. 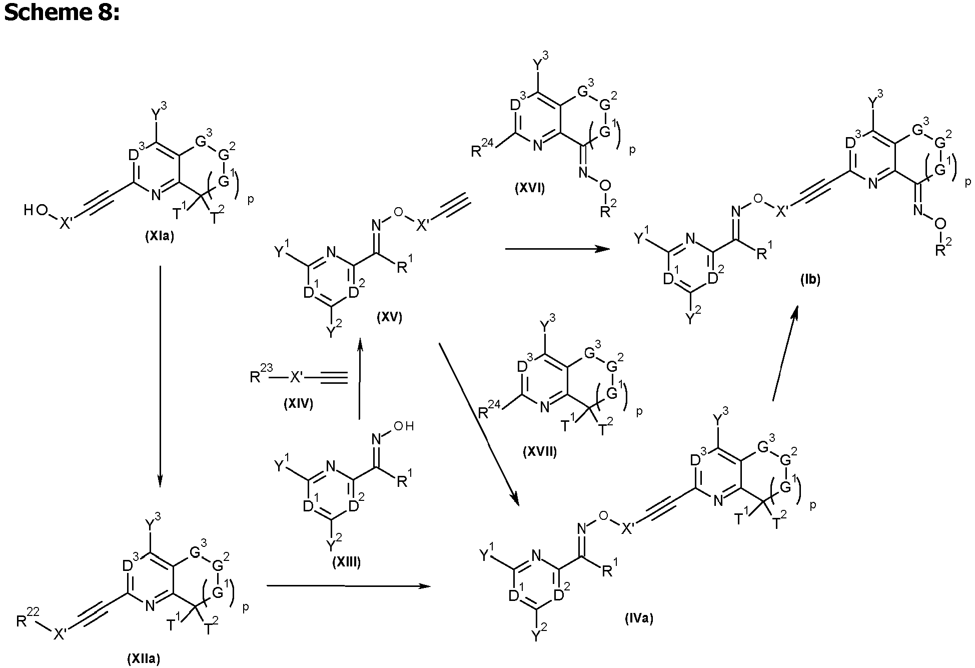 (20) Compounds of formula (Ib) may be prepared by a Sonogashira reaction from compounds of formula (XV) wherein X' represents X'-1, X'-2 or X'-3 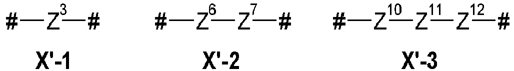 wherein Z³, Z⁴, Z⁶, Z⁷, Z¹⁰, Z¹¹, Z¹², R¹, D¹, D², Y¹ and Y² are as defined herein for compounds of formula (I) with compounds of formula (XVI) wherein R²⁴ is Cl, Br, I or a sulfonate and R², D³, Y³, G¹, G², G³ and p are as defined herein for compounds of formula (I); The reaction can be carried out in the presence of a palladium catalyst, for example tetrakis triphenyphosphine palladium (0) or dichlorobis (triphenylphosphine) palladium(II), a copper(I) salt like copper (I) chloride; copper (I) bromide or copper (I) iodide and a base, for example triethylamine, ethyldiisopropylamine, diethylamine, diisopropylamine or dicyclohexylamine. Where possible, the base may also serve as solvent. Examples for other suitable solvents are N,N-dimethylformamide, N,N-dimethylacetamide, acetonitrile, dimethylsulfoxide, dioxane or tetrahydrofuran. The reaction is usually carried out at a reaction temperature ranging from 0°C to the boiling point of the solvent. Examples of Sonogashira reactions can be found in Handbook of Organopalladium Chemistry for Organic Synthesis 2002,1, 493-529.
(21) Compounds of formula (XV) may be prepared by an alkylation reaction from compounds of formula (XIII) wherein R¹, D¹, D², Y¹ and Y² are as defined herein for compounds of formula (I) and compounds of (XIV) wherein X' represents X'-1, X'-2 or X'-3 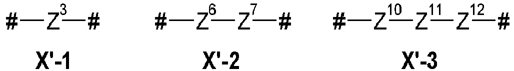 and wherein R²³ is a halogen, in particular chlorine, bromine or iodine, or a sulfonic acid ester group, such as mesylate, tosylate, triflate, phenylsulfonic acid ester, nitro-phenylsulfonic acid ester, or nonafluorobutylsulfonic acid ester and Z³, Z⁴, Z⁶, Z⁷, Z¹⁰, Z¹¹ and Z¹² are as defined herein for a compound of formula (I). Many of such compounds (XIV) are known in the literature and are commercially available or can be prepared by methods well known to the person skilled in the art.
(22) Alternatively as seen in **Scheme 8** compounds of formula (Ib) may be prepared from compounds of formula (IVa). This conversion is already described in **Scheme 3,** procedure 6.
(23) Compounds of formula (IVa) may be prepared from compounds of formula (XIIa) that is a compound of formula (XII) wherein X is X-3, X-4 or X-5, and wherein Z⁴ and Z⁵, Z⁸ and Z⁹ or Z¹³ and Z¹⁴ together form -C≡C- and Z³, Z⁶, Z⁷, Z¹⁰, Z¹¹ and Z¹² (represented in the scheme collectively as X') are as defined herein for compounds of formula (I) and compounds of formula (XIII). The alkylation reaction can be carried out analogously as shown in **Scheme 2** for procedure 3.
(24) Compounds of formula (XIIa) can be prepared from compounds of formula (XIa), that is a compound of formula (XI) wherein X is X-3, X-4 or X-5, and wherein Z⁴ and Z⁵, Z⁸ and Z⁹ or Z¹³ and Z¹⁴ together form -C≡C- and Z³, Z⁶, Z⁷, Z¹⁰, Z¹¹ and Z¹² (represented in the scheme collectively as X') are as defined herein for compounds of formula (I). Such transformations can be effected using a number of conditions well known to the person skilled in the art.
(25) Alternatively compounds of (IVa) may be prepared by a Sonogashira reaction of compounds of formula (XVII) wherein R²⁴ is Cl, Br, I or a sulfonate; T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and D³, Y³, G¹, G², G³ and p are as defined herein for compounds of formula (I) with compounds of formula (XV). The Sonogashira reaction can be carried out analogously to procedure 20. 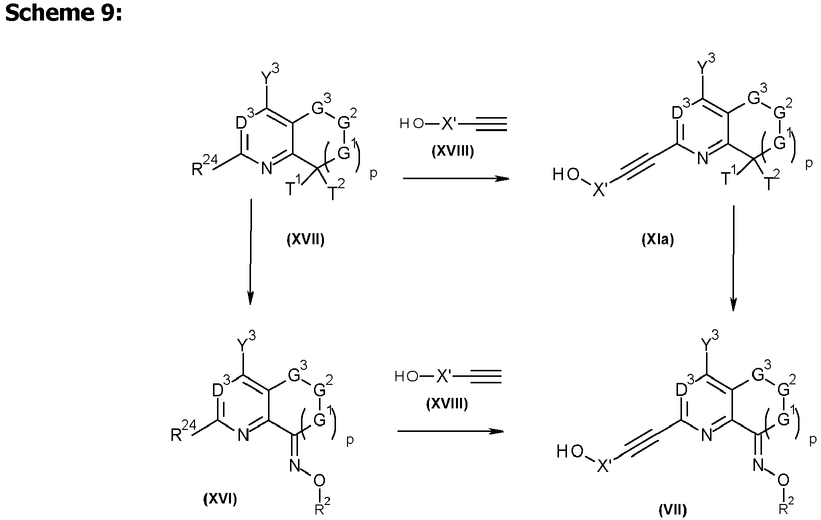 (26) Compounds of formula (VII) may be prepared by a Sonogashira reaction from compounds of formula (XVIII) wherein X' represents X'-1, X'-2 or X'-3 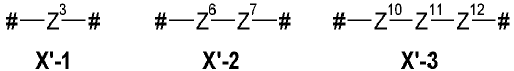 Z³, Z⁴, Z⁶, Z⁷, Z¹⁰, Z¹¹ and Z¹² are as defined herein for a compound of formula (I) and compounds of formula (XVI). The Sonogashira reaction can be carried out analogously to procedure 20 as shown in **Scheme 8.** It is obvious to the person skilled in the art that compounds of formula (VII) might be used to prepare compounds of formula (Ib) by using procedures similar to procedure 23 and procedure 24 as shown in **Scheme 8.**
(27) Compounds of formula (XVI) may be prepared by a condensation reaction from compounds of formula (XVII). The condensation reaction can be carried out analogously to procedure 2 or procedures 3 and 4 as shown in **Scheme 2.**
(28) Alternatively it might be advantageous to prepare compounds of formula (VII) from compounds of formula (XIa) by a condensation reaction analogously to procedure 27.
(29) Compounds of formula (XIa) may be prepared by a Sonogashira reaction from compounds of formula (XVIII) and compounds of (XVII). The Sonogashira reaction can be carried out analogously to procedure 22 as shown in **Scheme 8.** 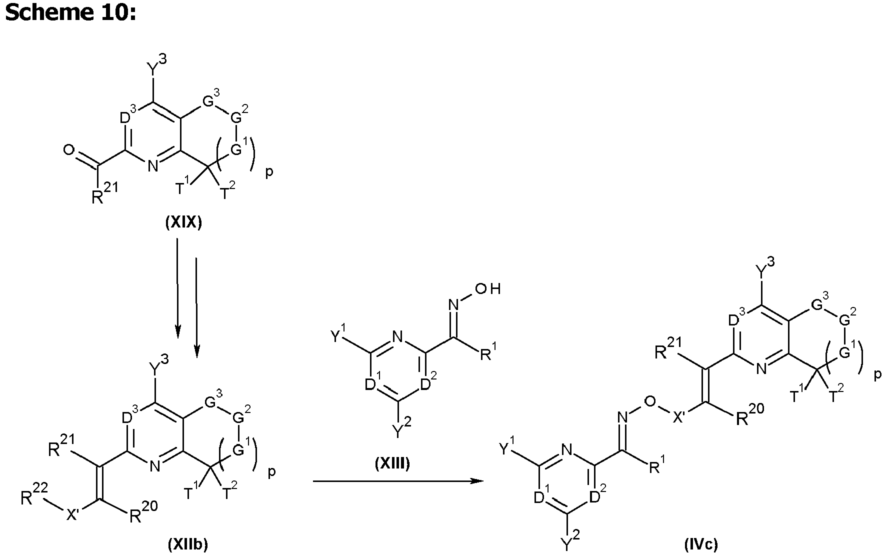 (25) Compounds of formula (IVc) may be prepared from compounds of formula (XIIb), that is a compound of formula (XII) wherein X is X-3, X-4 or X-5, and wherein Z⁴ and Z⁵, Z⁸ and Z⁹ or Z¹³ and Z¹⁴ together form -CR²⁰=CR²¹-, R²² is a halogen, in particular chlorine, bromine or iodine, or a sulfonic acid ester group, such as mesylate, tosylate, triflate, phenylsulfonic acid ester, nitro-phenylsulfonic acid ester, or nonafluorobutylsulfonic acid, T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and Z³, Z⁴, Z⁶, Z⁷, Z¹⁰, Z¹¹ and Z¹² (represented in the scheme collectively as X'), D³, Y³, G¹, G², G³ and p are as defined herein for compounds of formula (I) and compounds of formula (XIII). The alkylation reaction can be carried out analogously to procedure 3 as shown in **Scheme 2.**
(26) Compounds of formula (XIIb) may be prepared from compounds of formula (XIX) wherein R²¹ represents hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy; T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and D³, Y³, G¹, G², G³ and p are as defined herein for compounds of formula (I) in a multistep synthesis. This can be done using one of several techniques well known to the person skilled in the art, including Wittig reaction or Horner-Wadsworth Emmons reactions in the first step and further transformations as seen in **Scheme 11.** 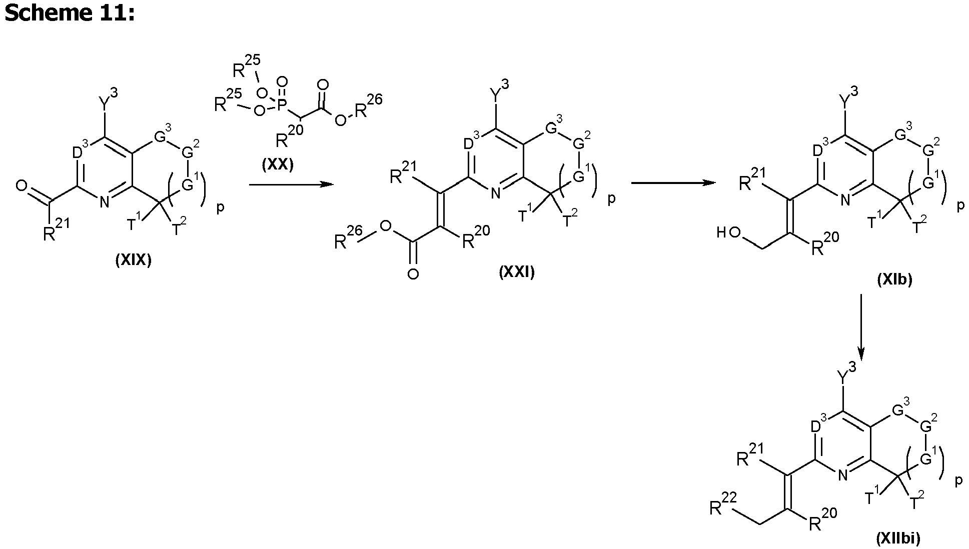 (27) Compounds of formula (XIIbi), that is a compound of formula (XIIb) wherein X' is X'-1, and Z³ is methylene may be prepared from compounds of formula (XIb) that is a compound of formula (XI) wherein X is X-3, and wherein Z⁴ and Z⁵ together form -CR²⁰=CR²¹-, wherein R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy. Such transformations can be effected using a number of conditions well known to the person skilled in the art.
(28) Compounds of formula (XIb) may be prepared from compounds of formula (XXI) wherein R²⁰ and R²¹ independently of one another represent hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy, R²⁶ represents C₁-C₄ alkyl and D³, Y³, G¹, G², G³ and p are as defined herein for compounds of formula (I) by reduction with a metal hydride, for example lithium aluminium hydride or diisobutyl aluminium hydride. Examples for such reductions can found in Journal of Combinatorial Chemistry, 7(6), 958-967; 2005. The reaction is usually carried out at temperatures between -100 to 20°C in the presence of a solvent.
(29) Compounds of formula (XXI) can be prepared from compounds of formula (XIX) and a phosphonate of formula (XX) wherein R²⁵ and each R²⁶ are independently selected from C₁-C₄ alkyl and R²⁰ represents hydrogen, halogen, C₁-C₄ alkyl, C₁-C₄ haloalkyl, phenyl or CN, wherein phenyl is optionally substituted by one or more groups, e.g. one to five groups, independently selected from halogen, CN, C₁-C₄ alkyl, C₁-C₄ haloalkyl, C₁-C₄ alkoxy and C₁-C₄ haloalkyoxy using a Horner-Wadsworth Emmons reaction. The reaction is carried out in the presence of a base. Appropriate bases are for example metal hydrides like calcium, lithium, sodium or potassium hydride, organometal compounds like buthyllithium or organic bases like for example triethylamine or ethyldiisopropylamine in combination with lithium chloride. Examples can be found in Bioorganic & Medicinal Chemistry, 11(18), 4015-4026; 2003; Synthesis, (4), 283-5; 1981 or in Journal of Medicinal Chemistry, 53(3), 1200-1210; 2010 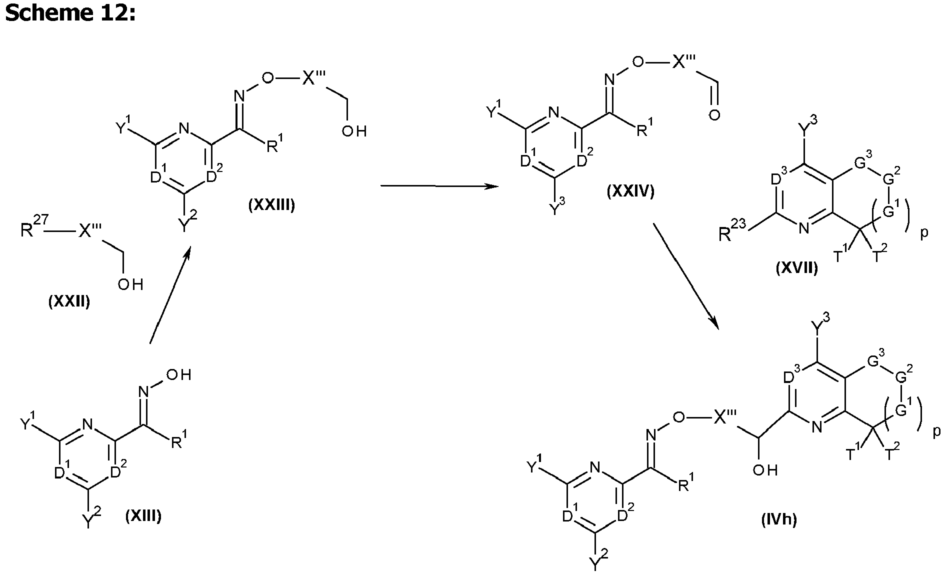 (30) Compounds of formula (IVh), that is a compound of formula (IV) wherein Z², Z⁵, Z⁹ or Z¹⁴ represent CH(OH) (Z¹, Z³, Z⁴, Z⁶, Z⁷, Z⁸, Z¹⁰, Z¹¹, Z¹² and Z¹³ are depicted by X''' in the scheme) may be prepared from aldehydes of formula (XXIV) wherein X''' represents X'''-1, X'''-2, X'''-3 or X'''-4 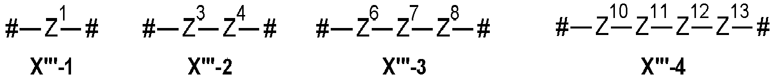 and wherein D¹, D², Y¹, Y³, R¹, Z¹, Z³, Z⁴, Z⁶, Z⁷, Z⁸, Z¹⁰, Z¹¹, Z¹² and Z¹³ are as defined for a compound of formula (I) and compounds of formula (XVII). Such a transformation may be performed by halogen metal exchange in compound (XVII) with an appropriate reagent like for example magnesium, isopropyl magnesium chloride, or n-buthyllithium and the reaction of this metalated pyridine intermediate with a compound of formula (XXIV).
Examples for such transformations can be found in Angewandte Chemie, International Edition, 43(25), 3333-3336; 2004; Organic Letters, 6(26), 4905-4907; 2004; Journal of the American Chemical Society, 130(38), 12592-12593; 2008 or in Organic Letters, 11(20), 4540-4543; 2009
Compounds of formula (IVh) are especially useful as intermediates to a number of other compounds, wherein the hydroxy group formed is transformed into other functional groups, for example acid halides. Such transformations can be effected using a number of conditions well known to the person skilled in the art.
(31) Compounds of formula (XXIV) may be prepared by oxidation from compounds of formula (XXVI) wherein X''' represents X'''-1, X"'-2, X'''-3 or X'''-4 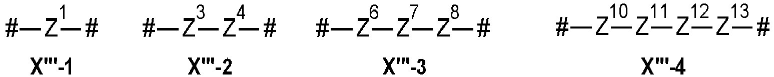 and wherein D¹, D², Y¹, Y², R¹, Z¹, Z³, Z⁴, Z⁶, Z⁷, Z⁸, Z¹⁰, Z¹¹, Z¹² and Z¹³ are as defined for a compound of formula (I). Such oxidations can be affected using a number of conditions well known to the person skilled in the art. Specific reaction conditions may be found in Organic & Biomolecular Chemistry, 6(21), 4036-4040; 2008; Bioorganic & Medicinal Chemistry Letters, 19(13), 3627-3631; 2009; Chemical Communications (Cambridge, United Kingdom), (37), 5618-5620; 2009; or in Synthesis, (1), 91-97; 2010.
(32) Compounds of formula (XXIII) may be prepared from compound of formula (XIII) and compounds of formula (XXII) wherein X''' represents X'''-1, X"-2 , X'''-3 or X'''-4 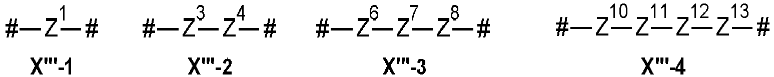 wherein Z¹, Z³, Z⁴, Z⁶, Z⁷, Z⁸, Z¹⁰, Z¹¹, Z¹² and Z¹³ are as defined herein for compounds of formula (I) and R²⁷ is a halogen, in particular chlorine, bromine or iodine, or a sulfonic acid ester group, such as mesylate, tosylate, triflate, phenylsulfonic acid ester, nitro-phenylsulfonic acid ester, or nonafluorobutylsulfonic acid ester. Many of such compounds are known in the literature and are commercially available or can be prepared by methods well known to the person skilled in the art. The alkylation reaction can be carried out analogously to procedure 3 as shown in **Scheme 2.** 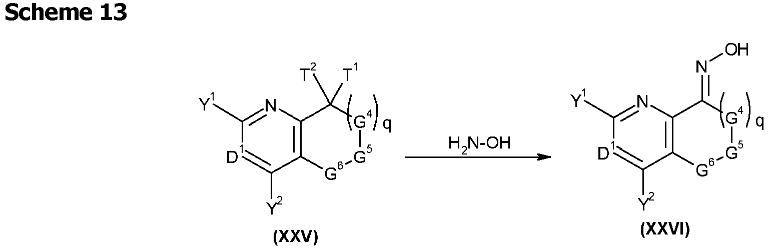 33) Oximes of formula (XXVI) wherein T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and D¹, Y¹, Y², G⁴, G⁵, G⁶ and q are as defined for a compound of formula (I) may be obtained by a condensation reaction, whereby a compound of formula (XXV), wherein T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl and D¹, Y¹, Y², G⁴, G⁵, G⁶ and q are as defined for a compound of formula (I) is reacted with hydroxylamine, or, alternatively, with a salt of hydroxylamine. A more detailed description of condensation processes is given below.
Related references include the following: Journal of Heterocyclic Chemistry, 46(1), 116-118; 2009; Journal of Medicinal Chemistry, 20(5), 718-21; 1977; Journal of Organic Chemistry, 73(11), 4017-4026; 2008; EJEAFChe, Electronic Journal of Environmental, Agricultural and Food Chemistry, 5(5), 1515-1521; 2006; Advanced Synthesis & Catalysis, 346(13-15), 1798-1811; 2004.
Some compounds of formula (XXV) are known and their preparation has been published or they are available commercially. A few typical examples are given in Table 6 together with the corresponding CAS numbers. Analogous protocols to those used to prepare the following compounds can be used to prepare other compounds of formula (XXV).

**Table 6**

| | | |
|---|---|---|
| 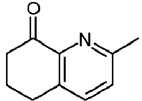 | 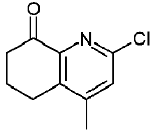 | 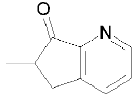 |
| 849643-01-2 | 904915-35-1 | 31170-79-3 |
| 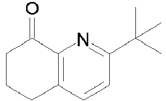 | 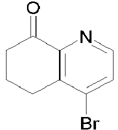 | 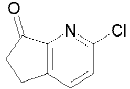 |
| 263566-88-7 | 1196155-16-4 | 1150617-92-7 |
| 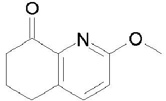 | 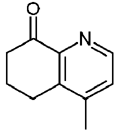 | 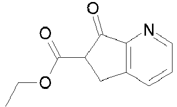 |
| 209741-58-2 | 73123-86-1 | 52402-29-6 |
| 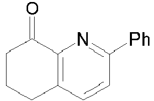 | 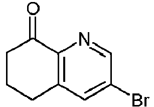 | 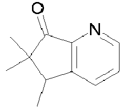 |
| 78590-01-9 | 904929-24-4 | 76474-76-5 |
| 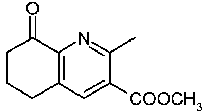 | 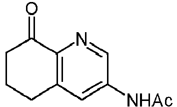 | 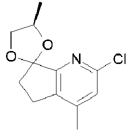 |
| 212762-37-3 | 331759-68-3 | 745075-86-9 |
| 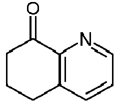 | 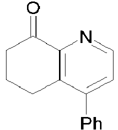 | 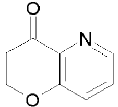 405174-48-3 |
| 56826-69-8 | 135761-75-0 | |
| 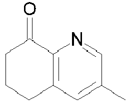 | 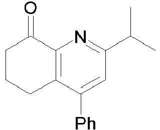 | 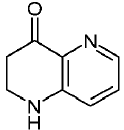 |
| 62230-65-3 | 906668-73-3 | 1211528-89-0 |
| 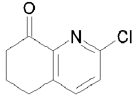 | 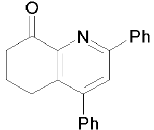 | 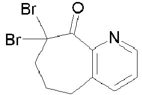 |
| 129337-86-6 | 1033623-16-3 | 41043-16-7 |
| 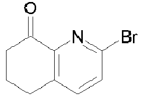 | 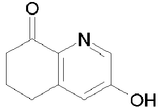 | 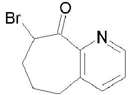 |
| 130861-70-010 | 1196153-30-6 | 41043-14-5 |
| 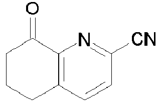 | 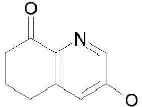 | 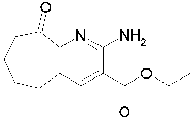 |
| 399042-43-4 | 558444-62-5 | 908231-09-4 |
| 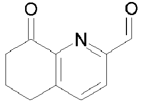 | 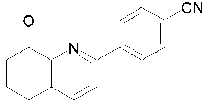 | 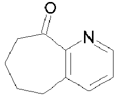 |
| 238755-38-9 | 423116-28-3 | 41043-13-4 |
| 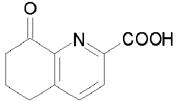 | 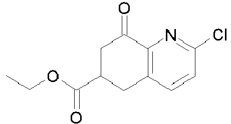 | 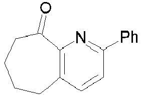 |
| 238755-39-0 | 864830-54-6 | 78509-53-2 |
| 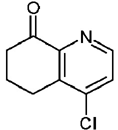 | 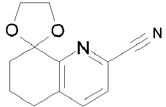 | 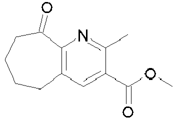 |
| 1196156-61-2 | 399042-44-5 | 212762-38-4 |
| 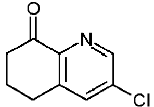 | 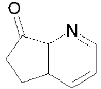 | 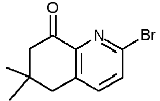 |
| 1196151-83-3 | 31170-78-2 | 1338093-82-5 |

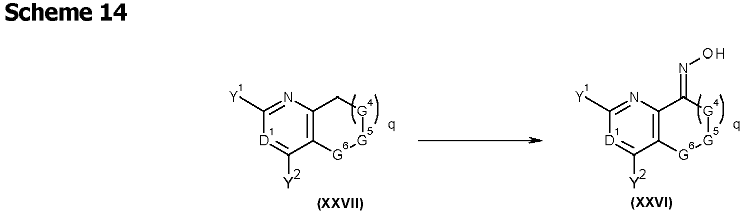

34) Alternatively, oximes of formula (XXVI) can be obtained by a nitrosation reaction of compounds of formula (XXVII), wherein G⁴, G⁵, G⁶, q, Y¹, Y² and D¹ are as defined herein for compounds of formula (I), with base and an alkyl nitrite, as seen in **scheme 14.** Typical bases include lithium diisopropyl amide (LDA), lithium hexamethyldisilazane, n-butyl lithium, s-butyl lithium, tert-butyl lithium, sodium tert-butylate or potassium tert-butylate. Typical alkyl nitrites include isopentyl nitrite and tert-butyl nitrite. The compound of formula (XXVII), the alkyl nitrite or the base can be used in different stoichiometric amounts, with each reagent possibly being in excess with respect to the others. Preferentially, such reactions are carried out under nonaqueous conditions in an inert solvent such as hexane, heptanes, cyclohexane, toluene or ethers such as THF or tert-butyl methyl ether. The reaction may be performed at temperatures ranging from -80 to 250°C, preferably between -50 and 120°C.
Such reactions can lead to a mixture of the E- and the Z-oxime (ether) product, or the product may also be exclusively either the E- or the Z-oxime (ether).
A large number of these types of transformations are known in the art. Typical reaction conditions for this type of reaction may be found in Crawford, Jason B.; Chen, Gang; Gauthier, David; Wilson, Trevor; Carpenter, Bryon; Baird, Ian R.; McEachern, Ernie; Kaller, Alan; Harwig, Curtis; Atsma, Bem; Skerlj, Renato T.; Bridger, Gary J., Organic Process Research & Development (2008), 12(5), 823-830, McEachern, E. J.; Yang, W.; Chen, G.; Skerlj, R. T.; Bridger, G. J., Synthetic Communications (2003), 33(20), 3497-350; and Bark, Thomas; Thummel, Randolph P., Inorganic Chemistry (2005), 44(24), 8733-8739.
Analogous protocols to those used to prepare the compounds in table 6 or table 7 can be used to prepare other compounds of formula (XXVII).
Ketone intermediates of formula XXVa, that is compound of formula XXV wherein D¹ is N, and T¹ and T² together with the carbon they are attached to form a carbonyl group; IIIa, that is a compound of formula III wherein D¹ is C-Y⁴, D² is N and T¹ and T² together with the carbon they are attached to form a carbonyl group; IIIb, that is a compound of formula III wherein D² is C-Y⁵, D¹ is N and T¹ and T² together with the carbon they are attached to form a carbonyl group; may be prepared by a variety of methods, illustrated in schemes 15-17. 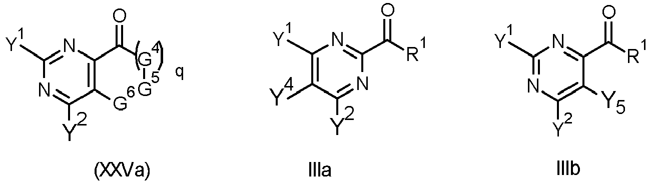 Some compounds of formula (XXVa) are known and their preparation has been published or they are available commercially. A few typical examples are given in Table 7 together with the corresponding CAS numbers.

**Table 7**

| | | |
|---|---|---|
| 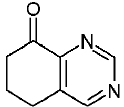 | 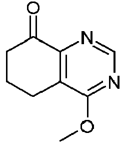 | 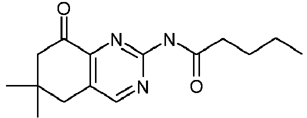 |
| 89967-17-9 | 64571-29-5 | 420824-47-1 |
| 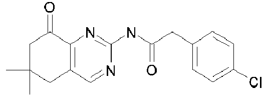 | 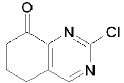 | 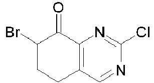 |
| 420824-53-9 | 1105664-58-1 | 1105664-60-5 |
| 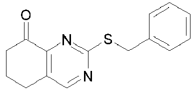 | | |
| 802541-55-5 | | |

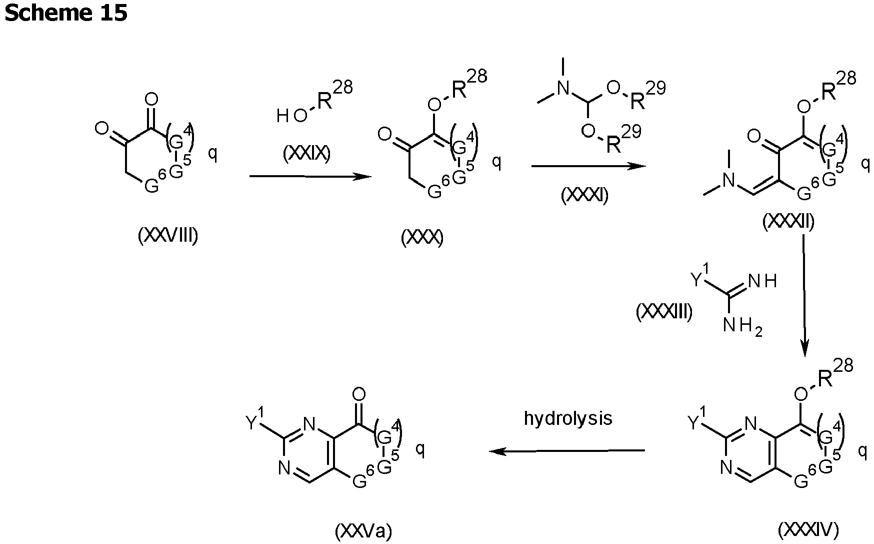

35) Compounds of formula (XXVa) can be obtained by acidic hydrolysis methods, well known to the person skilled in the art of compounds of formula (XXXIV), wherein R²⁸ is alkyl and G⁴, G⁵, G⁶, q and Y¹ are as defined herein for compounds of formula (I).
37) Enolethers of formula (XXXIV) can be obtained by reacting enaminone of formula (XXXII) wherein R²⁸ is alkyl and G⁴, G⁵, G⁶, q and Y¹ are as defined herein for compounds of formula (I)with amidines or guanidines of formula (XXXIII) wherein Y¹ is as defined for compounds of formula (I). Such condensation reactions can be performed using a number of conditions well known to the person skilled in the art. Such reactions are carried out in the presence of a base preferentially sodium or potassium alcoholate in ethanol or methanol. Specific reaction conditions may be found in Synthesis, 1012-1018, 1996; J. Heterocycl. Chem. 20, 649-653, 1983 or in WO2009010488.
38) Enaminone of formula (XXXII) can be obtained by reacting ketone of formula (XXX) wherein R²⁸ is alkyl and G⁴, G⁵, G⁶ and q are as defined herein for compounds of formula (I) with N,N-dimethylformamide dialkyl acetal of formula (XXXI) wherein R²⁹ is alkyl. Such condensation reactions are well known to the person skilled in the art. The reaction is carried out in an inert solvent or without solvent at temperatures ranging from 0°C to 250°C, preferably between 50 and 160°C. Specific reaction conditions may be found in Synthesis, (97), 3397, 1964; Synthesis, (97), 3407, 1964; Journal of Med. Chem., Vol. 52, No. 16, 5152-5163; Tetrahedron Lett, (27), 2567, 1986; Tetrahedron Lett, (50), 2255-2264, 1994; Synthetic Commun., (28), 10, 1743-1753, 1998 or in Chem. Ber., (104), 2975, 1971.
39) Enol ether of formula (XXX) can be obtained by reacting ketones of formula (XXVIII) wherein G⁴, G⁵, G⁶ and q are as defined herein for compounds of formula (I) with an alcohol of formula (XXIX) wherein R²⁸ is alkyl. Such enol reactions can be affected using a number of conditions well known to the person skilled in the art. Specific reaction conditions may be found in WO2004104007. 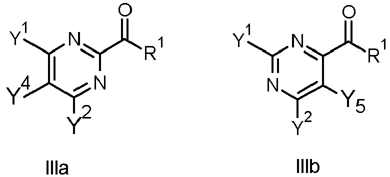 Some compounds of formula (IIIa), that is a compound of formula (III) wherein T¹ and T² together with the carbon they are attached to form a carbonyl group, D¹ is C-Y⁴, D² is N and R¹, Y¹, Y² and Y⁴ are as described herein for compound of formula (I) or (IIIb), that is a compound of formula (III) wherein T¹ and T² together with the carbon they are attached to form a carbonyl group, D¹ is N, D² is C-Y⁵ and R¹, Y¹, Y² and Y⁵ are as described herein for compound of formula (I) are known and their preparation has been published or they are available commercially. A few typical examples are given in Table 8 together with the corresponding CAS numbers.

**Table 8**

| | | |
|---|---|---|
| 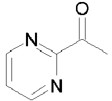 | 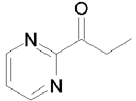 | 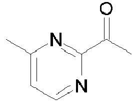 |
| 53342-27-1 | 54643-09-3 | 71850-85-6 |
| 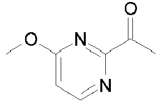 | 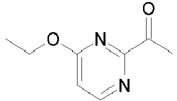 | 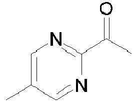 |
| 463337-53-3 | 145947-97-3 | 122372-22-9 |
| 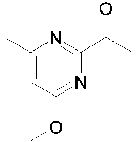 | 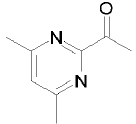 | 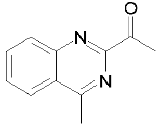 |
| 64571-49-9 | 64571-50-2 | 126994-78-3 |
| 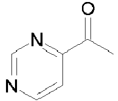 | 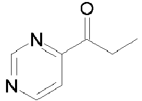 | 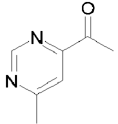 |
| 39870-05-8 | 54643-10-6 | 67073-96-5 |
| 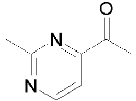 | 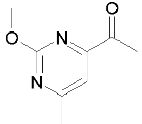 | 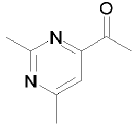 |
| 67860-38-2 | 64571-47-7 | 73937-21-0 |
| 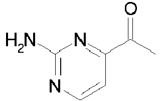 | 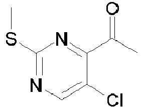 | 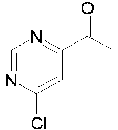 |
| 106157-82-8 | 122372-21-8 | 210295-81-1 |

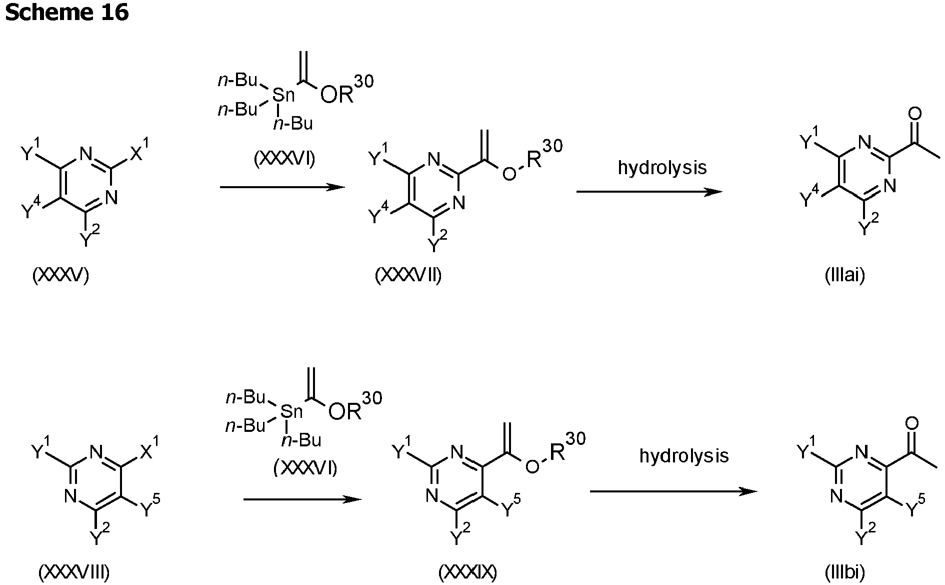

40) Ketone intermediates of formula (IIIai) or (IIIbi), that is compounds of formula (IIIa) and (IIIb) wherein R¹ is methyl can be obtained by a Stille cross-coupling reaction of the halo pyrimidine derivatives of formula (XXXV) or (XXXVIII)wherein X¹ is a halogen, preferably chlorine or bromine and Y¹, Y², Y⁴ and Y⁵ are as defined for compounds of formula (I) with tri-n-butyl(1-alkoxyvinyl)tin (XXXVI) wherein R³⁰ is alkyl to give the vinyl ether of formula (XXXVII) or (XXXIX), wherein Y¹, Y², Y⁴ and Y⁵ are as defined for compounds of formula (I) and R³⁰ is alkyl which by acidic hydrolysis methods give the desired ketone intermediate of formula (IIIai) or (IIIbi). Examples for such Stille cross-coupling reaction can be found in Angew. Chem. Int. Ed. Engl., 25, 508-524 (1986); Bull. Chem Soc. Jpn. 60, 767, 1987; Tetrahedron, 45, 993-1006 (1989); Acta Chem. Scand., 43, 62-68 (1989); Tetrahedron (1996), 52(15), 5625-38 or Angewandte Chemie, International Edition (2002), 41(7), 1195-1198. 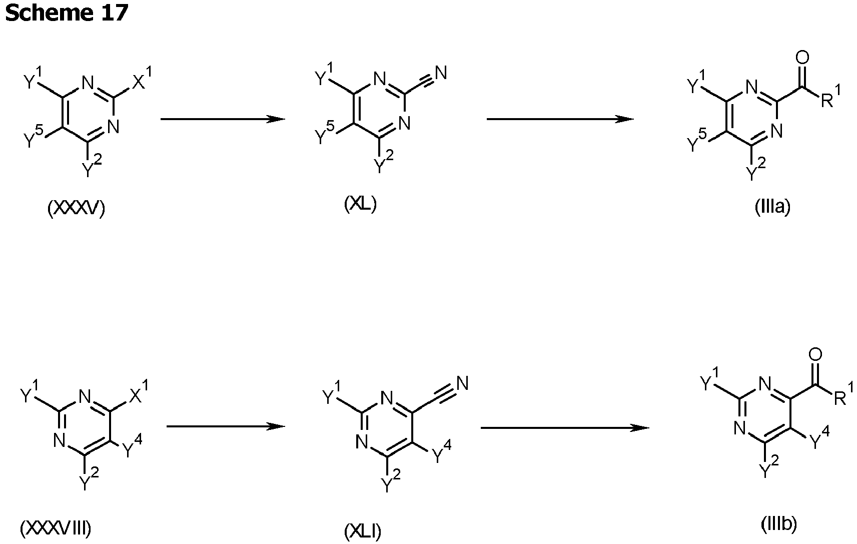 41) Alternatively compounds of formula (XXXV) or (XXXVIII) can be converted to the corresponding nitrile of formula (XL) or (XLI) wherein Y¹, Y², Y⁴ and Y⁵ are as defined for compounds of formula (I) by well known standard procedures. A subsequent reaction of nitrile derivative of formula (XL) or (XLI) with a Grignard reagent of the formula R¹-MgBr, wherein R¹ is as defined under formula I, yields the ketone intermediate of formula (IIIa) or (IIIb) as described in scheme 17. 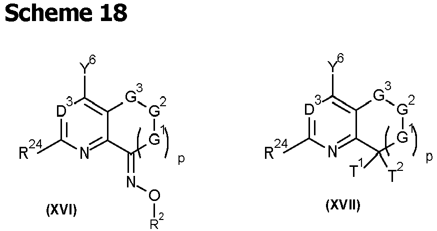 (42) Compounds of formula (XVI) and compounds of formula (XVII) wherein R²⁴ is Cl, Br, I or a sulfonate might be prepared by using similar procedures as described in schemes 13-15. The group R²⁴ might be introduced at any stage of their preparation depending on stability to the reaction conditions. The introduction of a group R²⁴ from for example OH, NH₂ or from an appropriate protecting group like for example benzyloxy is well known to the person skilled in the art. Some compounds of formula (XVII) are known and their preparation has been published. A few typical examples are given in Table 6 and Table 7 together with the corresponding CAS numbers.

### Typical conditions for condensation reactions:

This applies to procedures 1, 2, 4, 6, 37.

Different stoichiometric set-ups may be used for these reactions, depending on the properties of reactants and product. An excess of the electrophile, the nucleophile, or equimolar amounts may be chosen. Preferentially equimolar amounts of electrophilic and nucleophilic compounds are used.

The reaction may be performed in the presence or absence of an inert organic or inorganic solvent, or in the presence of a mixture of such solvents. Preferentially, it is performed in the presence of one or more solvents. Preferred solvents include the following aliphatic or aromatic hydrocarbons, which may optionally be substituted by one or more halogen atoms, such as pentane, hexanes, heptanes, cyclohexane, petroleum ether, benzene, toluene, xylene, chlorobenzene, dichlorobenzenes, dichloromethane, chloroform, 1,2-dichloroethane or carbon tetrachloride, ethers such as diethylether, diisopropyl ether, tert-butyl methyl ether, tetrahydrofuran, 1,4-dioxane, dimethoxyethane or diglycol dimethyl ether, ketones such as acetone, methyl ethyl ketone, methyl isopropyl ketone or methyl isobutyl ketone, acids and ester such as acetic acid, ethyl acetate or methyl acetate, aprotic polar solvents such as acetonitrile, pripionitril, dimethyl formamide, dimethyl acetamide, N-methyl-pyrrolidone, dimethyl sulfoxide, sulfolane, DMPU, or pyridine and picolines. The selection of solvents includes water and alcohols such as methanol, ethanol, propanol, isopropanol, butanol, isobutanol, tert-butanol, pentanol, isopentanol, hexanol, trifluorethanol, ethylene glycol or methoxyethanol. The reaction may be performed between -20°C and 250°C, preferentially between 0°C and 100°C. In some cases the reaction mixture may be heated to reflux.

Where appropriate, compounds can be used in the form of the free compound, or, alternatively, they can be used in the form of a salt such as the acetate, trifluoroacetate, propionate, benzoate, oxalate, methylsolfonate, phenylsulfonate, p-tolylsulfonate, trifluormethylsulfonate, fluoride, chloride, bromide, iodide, sulphate, hydrogensulphate or nitrate, including bis-salts if appropriate.

The reaction can be carried out in the absence of an acid using the free compounds. Alternatively, the reaction may be performed in the presence of an acid in catalytic, stoichiometric or excess amounts. Acids that could be used include acetic acid, propionic acid, oxalic acid, trifluoroacetic acid, hydrochloric acid, hydrobromic acid, hydroiodic acid, methansulfonic acid, para-toluenesulfonic acid, sulphuric acid, sodium hydrogensulphate and phosphoric acid. The reaction can optionally be carried out in a water-free solvent system in the presence of a drying agent, such as sodium or magnesium sulphate, potassium carbonate or molecular sieves.

If the two substituents at the carbon atom of the oxime or oxime ether function are different from each other, the condensation reaction can lead to a mixture of the E- and the Z-oxime (ether) product. The condensation product may also be exclusively either the E- or the Z- oxime (ether).

Condensations can be performed under reduced pressure, normal pressure or increased pressure. Preferentially the reaction is performed under normal pressure.

### Typical conditions for alkylation reactions:

This applies to procedure 3, 18, 21, 23, 29, 36.

Different stoichiometric set-ups may be used for these reactions, depending on the properties of reactants and product. An excess of the electrophile, the nucleophile, or neither may be chosen. Usually, it is preferable that equimolar amounts of electrophilic and nucleophilic compounds are used.

The reaction may be performed in the absence or presence of a solvent or a mixture of solvents. Preferential solvents include the following aliphatic or aromatic hydrocarbons that may optionally be substituted by one or more halogen atoms such as pentane, hexanes, heptanes, cyclohexane, petroleum ether, benzene, toluene, xylene, chlorobenzene, dichlorobenzenes, dichloromethane, chloroform, 1,2-dichloroethanev or carbon tetrachloride, ethers such as diethyl ether, diisopropyl ether, tert-butyl-methyl ether, tetrahydrofuran, 1,4-dioxane, dimethoxyethane or diglycol dimethyl ether, ketones such as acetone, methyl ethyl ketone, methyl isopropyl ketone or methyl isobutyl ketone, acids and ester such as acetic acid, ethyl acetate or methyl acetate, aprotic polar solvents such as acetonitrile, pripionitrile, dimethyl formamide, dimethyl acetamide, N-methyl-pyrrolidone, dimethyl sulfoxide, sulfolane, DMPU, or pyridine and picolines. The selction of solvents includes also water and alcohols such as methanol, ethanol, propanol, isopropanol, butanol, isobutanol, tert-butanol, pentanol, isopentanol, hexanol, trifluorethanol, ethylene glycol or methoxyethanol.

The reaction may be performed in a biphasic system comprising an organic solvent that is not miscible with water, such as toluene, dichloromethane, dichloro-ethylene, and an aqueous solvent, such as water. Such a reaction would be performed in the presence of a phase-transfer catalyst, such as tetra-n-butylammonium bromide (TBAB), Tetradecyldimethylbenzylammonium chloride (TDMBAC), N-Benzyltrimethylammonium hydroxide, along with aqueous sodium or potassium hydroxide in stoichiometric amounts. The biphasic reaction may be performed with or without ultrasonication.

The reaction may be carried out at temperatures varying from -100°C and 250°C. Preferentially, the temperature range is between 0°C and 100°C.

Optionally, an organic or inorganic base may be present such as alkali- and earth alkali acetates, amides, carbonates, hydrogencarbonates, hydrides, hydroxides or alcoholates such as sodium, potassium, caesium or calcium acetate, sodium, potassium, caesium or calcium carbonate, sodium, potassium, caesium or calcium hydrogencarbonate, sodium, potassium, caesium or calcium hydride, sodium, potassium, caesium or calcium amide, sodium, potassium, caesium or calcium hydroxide, sodium, potassium, caesium or calcium methanolate, sodium, potassium, caesium or calcium ethanolate, sodium, potassium, caesium or calcium n-, i-, s- or t-butanolate, triethylamine, tripropylamine, tributylamine, di-isopropyl-ethylamine, N,N-dimethyl-cyclohexylamine, N-methyl-dicyclohexylamine, N,N-dimethyl-aniline, N,N-diethyl-aniline, N,N-dimethyl-benzylamine, N,N-diethyl-benzylamine, pyridine, 2-methyl-pyridine, 3-methyl-pyridine, 4-methyl-pyridine, 2,6-dimethyl-pyridine, 2,4,6-trimethyl-pyridine, 4-dimethylamino-pyridine, N-methyl-piperidine, N-ethyl-piperidine, N-methyl-morpholine, N-ethyl-morpholine, N,N'-dimethylpiperazine, 1,4-Diazabicyclo[2.2.2]octane (DABCO), 1,8-Diaza-7-bicyclo[5.4.0]undecene (DBU), 1,5-Diazabicyclo[4.3.0]non-5-ene (DBN), 1-tert-Butyl-2,2,2-tri(1-pyrrolidinyl)phosphazene (BTPP), 1-tert-Butyl-2,2,2-tris(dimethylamino)phosphazene, sodium hexamethyldisilazane, potassium hexamethyldisilazane, lithium diisopropylamide, ethyl magnesium chloride, isopropylmagnesium chloride.

The alkylation can be performed under reduced pressure, normal pressure or increased pressure. Preferentially the reaction is performed under normal pressure.

The products of steps 1) to 31) may be required to be purified using, for example, chromatography, crystallisation or other purification techniques well known to the person skilled in the art.

The compounds of formula (I) to formula (XXXIV) and, where appropriate, the tautomers thereof, can, if appropriate, also be obtained in the form of hydrates and/or include other solvents, for example those which may have been used for the crystallization of compounds which are present in solid form.

The alkylation can be performed under reduced pressure, normal pressure or increased pressure. Preferentially the reaction is performed under normal pressure.

The products of steps 1) to 43) may be required to be purified using, for example, chromatography, crystallisation or other purification techniques well known to the person skilled in the art.

It has now been found that the compounds of formula (I) according to the invention have, for practical purposes, a very advantageous spectrum of activities for protecting useful plants against diseases that are caused by phytopathogenic microorganisams, such as fungi, bacteria or viruses.

The invention therefore also relates to a method of controlling or preventing infestation of useful plants by phytopathogenic microorganisms, wherein a compound of formula (I) is applied as active ingredient to the plants, to parts thereof or the locus thereof. The compounds of formula (I) according to the invention are distinguished by excellent activity at low rates of application, by being well tolerated by plants and by being environmentally safe. They have very useful curative, preventive and systemic properties and are used for protecting numerous useful plants. The compounds of formula (I) can be used to inhibit or destroy the diseases that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula (I) as dressing agents for the treatment of plant propagation material, in particular of seeds (fruit, tubers, grains) and plant cuttings (e.g. rice), for the protection against fungal infections as well as against phytopathogenic fungi occurring in the soil.

Furthermore the compounds of formula (I) according to the invention may be used for controlling fungi in related areas, for example in the protection of technical materials, including wood and wood related technical products, in food storage or in hygiene management.

The compounds of formula (I) are, for example, effective against the phytopathogenic fungi of the following classes: Fungi imperfecti (e.g. Botrytis, Pyricularia, Helminthosporium, Fusarium, Septoria, Cercospora and Alternaria) and Basidiomycetes (e.g. Rhizoctonia, Hemileia, Puccinia). Additionally, they are also effective against the Ascomycetes classes (e.g. Venturia and Erysiphe, Podosphaera, Monilinia, Uncinula) and of the Oomycetes classes (e.g. Phytophthora, Pythium, Plasmopara). Within the scope of the invention, useful plants to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as ornamentals.

The term "useful plants" is to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield® summer rape (Canola). Examples of crops that have been rendered tolerant to herbicides or classes of herbicides by genetic engineering methods include glyphosate- and glufosinate-resistant maize varieties commercially available under the trade names RoundupReady®, Herculex I® and LibertyLink®.

The term "useful plants" is to be understood as including also useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard® (maize variety that expresses a CryIA(b) toxin); YieldGard Rootworm® (maize variety that expresses a CryIIIB(b1) toxin); YieldGard Plus® (maize variety that expresses a CryIA(b) and a CryIIIB(b1) toxin); Starlink® (maize variety that expresses a Cry9(c) toxin); Herculex I® (maize variety that expresses a CryIF(a2) toxin and the enzyme phosphinothricine N-acetyltransferase (PAT) to achieve tolerance to the herbicide glufosinate ammonium); NuCOTN 33B® (cotton variety that expresses a CryIA(c) toxin); Bollgard I® (cotton variety that expresses a CryIA(c) toxin); Bollgard II® (cotton variety that expresses a CryIA(c) and a CryIIA(b) toxin); VIPCOT® (cotton variety that expresses a VIP toxin); NewLeaf® (potato variety that expresses a CryIIIA toxin); NatureGard® Agrisure® GT Advantage (GA21 glyphosate-tolerant trait), Agrisure® CB Advantage (Bt11 corn borer (CB) trait), Agrisure® RW (corn rootworm trait) and Protecta®.

The term "useful plants" is to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

The term "locus" of a useful plant as used herein is intended to embrace the place on which the useful plants are growing, where the plant propagation materials of the useful plants are sown or where the plant propagation materials of the useful plants will be placed into the soil. An example for such a locus is a field, on which crop plants are growing.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

The compounds of formula (I) can be used in unmodified form or, preferably, together with carriers and adjuvants conventionally employed in the art of formulation.

Therefore the invention also relates to compositions for controlling and protecting against phytopathogenic microorganisms, comprising a compound of formula (I) and an inert carrier, and to a method of controlling or preventing infestation of useful plants by phytopathogenic microorganisms, wherein a composition, comprising a compound of formula (I) as acitve ingredient and an inert carrier, is applied to the plants, to parts thereof or the locus thereof.

To this end compounds of formula (I) and inert carriers are conveniently formulated in known manner to emulsifiable concentrates, coatable pastes, directly sprayable or dilutable solutions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects. Suitable carriers and adjuvants (auxiliaries) can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula (I) or compositions, comprising a compound of formula (I) as active ingredient and an inert carrier, can be applied to the locus of the plant or plant to be treated, simultaneously or in succession with further compounds. These further compounds can be e.g. fertilizers or micronutrient donors or other preparations which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

A preferred method of applying a compound of formula (I), or a composition, comprising a compound of formula (I) as active ingredient and an inert carrier, is foliar application. The frequency of application and the rate of application will depend on the risk of infestation by the corresponding pathogen. However, the compounds of formula (I) may also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula (I) may also be applied to seeds (coating) by impregnating the seeds or tubers either with a liquid formulation of the fungicide or coating them with a solid formulation. A formulation, i.e. a composition comprising the compound of formula (I) and, if desired, a solid or liquid adjuvant, is prepared in a known manner, typically by intimately mixing and/or grinding the compound with extenders, for example solvents, solid carriers and, optionally, surface-active compounds (surfactants).

The agrochemical formulations will usually contain from 0.1 to 99% by weight, preferably from 0.1 to 95% by weight, of the compound of formula (I), 99.9 to 1% by weight, preferably 99.8 to 5% by weight, of a solid or liquid adjuvant, and from 0 to 25% by weight, preferably from 0.1 to 25% by weight, of a surfactant.

Whereas it is preferred to formulate commercial products as concentrates, the end user will normally use dilute formulations.

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient rates of application are from 10mg to 1g of active substance per kg of seeds. The rate of application for the desired action can be determined by experiments. It depends for example on the type of action, the developmental stage of the useful plant, and on the the application (location, timing, application method) and can, owing to these parameters, vary within wide limits.

The compounds of formula (I), or a pharmaceutical salt thereof, described above may also have an advantageous spectrum of activity for the treatment and/or prevention of microbial infection in an animal. "Animal" can be any animal, for example, insect, mammal, reptile, fish, amphibian, preferably mammal, most preferably human. "Treatment" means the use on an animal which has microbial infection in order to reduce or slow or stop the increase or spread of the infection, or to reduce the infection or to cure the infection. "Prevention" means the use on an animal which has no apparent signs of microbial infection in order to prevent any future infection, or to reduce or slow the increase or spread of any future infection.

According to the present invention there is provided the use of a compound of formula (I) in the manufacture of a medicament for use in the treatment and/or prevention of microbial infection in an animal. There is also provided the use of a compound of formula (I) as a pharmaceutical agent. There is also provided the use of a compound of formula (I) as an antimicrobial agent in the treatment of an animal. According to the present invention there is also provided a pharmaceutical composition comprising as an active ingredient a compound of formula (I), or a pharmaceutically acceptable salt thereof, and a pharmaceutically acceptable diluent or carrier. This composition can be used for the treatment and/or prevention of antimicrobial infection in an animal. This pharmaceutical composition can be in a form suitable for oral administration, such as tablet, lozenges, hard capsules, aqueous suspensions, oily suspensions, emulsions dispersible powders, dispersible granules, syrups and elixirs. Alternatively this pharmaceutical composition can be in a form suitable for topical application, such as a spray, a cream or lotion. Alternatively this pharmaceutical composition can be in a form suitable for parenteral administration, for example injection. Alternatively this pharmaceutical composition can be in inhalable form, such as an aerosol spray.

The compounds of formula (I) may be effective against various microbial species able to cause a microbial infection in an animal. Examples of such microbial species are those causing Aspergillosis such as *Aspergillus fumigatus, A. flavus, A. terrus, A. nidulans* and *A. niger,* those causing Blastomycosis such as *Blastomyces dermatitidis*; those causing Candidiasis such as *Candida albicans, C. glabrata, C. tropicalis, C. parapsilosis, C. krusei* and *C*. *lusitaniae;* those causing Coccidioidomycosis such as *Coccidioides immitis;* those causing Cryptococcosis such as *Cryptococcus neoformans;* those causing Histoplasmosis such as *Histoplasma capsulatum* and those causing Zygomycosis such as *Absidia corymbifera, Rhizomucor pusillus* and *Rhizopus arrhizus.* Further examples are Fusarium Spp such as *Fusarium oxysporum* and *Fusarium solani* and Scedosporium Spp such as *Scedosporium apiospermum* and *Scedosporium prolificans.* Still further examples are Microsporum Spp, Trichophyton Spp, Epidermophyton Spp, Mucor Spp, Sporothorix Spp, Phialophora Spp, Cladosporium Spp, Petriellidium spp, Paracoccidioides Spp and Histoplasma Spp.

The compositions of this invention may contain other compounds having biological activity, for example micronutrients or compounds having fungicidal activity or which possess plant growth regulating, herbicidal, insecticidal, nematicidal or acaricidal activity.

The compound of formula I (herein after abbreviated by the term "TX" thus means a compound encompassed by the compounds of formula I, or preferably the term "TX" refers to a compound selected from the Tables 1-5 or Table 9) may be the sole active ingredient of the composition or it may be admixed with one or more additional active ingredients such as a pesticide (insect, acarine, mollusc and nematode pesticide), fungicide, synergist, herbicide, safener or plant growth regulator where appropriate. The activity of the compositions according to the invention may thereby be broadened considerably and may have surprising advantages which can also be described, in a wider sense, as synergistic activity. An additional active ingredient may: provide a composition having a broader spectrum of activity or increased persistence at a locus; provide a composition demonstrating better plant/crop tolerance by reducing phytotoxicity; provide a composition controlling insects in their different development stages; synergise the activity or complement the activity (for example by increasing the speed of effect or overcoming repellency) of the TX; or help to overcome or prevent the development of resistance to individual components. The particular additional active ingredient will depend upon the intended utility of the composition. Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl-3-(2-oxothiolan-3-ylidenemethyl)cyclopropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e)Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, or spinosad, spinetoram or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l)Neonicotinoid compounds such as imidacloprid, thiacloprid, acetamiprid, clothianidin, nitenpyram, dinotefuran or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine or pyrifluquinazon;
r) Spirotetramat, spirodiclofen or spiromesifen;
s) Flubendiamide, chloranthraliniprole, or cyanthraniliprole;
t) Cyenopyrafen or cyflumetofen; or
u) Sulfoxaflor.

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron). The following mixtures of the compounds of formula I with active ingredients are preferred, wherein, preferably, the term "TX" refers to a compound covered by the compounds of formula I or preferably the term "TX" refers to a compound selected from the Tables 1 to 9 and the following List shows specific examples of mixtures comprising the component TX and the component (B):
an adjuvant selected from the group of substances consisting of petroleum oils (alternative name) (628) + TX, an acaricide selected from the group of substances consisting of 1,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulphide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (alternative name) (473) + TX, isopropyl *O*-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulphur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
   an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
   an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX, an avicide selected from the group of substances consisting of chloralose (127) + TX, endrin (1122) + TX, fenthion (346) + TX, pyridin-4-amine (IUPAC name) (23) and strychnine (745) + TX,
   a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
   a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H*. *megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,
   a soil sterilant selected from the group of substances consisting of iodomethane (IUPAC name) (542) and methyl bromide (537) + TX, a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,
   an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (*E*,*Z*)-tetradeca-4,10-dien-1-yl acetate (IUPAC name) (779) + TX, (*Z*)-dodec-7-en-1-yl acetate (IUPAC name) (285) + TX, (*Z*)-hexadec-11-enal (IUPAC name) (436) + TX, (*Z*)-hexadec-11-en-1-yl acetate (IUPAC name) (437) + TX, (*Z*)-hexadec-13-en-11-yn-1-yl acetate (IUPAC name) (438) + TX, (*Z*)-icos-13-en-10-one (IUPAC name) (448) + TX, (*Z*)-tetradec-7-en-1-al (IUPAC name) (782) + TX, (*Z*)-tetradec-9-en-1-ol (IUPAC name) (783) + TX, (*Z*)-tetradec-9-en-1-yl acetate (IUPAC name) (784) + TX, (7*E*,9*Z*)-dodeca-7,9-dien-1-yl acetate (IUPAC name) (283) + TX, (9Z,11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX, an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
   an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulphinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin *S*-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, *d*-limonene (alternative name) [CCN] + TX, *d*-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl *O-*(methoxyaminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, *m*-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulphonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl *O*-ethyl ethylphosphonothioate (IUPAC name) (1057) + TX, *O*,*O*-diethyl *O*-4-methyl-2-oxo-2*H*-chromen-7-yl phosphorothioate (IUPAC name) (1074) + TX, *O*,*O*-diethyl *O*-6-methyl-2-propylpyrimidin-4-yl phosphorothioate (IUPAC name) (1075) + TX, *O*,*O*,*O'*,*O'*-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulphuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19] + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX, a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX,
   a nitrification inhibitor selected from the group of substances consisting of potassium ethylxanthate [CCN] and nitrapyrin (580) + TX,
   a plant activator selected from the group of substances consisting of acibenzolar (6) + TX, acibenzolar-*S*-methyl (6) + TX, probenazole (658) and *Reynoutria sachalinensis* extract (alternative name) (720) + TX,
   a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
   a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX, an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX, a virucide selected from the group of substances consisting of imanin (alternative name) [CCN] and ribavirin (alternative name) [CCN] + TX,
   a wound protectant selected from the group of substances consisting of mercuric oxide (512) + TX, octhilinone (590) and thiophanate-methyl (802) + TX,
   and biologically active compounds selected from the group consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxiconazole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebuconazole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metomi-nostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3 ] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil *[1897-45-6]* + TX, cyflufenamid *[180409-60-3]* + TX, cymoxanil *[57966-95-7]* + TX, dichlone *[117-80-6]* + TX, diclocymet [139920-32-4] + TX, diclomezine *[62865-36-5]* + TX, dicloran *[99-30-9]* + TX, diethofencarb *[87130-20-9]* + TX, dimethomorph *[110488-70-5]* + TX, SYP-LI90 (Flumorph) *[211867-47-9] +* TX, dithianon *[3347-22-6]* + TX, ethaboxam *[162650-77-3]* + TX, etridiazole *[2593-15-9] +* TX, famoxadone *[131807-57-3]* + TX, fenamidone *[161326-34-7]* + TX, fenoxanil *[115852-48-7] +* TX, fentin *[668-34-8]* + TX, ferimzone *[89269-64-7]* + TX, fluazinam *[79622-59-6] +* TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[120116-88-3]* + TX, kasugamycin [6980-18-3] + TX, methasulfocarb *[66952-49-6]* + TX, metrafenone *[220899-03-6]* + TX, pencycuron *[66063-05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulphur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1,2,3,4-tetrahydro-1,4-methano-naphthalen-5-yl)-amide (dislosed in WO 2007/048556) + TX, 3-difluoromethyl-1-methyl-1H-pyrazole-4-carboxylic acid [2-(2,4-dichlorophenyl)-2-methoxy-1-methyl-ethyl]-amide (disclosed in WO 2008/148570) + TX, 1-[4-[4-[(5S)5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone + TX, 1-[4-[4-[5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone [1003318-67-9], both disclosed in WO 2010/123791, WO 2008/013925, WO 2008/013622 and WO 2011/051243 page 20) +TX, *S*)-[3-(4-Chloro-2-fluoro-phenyl)-5 - (2,4-difluoro-phenyl)-isoxazol-4-y l]-pyridin-3-yl-methanol + TX, 3-(4-Chloro-2-fluoro-phenyl)-5 - (2,4-difluoro-phenyl)-isoxazol-4-y l]-pyridin-3-yl-methanol + TX, 3-difluoromethyl-1-methyl-1H-pyrazole-4-carboxylic acid (3',4',5'-trifluoro-biphenyl-2-yl)-amide (disclosed in WO 2006/087343) + TX, 3-(difluoromethyl)-N-methoxy-1-methyl-N-[1-methyl-2-(2,4,6-trichlorophenyl)ethyl]-1H-Pyrazole-4-carboxamide + TX, 4-[(5*S*)-5-(3,5-dichlorophenyl)-5-(trifluoromethyl)-4H-isoxazol-3-yl]-2-methyl-N-(thietan-3-yl)benzamide (WO2011/104089) + TX, 4-[(5R)-5-(3,5-dichlorophenyl)-5-(trifluoromethyl)-4H-isoxazol-3-yl]-2-methyl-N-(thietan-3-yl)benzamide (WO2011/104089) + TX, 4-[(5*S*)-5-(3,5-dichlorophenyl)-5-(trifluoromethyl)-4H-isoxazol-3-yl]-2-methyl-N-(cis-1-oxo-thietan-3-yl)benzamide (WO2011/104089) + TX, 4-[(5R)-5-(3,5-dichlorophenyl)-5-(trifluoromethyl)-4H-isoxazol-3-yl]-2-methyl-N-(cis-1-oxo-thietan-3-yl)benzamide (WO2011/104089) + TX, 4-[(5*S*)-5-(3,5-dichlorophenyl)-5-(trifluoromethyl)-4H-isoxazol-3-yl]-2-methyl-N-(trans-1-oxo-thietan-3-yl)benzamide (WO2011/104089) + TX, 4-[(5*R*)-5-(3,5-dichlorophenyl)-5-(trifluoromethyl)-4H-isoxazol-3-yl]-2-methyl-N-(trans-1-oxo-thietan-3-yl)benzamide (WO2011/104089) + TX, 4-[(5*S*)-5-(3,5-dichlorophenyl)-5-(trifluoromethyl)-4H-isoxazol-3-yl]-N-(1,1-dioxothietan-3-yl)-2-methyl-benzamide (WO2011/104089) + TX, 4-[(5R)-5-(3,5-dichlorophenyl)-5-(trifluoromethyl)-4H-isoxazol-3-yl]-N-(1,1-dioxothietan-3-yl)-2-methyl-benzamide (WO2011/104089) + TX, 4-[(5*S*)-5-(3,5-dichlorophenyl)-5-(trifluoromethyl)-4H-isoxazol-3-yl]-2-methyl-N-[2-oxo-2-(2,2,2-trifluoroethylamino)ethyl]benzamide (WO2011/104089) + TX, 4-[(5R)-5-(3,5-dichlorophenyl)-5-(trifluoromethyl)-4H-isoxazol-3-yl]-2-methyl-N-[2-oxo-2-(2,2,2-trifluoroethylamino)ethyl]benzamide (WO2011/104089) + TX, Penflufen [494793-67-8] + TX, 5-[(5S)-5-(3,5-dichlorophenyl)-5-(trifluoromethyl)-4H-isoxazol-3-yl]-2-(1,2,4-triazol-1-yl)benzonitrile (WO2007/075459) + TX, 5-[(5R)-5-(3,5-dichlorophenyl)-5-(trifluoromethyl)-4H-isoxazol-3-yl]-2-(1,2,4-triazol-1-yl)benzonitrile (WO2007/075459) +TX.

The components (B) are known. The references in brackets behind the active ingredients, e.g. *[3878-19-1]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet under the internet address http://www.alanwood.net/pesticides/ [A. Wood; Compendium of Pesticide Common Names, Copyright © 1995-2012]; or preferably one of the further pesticides listed below.

In the above different lists of active ingredients to be mixed with a TX, the compound of the formula I is preferably a compound selected from the Tables 1-5 or Table 9; more preferably Table 9;
and even more preferably a compound selected from P.01 or P.02.

In the above-mentioned mixtures of compounds of formula I, in particular a compound selected from said Tables 1-5 or Table 9, with other insecticides, fungicides, herbicides, safeners, adjuvants and the like, the mixing ratios can vary over a large range and are, preferably 100:1 to 1:6000, especially 50:1 to 1:50, more especially 20:1 to 1:20, even more especially 10:1 to 1: 10. Those mixing ratios are understood to include, on the one hand, ratios by weight and also, on other hand, molar ratios.

The mixtures can advantageously be used in the above-mentioned formulations (in which case "active ingredient" relates to the respective mixture of TX with the mixing partner).

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The mixtures comprising a TX selected from Tables 1-5 or Table 9 and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables 1-5 or Table 9 and the active ingredients as described above is not essential for working the present invention.

The following non-limiting Examples illustrate the above-described invention in greater detail without limiting it. Those skilled in the art will promptly recognize appropriate variations from the procedures both as to reactants and as to reaction conditions and techniques. All references mentioned herein are incorporated by reference in their entirety.

### Preparatory examples:

Throughout these examples, the isomer drawn is in excess in the reaction mixture and/or product.

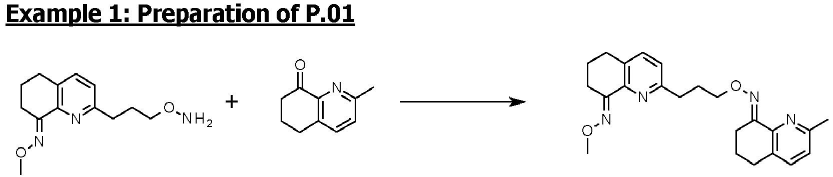

O-[3-[(8E)-8-Methoxyimino-6,7-dihydro-5H-quinolin-2-yl]propyl]hydroxylamine (0.060 g) was disolved in ethanol (4.8 mL) and 2-methyl-6,7-dihydro-5H-quinolin-8-one (0.047 g; CA Registry Number: 849643-01-2) was added. After the addition of p-toluenesufonic acid monohydrate (0.0028 g) the orange solution was stirred at ambient temperature for 2h. Then the reaction mixture was diluted with dichloromethane and quenched with NaHCO₃ sat aq. The water phase was extracted twice with dichloromethane. The combined organic phases were then washed with brine, dried over sodium sulfate, filtered, concentrated and purified over silica to give an orange gum (87 mg)

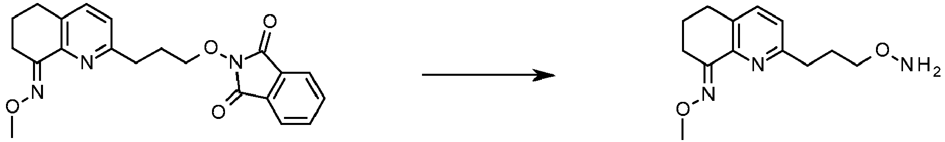

2-[3-[(8E)-8-Methoxyimino-6,7-dihydro-5H-quinolin-2-yl]propoxy]isoindoline-1,3-dione (0.180 g) was disolved in ethanol (9 mL). Hydrazine hydrate (0.0461 mL; 0.0475 g) dropwise and the solution was stirred for 1h at ambient temperature. The reaction mixture was diluted with dichloromethane and NaOH (1M) and extracted 3 times with dichloromethane. The combined organic phases were then washed with brine, dried over sodium sulfate, filtered and concentrated to give O-[3-[(8E)-8-methoxyimino-6,7-dihydro-5H-quinolin-2-yl]propyl]hydroxylamine (115 mg) as a yellow gum. LC-MS (OA_standard) UV Detection: 220 nm; Rt = 0.35 , MS: (M+1) = 250.

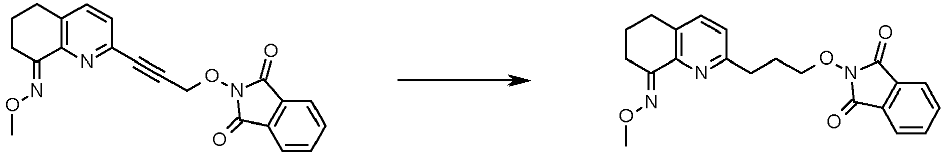

2-[3-[(8E)-8-Methoxyimino-6,7-dihydro-5H-quinolin-2-yl]prop-2-ynoxy]isoindoline-1,3-dione (0.490 g) was disolved in THF (73 mL). The hydrogenation was done in a flow reactor (H-Cube® Continuous-flow Hydrogenation Reactor) under the following conditions: H2, 10bar, Pd/C cartridge, 1mL solvent??/min at 25°C. After 75min the collected fractions were evaporated and purified over silica to give 2-[3-[(8E)-8-methoxyimino-6,7-dihydro-5H-quinolin-2-yl]propoxy]isoindoline-1,3-dione (160mg) as a yellow oil.

LC-MS (OA_3min_30V) UV Detection: 220 nm; Rt = 1.37min , MS: (M+1) = 380.

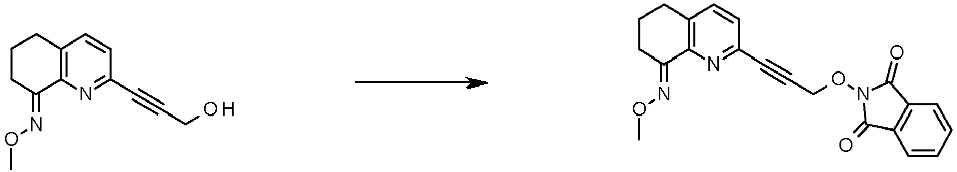

3-[(8E)-8-Methoxyimino-6,7-dihydro-5H-quinolin-2-yl]prop-2-yn-1-ol (0.330 g), N-hydroxyphthalimide (0.280 g) and triphenylphosphine (0.451 g) were stirred in THF (10 mL) at 0-5°C. Diisopropyl azo-dicarboxylate (0.339 mL; 0.348 g) disolved in THF (4 mL) was then added dropwise and the resulting yellow solution was stirred from 0°C to RT for 3h. The reaction mixture was diluted with ethy lacetate and water and extracted 3 times with ethyl acetate. The combined organic phases were then washed with brine, dried over sodium sulfate, filtered , concentrated and purifided over silica to give 2-[3-[(8E)-8-methoxyimino-6,7-dihydro-5H-quinolin-2-yl]prop-2-ynoxy]isoindoline-1,3-dione (450 mg) as a white solid. LC-MS (OA_3min_30V) UV Detection: 220 nm; Rt = 1.37min , MS: (M++1) = 380.

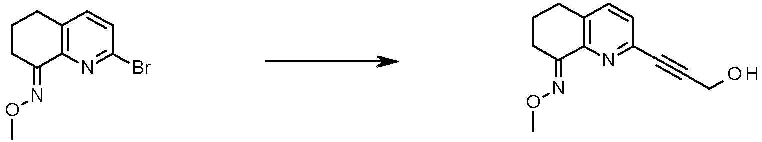

2-Bromo-N-methoxy-6,7-dihydro-5H-quinolin-8-imine (0.600 g), diethylamine (3.65 mL; 2.58 g), bis(triphenylphosphine)palladium(II) dichloride (0.0825 g), CuI (0.0224 g) and triphenylphosphine (0.123 g) in dimethyl formamide (24 mL) were stirred at ambient temperature. 2-Propyn-1-ol (0.208 mL, 0.198 g) was then added and the solution was stirred for 2h at 60°C under an argon atmosphere. The reaction mixture was diluted diluted with ethyl acetate and water and extracted 3 times with etyl acetate. The combined organic phases were then washed with NaHCO₃ sat aq, with water and with brine, dried over sodium sulfate, filtered, concentrated and purifided over silica to give 3-[(8E)-8-methoxyimino-6,7-dihydro-5H-quinolin-2-yl]prop-2-yn-1-ol (380mg) as a beige solid. LC-MS (OA_3min_30V) UV Detection: 220 nm; Rt = 1.32min , MS: (M+1) = 231

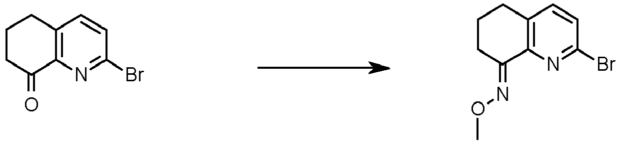

2-Bromo-6,7-dihydro-5H-quinolin-8-one (0.60 g), sodium acetate (0.37 g), O-methyl hydroxylamine hydrochloride (0.33 g) were suspended in ethyl alcohol (30 mL). After stirring for 22h at ambient temperature the reaction mixture was diluted with water and extracted with ethylacetate. The combined organic phases were dried over sodium sulfate, filtered and concentrated to give 2-bromo-N-methoxy-6,7-dihydro-5H-quinolin-8-imine (600 mg). LC-MS (OA_3min_30V) UV Detection: 220 nm; Rt = 1.57min, MS: (M+1) = 255

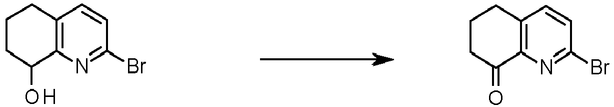

2-Bromo-5,6,7,8-tetrahydroquinolin-8-ol (1.1 g; CAS Registry Number: 130861-70-0) and 1,1,1-triacetoxy-1,1-dihydro-1,2-benziodoxol-3(1H)-one (2.3 g) in dichloromethane (145 mL) were stirred for 90min at ambient temperature. Sodium bicarbonate solution (70 mL; 20% in water) and sodium thiosulfate solution (70 mL; 40% in water) were added and the mixture was stirred for 60min at ambient temperature. The phases were separated and the water phase was extracted with dichloromethane. The combined organic phases were dried over sodium sulfate, filtered and concentrated to give 2-bromo-6,7-dihydro-5H-quinolin-8-one (0.60 g). LC-MS (OA_3min_30V) UV Detection: 220 nm; Rt = 1.16min , MS: (M+1) = 226.

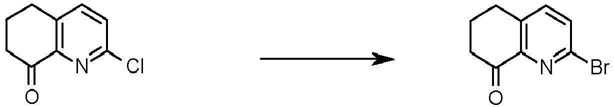

A solution of 2-chloro-6,7-dihydro-5H-quinolin-8-one (0.8 g; CAS Registry Number: 129337-86-6) and trimethyl silylbromide in acetinitrile (15 mL) was placed in a closed vial and heated in a microwave oven to 150°C for 20min. The reaction mixture was diluted with dichloromethane and quenched with a solution of sodium bicarbonate (15% in water). The organic phase was then washed wit brine, dried over sodium sulfate, filtered, concentrated and purified over silica to give 2-bromo-6,7-dihydro-5H-quinolin-8-one (820 mg) as a yellow solid. LC-MS (OA_standard) UV Detection: 220 nm; Rt = 0.63 , MS: (M+1) = 226.

**Table 9: Physical data of compounds of formula (I):**

| | **Structure** | **RT (mins) (method)** | **Molecular ion** |
|---|---|---|---|
| P.01 | 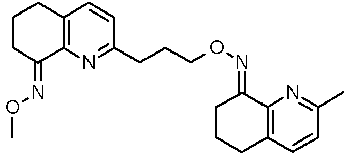 | 0.68 (OA_Standard) | 393 ([M+1]⁺) |
| P.02 | 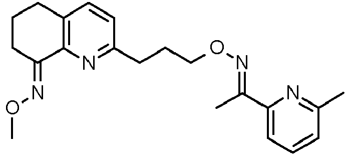 | 0.81 (OA_Standard) | 367 ([M+1]⁺) |

### LC-method used

### OA_Standard

ZQ Mass Spectrometer from Waters (Single quadrupole mass spectrometer) Instrument Parameter:
Ionization method: Electrospray
Polarity: positive and negative ions
Capillary: 3.00 kV
Cone: 30 V
Extractor: 2.00 V
Source Temperature: 150°C,
Desolvation Temperature: 350C
Cone Gas Flow: 50 L/Hr
Desolvation Gas Flow: 400 L/Hr
Mass range: 100 to 900 Da

Acquity UPLC from Waters:
Binary pump, heated column compartment and diode-array detector.
Solvent degasser, binary pump, heated column compartment and diode-array detector.
Column: Waters UPLC HSS T3 , 1.8 ·m, 30 x 2.1 mm,
Temp: 60 °C
DAD Wavelength range (nm): 210 to 500

Solvent Gradient:
A = H2O + 5% MeOH + 0.05 % HCOOH
B= Acetonit ril + 0.05 % HCOOH

| Time | A% | B% | Flow (ml/min) |
|---|---|---|---|
| 0.00 | 90 | 10 | 0.85 |
| 2.70 | 0 | 100.0 | 0.85 |
| 3.00 | 0 | 100.0 | 0.85 |

### OA_3min_30V

ZQ Mass Spectrometer from Waters (Single quadrupole mass spectrometer):
Ionization method: Electrospray; Polarity: positive and negative ions; Capillary (kV): 3.00; Cone (V): 30.00; Extractor (V): 2.00; Source Temperature (°C): 100; Desolvation Temperature (°C): 250; Cone Gas Flow (L/Hr): 50; Desolvation Gas Flow (L/Hr): 400; Mass range: 100 to 900 Da HP 1100 HPLC from Agilent:
Solvent degasser, binary pump, heated column compartment and diode-array detector; Column: Phenomenex Gemini C18, 3 ·m, 30 x 3 mm; Temp: 60°C; DAD Wavelength range (nm): 210 to 500;

Solvent Gradient:
A = water + 5% MeOH + 0.05 % HCOOH
B= Acetonitrile + 0.05 % HCOOH

| Time | A% | B% | Flow | (mL/min) |
|---|---|---|---|---|
| 0.00 | | 100 | 0 | 1.700 |
| 2.00 | | 0 | 100 | 1.700 |
| 2.80 | | 0 | 100 | 1.700 |
| 2.90 | | 100 | 0 | 1.700 |
| 3.00 | | 100 | 0 | 1.700 |

### Biological examples:

***Phytophthora infestans*** / tomato / leaf disc preventative (late blight) Tomato leaf disks were placed on water agar in multiwell plates (24-well format) and sprayed with the formulated test compound diluted in water at an application rate of 200ppm. The leaf disks were inoculated with a spore suspension of the fungus 1 day after application. The inoculated leaf disks were incubated at 16°C and 75% relative humidity under a light regime of 24 h darkness followed by 12/12 h (light/dark) in a climate cabinet and the activity of a compound was assessed as percent disease control compared to untreated when an appropriate level of disease damage appears in untreated check leaf disks (5 - 7 days after application). The following compounds gave at least 80% control of *Phytophthora infestans*: P.01

### Puccinia recondita f. sp. tritici l wheat / leaf disc preventative (Brown rust):

Wheat leaf segments cultivated variety (cv) Kanzler were placed on agar in 24-well plates and sprayed with formulated test compound diluted in water at an application rate of 200ppm. The leaf disks were inoculated with a spore suspension of the fungus 1 day after application. The inoculated leaf segments were incubated at 19°C and 75% relative humidity under a light regime of 12/12 h (light/dark) in a climate cabinet and the activity of a compound was assessed as percent disease control compared to untreated when an appropriate level of disease damage appears in untreated check leaf segments (7 - 9 days after application). The following compounds gave at least 80% control of *Puccinia recondita* f. sp. *tritici*: P.01, P.02

### Puccinia recondita f. sp. tritici / wheat / leaf disc curative (Brown rust):

Wheat leaf segments cv Kanzler were placed on agar in 24-well plates. The leaf segments were inoculated with a spore suspension of the fungus. The plates were stored in darkness at 19°C and 75% relative humidity. The formulated test compound diluted in water was applied at an application rate of 200ppm 1 day after inoculation. The leaf segments were incubated at 19°C and 75% relative humidity under a light regime of 12/12 h (light/dark) in a climate cabinet and the activity of a compound was assessed as percent disease control compared to untreated when an appropriate level of disease damage appears in untreated check leaf segments (6 - 8 days after application). The following compounds gave at least 80% control of *Puccinia recondita f. sp. tritici*: P.01, P.02

### Phaeosphaeria nodorum (Septorla nodorum) / wheat / leaf disc preventative (Glume blotch):

Wheat leaf segments cv Kanzler were placed on agar in a 24-well plate and sprayed with formulated test compound diluted in water at an application rate of 200ppm. The leaf disks were inoculated with a spore suspension of the fungus 2 days after application. The inoculated test leaf disks were incubated at 20°C and 75% relative humidity under a light regime of 12/12 h (light/dark) in a climate cabinet and the activity of a compound was assessed as percent disease control compared to untreated when an appropriate level of disease damage appears in untreated check leaf disks (5 - 7 days after application). The following compounds gave at least 80% control of *Phaeosphaeria nodorum*: P.01, P.02

### Pyrenophora teres / barley / leaf disc preventative (Net blotch):

Barley leaf segments cv Hasso were placed on agar in a 24-well plate and sprayed with formulated test compound diluted in water at an application rate of 200ppm. The leaf segments were inoculated with a spore suspension of the fungus two days after application of the test solution. The inoculated leaf segments were incubated at 20°C and 65% relative humidity under a light regime of 12/12 h (light/dark) in a climate cabinet and the activity of a compound was assessed as disease control compared to untreated when an appropriate level of disease damage appears in untreated check leaf segments (5 - 7 days after application). The following compounds gave at least 80% control of *Pyrenophora teres*: P.01, P.02

### Botryotinia fuckeliana (Botrytis cinerea) / liquid culture (Gray mould):

Conidia of the fungus from cryogenic storage were directly mixed into nutrient broth (Vogels broth). After placing a DMSO solution of test compound into a 96-well microtiter plate at an application rate of 200ppm, the nutrient broth containing the fungal spores was added. The test plates were incubated at 24°C and the inhibition of growth was determined photometrically 3-4 days after application. The following compounds gave at least 80% control of *Botryotinia fuckeliana*: P.01, P.02

### Glomerella lagenarium (Colletotrichum lagenarium) / liquid culture (Anthracnose) :

Conidia of the fungus from cryogenic storage were directly mixed into nutrient broth (PDB potato dextrose broth). After placing a DMSO solution of test compound into a 96-well microtiter plate at an application rate of 200ppm, the nutrient broth containing the fungal spores was added. The test plates were incubated at 24°C and the inhibition of growth was measured photometrically 3-4 days after application. The following compounds gave at least 80% control of *Glomerella lagenarium*: P.01, P.02

### Mycosphaerella arachidis (Cercospora arachidicola) / liquid culture (early leaf spot):

Conidia of the fungus from cryogenic storage were directly mixed into nutrient broth (PDB potato dextrose broth). After placing a DMSO solution of test compound into a 96-well microtiter plate at an application rate of 200ppm, the nutrient broth containing the fungal spores was added. The test plates were incubated at 24°C and the inhibition of growth was determined photometrically 4-5 days after application. The following compounds gave at least 80% control of *Mycosphaerella arachidis*: P.01, P.02

### Mycosphaerella graminicola (Septoria tritici) / liquid culture (Septoria blotch):

Conidia of the fungus from cryogenic storage were directly mixed into nutrient broth (PDB potato dextrose broth). After placing a DMSO solution of test compound into a 96-well microtiter plate at an application rate of 200ppm, the nutrient broth containing the fungal spores was added. The test plates were incubated at 24°C and the inhibition of growth was determined photometrically 4-5 days after application. The following compounds gave at least 80% control of *Mycosphaerella graminicola*: P.01, P.02

### Gaeumannomyces graminis / liquid culture (Take-all of cereals):

Mycelial fragments of the fungus from cryogenic storage were directly mixed into nutrient broth (PDB potato dextrose broth). After placing a DMSO solution of test compound into a 96-well microtiter plate at an application rate of 200ppm, the nutrient broth Cp.33, containing the fungal spores is added. The test plates were incubated at 24°C and the inhibition of growth was determined photometrically 4-5 days after application. The following compounds gave at least 80% control of *Gaeumannomyces graminis*: P.01

### Thanatephorus cucumeris (Rhizoctonia solani) / liquid culture (foot rot, damping-off):

Mycelia fragments of a newly grown liquid culture of the fungus were directly mixed into nutrient broth (PDB potato dextrose broth). After placing a DMSO solution of the test compounds into a 96-well microtiter plate at an application rate of 200ppm, the nutrient broth containing the fungal material was added. The test plates were incubated at 24°C and the inhibition of growth was determined photometrically 3-4 days after application. The following compounds gave at least 80% control of *Thanatephorus cucumeris*: P.01, P.02

### Monographella nivalis (Microdochium nivale) / liquid culture (foot rot cereals):

Conidia of the fungus from cryogenic storage were directly mixed into nutrient broth (PDB potato dextrose broth). After placing a DMSO solution of test compound into a 96-well microtiter plate at an application rate of 200ppm, the nutrient broth containing the fungal spores was added. The test plates were incubated at 24°C and the inhibition of growth was determined photometrically 4-5 days after application. The following compounds gave at least 80% control of *Monographella nivalis*: P.01, P.02

### Blumeria graminis f. sp. tritici (Erysiphe graminis f. sp. tritici) / wheat / leaf disc preventative (Powdery mildew on wheat):

Wheat leaf segments cv. Kanzler were placed on agar in a 24-well plate and sprayed with the formulated test compound diluted in water at an application rate of 200ppm. The leaf disks were inoculated by shaking powdery mildew infected plants above the test plates 1 day after application. The inoculated leaf disks were incubated at 20°C and 60% relative humidity under a light regime of 24 h darkness followed by 12h/12h (dark/light) in a climate chamber and the activity of a compound was assessed as percent disease control compared to untreated when an appropriate level of disease damage appears on untreated check leaf segments (6 - 8 days after application). The following compounds gave at least 80% control of *Blumeria graminis*: P.01, P.02

### Alternaria solani / tomato / leaf disc (early blight)

Tomato leaf disks cultivated variety (cv.) Baby were placed on agar in multiwell plates (24-well format) and sprayed with the formulated test compound diluted in water at an application rate of 200ppm. The leaf disks were inoculated with a spore suspension of the fungus 2 days after application. The inoculated leaf disks were incubated at 23°C/21°C (day/night) and 80% relative humidity under a light regime of 12/12 h (light/dark) in a climate cabinet and the activity of a compound was assessed as percent disease control compared to untreated when an appropriate level of disease damage appears on untreated check disk leaf disks (5 - 7 days after application). The following compounds gave at least 80% control of *Alternaria solani*: P.01

### Pythium ultimum / liquid culture (seedling damping off)

Mycelia fragments and oospores of a newly grown liquid culture of the fungus were directly mixed into nutrient broth (potato dextrose broth). After placing a DMSO solution of test compound into a 96-well format microtiter plate at an application rate of 200ppm, the nutrient broth containing the fungal mycelia/spore mixture was added. The test plates were incubated at 24°C and the inhibition of growth was determined photometrically 2-3 days after application. The following compounds gave at least 80% control of *Pythium ultimum*: P.01, P.02

### Magnaporthe grisea (Pyricularia oryzae)/rice/leaf disc preventative (Rice Blast):

Rice leaf segments cv. Ballila were placed on agar in multiwell plate (24-well format) and sprayed with the formulated test compound diluted in water at an application rate of 200ppm. The leaf segments were inoculated with a spore suspension of the fungus 2 days after application. The inoculated leaf segments were incubated at 22°C and 80% rh under a light regime of 24 h darkness followed by 12/12 h (light/dark) in a climate cabinet and the activity of a compound was assessed as percent disease control compared to untreated when an appropriate level of disease damage appears in untreated check leaf segments (5 - 7 days after application). The following compounds gave at least 80% control of *Magnaporthe grisea*: P.01, P.02

## Claims

1. A compound of formula (I) 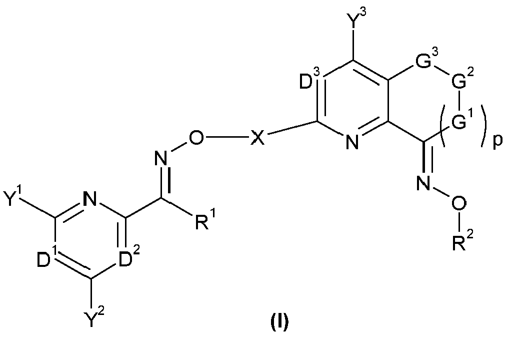 wherein
D¹ represents N or C-Y⁴;
D² represents N or C-Y⁵;
D³ represents N or C-Y⁶;
wherein both D¹ and D² cannot be N;
Y¹, Y², Y³, Y⁴, Y⁵ and Y⁶ independently of one another represent hydrogen, halogen, CN, NO₂, C₁-C₈ alkyl, C₁-C₄-alkoxy-C₁-C₄-alkyl, C₁-C₄-alkoxy-C₁-C₄-alkoxy-C₁-C₄-alkyl, C₃-C₈ cycloalkyl, C₂-C₈ alkenyl, C₂-C₈ alkynyl, phenyl, pyridyl, pyrimidinyl, OR³, COR⁴, SH, C₁-C₈ alkylthio, C₁-C₈ alkylsulphinyl, C₁-C₈ alkylsulphonyl, N(R⁵)₂, CO₂R³, O(CO)R⁴, CON(R⁵)₂, NR⁵COR⁴ or CR⁴N-OR³, wherein the alkyl, cycloalkyl, alkenyl, alkynyl, phenyl, pyrimidinyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from O, S, N and N(R⁵), providing that the heterocycle does not contain adjacent oxygen atoms, adjacent sulphur atoms, or adjacent sulphur and oxygen atoms, and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
R¹ represents hydrogen, halogen, CN, OH, SH, C₁-C₈ alkylthio, C₁-C₈ alkylsulphinyl, C₁-C₈ alkylsulphonyl, NH₂, C₁-C₁₀ alkyl, C₃-C₈ cycloalkyl, C₂-C₈ alkenyl, C₂-C₈ alkynyl, (R³O)carbonyl(C₁-C₄-alkyl), phenyl or pyridyl, wherein the alkyl, cycloalkyl, alkenyl, alkynyl, phenyl and pyridyl are optionally substituted by one or more groups independently selected from halogen, CN, NH₂, NO₂, OH, C₁-C₄ alkyl, C₁-C₄ haloalkyl, C₁-C₄ alkoxy, C₁-C₄ haloalkoxy, C₃-C₆ cycloalkyl and a 5- or 6-membered heterocycle containing one to three heteroatoms independently selected from O, S and N, providing that the heterocycle does not contain adjacent oxygen atoms, adjacent sulphur atoms, or adjacent sulphur and oxygen atoms;or if D² is C-Y⁵ then R¹ and Y⁵ together may be - (G⁴)_{q}-G⁵-G⁶-;
R² represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
each R³ independently of one another represents hydrogen, C₁-C₈ alkyl, C₃-C₈ cycloalkyl, C₃-C₈ alkenyl, C₃-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄-haloalkyl, C₁-C₄ alkoxy, C₁-C₄ haloalkoxy and C₁-C₄-alkoxy-C₁-C₄-alkyl;
each R⁴ independently of one another represents hydrogen, C₁-C₈ alkyl, C₃-C₈ cycloalkyl, C₂-C₈ alkenyl, C₂-C₈ alkynyl, benzyl, phenyl or pyridyl, wherein the alkyl, cycloalkyl, alkenyl, alkynyl, phenyl, benzyl and pyridyl are optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
each R⁵ independently of one another represents hydrogen, OH, C₁-C₈ alkyl, C₁-C₈ alkoxy, C₁-C₈-alkoxy-C₁-C₄-alkyl, C₃-C₈ alkenyl, C₃-C₈ alkynyl, COR⁴ or phenyl wherein the alkyl, alkoxy, alkenyl, alkynyl and phenyl are optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5, B-6, B-7 or B-8: 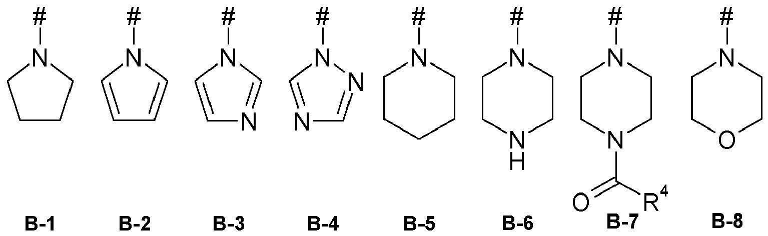 wherein the cycle formed is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
X represents X-2, X-3, X-4 or X-5: 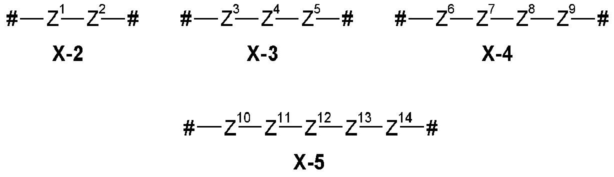 Z¹, Z², Z³, Z⁵, Z⁶, Z⁷, Z⁸, Z⁹, Z¹⁰, Z¹¹, Z¹³ and Z¹⁴ independently of one another represent CR⁶R⁷, C=CR⁸R⁹ or C=O;
Z⁴ and Z¹² independently of one another represent C=CR⁸R⁹, CR¹⁰R¹¹, SiR¹²R¹³ or C=O;
or in each case two adjacent radicals Z⁴ and Z⁵ or Z⁷ and Z⁸ or Z⁸ and Z⁹ or Z¹¹ and Z¹² or Z¹² and Z¹³ or Z¹³ and Z¹⁴ may together represent a group selected from CR¹⁴=CR¹⁵- and -C≡C-, wherein X-4 or X-5 may not contain more than one such group;
each R⁶ and R⁷ independently of one another represent hydrogen, halogen, OH, C₁-C₄ alkyl, C₁-C₄ haloalkyl, C₃-C₆ cycloalkyl, C₃-C₆ halocycloalkyl, C₁-C₄ alkylthio, C₁-C₄ alkylsulphinyl, C₁-C₄ alkylsulphonyl, phenyl or CN, wherein the phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R⁶ and R⁷ together with the carbon atom to which they are attached may form a C₃-C₆ cycloalkyl group or a C₃-C₆ halocycloalkyl group;
each R⁸ and R⁹ independently of one another represent hydrogen, halogen, C₁-C₄ alkyl or C₁-C₄ haloalkyl;
each R¹⁰, R¹¹, R¹² and R¹³ independently of one another represent hydrogen, halogen, OH, C₁―C₄ alkyl, C₁―C₄ haloalkyl, C₃-C₆ cycloalkyl, C₃-C₆ halocycloalkyl, phenyl or CN, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R¹⁰ and R¹¹ together with the carbon atom to which they are attached may form a C₃-C₆ cycloalkyl group or a C₃-C₆ halocycloalkyl group;
each R¹⁴ and R¹⁵ independently of one another represent hydrogen, halogen, CN, OH, C₁-C₄ alkyl, C₁-C₄ haloalkyl or phenyl, wherein the phenyl is optionally substituted by one or more groups independently selected from halogen, CN, C₁-C₄ alkyl, C₁-C₄ haloalkyl, C₁-C₄ alkoxy and C₁-C₄ haloalkoxy;
or R¹⁴ and R¹⁵ together with the carbon atom to which they are attached may form a C₃-C₆ cycloalkenyl group or a C₃-C₆ halocycloalkenyl group;
wherein the groupings X-2, X-3, X-4 and X-5 may contain at most one ring which contains either only one of the radicals Z¹ to Z¹⁴ or two radicals Z¹ to Z¹⁴ or three radicals Z¹ to Z¹⁴ or four radicals Z¹ to Z¹⁴ as ring members; and wherein radicals Z¹, Z², Z³, Z⁵, Z⁶, Z⁹, Z¹⁰ and Z¹⁴ are not substituted by OH;
G¹, G², G⁴ and G⁵ independently of one another represent -C(R¹⁶R¹⁷)-;
G³ and G⁶ independently of one another represent -C(R¹⁶R¹⁷)-, O, N(R¹⁸) or S;
or G¹ and G², or G² and G³, or G¹ and G¹, or G⁴ and G⁵, or G⁵ and G⁶, or G⁴ and G⁴ together represent -CR¹⁶=CR¹⁷-;
each R¹⁶ and R¹⁷ independently of one another represent hydrogen, halogen, C₁-C₄ alkyl, C₁-C₄ haloalkyl, C₁-C₄ alkoxy or C₁-C₄ haloalkoxy;
R¹⁸ represents hydrogen, OH, C₁-C₄ alkyl, C₁-C₄ alkoxy, C₃-C₆ cycloalkyl, C₁-C₈ alkylcarbonyl or C₁-C₈ haloalkylcarbonyl; and
p and q are each independently 1 or 2;
or a salt or an N-oxide thereof.

2. A compound according to claim 1, wherein
Y¹, Y², Y³, Y⁴, Y⁵ and Y⁶ independently of one represent hydrogen, halogen, OH, CN, C₁-C₈ alkyl, C₁-C₈ haloalkyl, C₁-C₈ alkoxy, C₁-C₈ haloalkoxy, C₁-C₈ alkylthio, C₃-C₈ cycloalkyl, phenyl, pyridyl, N(R⁵)₂, or NR⁵COR⁴, wherein phenyl and pyridyl are optionally substituted by one or more groups independently selected from halogen, CN, C₁-C₄ alkyl, C₁-C₄ haloalkyl, C₁-C₄ alkoxy and C₁-C₄ haloalkoxy;
or independently Y¹ and Y⁴, Y² and Y⁴, Y² and Y⁵, and Y³ and Y⁶ together with the fragment of the ring to which they are attached may form a partially or fully unsaturated 5- to 7-membered carbocyclic ring or a 5- to 7-membered heterocyclic ring containing one to three heteroatoms independently selected from N and N(R⁵)₂ and wherein the ring formed by Y¹ and Y⁴, Y² and Y⁴, Y² and Y⁵, and Y³ and Y⁶ is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy; R¹ represents hydrogen, C₁-C₈ alkyl, C₃-C₈ cycloalkyl, phenyl or pyridyl, wherein the alkyl, cycloalkyl, phenyl and pyridyl are optionally substituted by one or more groups independently selected from halogen, CN, C₁-C₄ alkyl, C₁-C₄ haloalkyl, OH, C₁-C₄ alkoxy, C₁-C₄ haloalkoxy and C₃-C₆ cycloalkyl;
R² represents C₁-C₄ alkyl, C₁-C₄ haloalkyl, C₃-C₈ cycloalkyl, C₃-C₈ halocycloalkyl, C₃-C₈ alkenyl, C₃-C₈ haloalkenyl, C₃-C₈ alkynyl, C₃-C₈ haloalkynyl, benzyl or pyridyl, wherein the, benzyl and pyridyl are optionally substituted by one or more groups, e.g. one to five groups, independently selected from halogen, CN, C₁-C₄ alkyl, C₁-C₄ haloalkyl, C₁-C₄ alkoxy and C₁-C₄ haloalkoxy;
each R⁴ independently represents C₁-C₈ alkyl or C₁-C₈ haloalkyl;
each R⁵ independently of one another represents hydrogen, C₁-C₈ alkyl, COR⁴ or phenyl, wherein the phenyl is optionally substituted by one or more group independently selected from halogen, CN, NH₂, NO₂, OH, C₁-C₄ alkyl, C₁-C₄ haloalkyl, C₁-C₄ alkoxy, C₁-C₄ haloalkoxy, C₁-C₄ alkylthio, C₁-C₄ alkylsulphinyl and C₁-C₄ alkylsulphonyl;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5;
wherein the cycle formed is optionally substituted by one or more groups independently selected from halogen, methyl and halomethyl;
X represents X-3;
Z³ and Z⁵ independently of one another represent methylene or halomethylene;
Z⁴ represent C=CR⁸R⁹ or CR¹⁰R¹¹;
each R⁸ and R⁹ independently of one another represent hydrogen, halogen, methyl or halomethyl;
each R¹⁰ or R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴, G⁵ and G⁶ independently of one another represent -C(R¹⁶R¹⁷)-;
p and q are each independently 1 or 2.

3. A compound according to claim 1, wherein
Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₃-C₆ cycloalkyl, C₁-C₄ alkylthio, N(R⁵)₂, NR⁵COR⁴ or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
R¹ represents hydrogen, C₁-C₄ alkyl, phenyl or pyridyl, wherein alkyl is optionally substituted by one or more groups independently selected from halogen, OH, C₁-C₄ alkoxy and C₁-C₄ haloalkoxy, and wherein phenyl and pyridyl are optionally substituted by one or more groups independently selected from halogen, CN, C₁-C₄ alkyl, C₁-C₄ haloalkyl, OH, C₁-C₄ alkoxy, C₁-C₄ haloalkoxy and C₃-C₆ cycloalkyl;
R² represents C₁-C₄ alkyl, C₃-C₆ alkenyl, C₃-C₆ alkynyl or benzyl, wherein the alkyl, alkenyl, alkynyl and benzyl are optionally substituted by one or more groups, e.g. one to five groups, independently selected from halogen, CN, C₁-C₄ alkoxy and C₁-C₄ haloalkoxy;
each R⁴ independently represents C₁-C₄ alkyl or C₁-C₄ haloalkyl;
each R⁵ independently of one another represents hydrogen, C₁-C₈ alkyl or phenyl, wherein phenyl is optionally substituted by one or more groups independently selected from halogen, CN, NH₂, NO₂, OH, C₁-C₄ alkyl, C₁-C₄ haloalkyl, C₁-C₄ alkoxy and C₁-C₄ haloalkoxy;
wherein when two radicals R⁵ are attached to the same nitrogen atom, these radicals can be identical or different;
wherein when two radicals R⁵ are attached to the same nitrogen atom, both of these radicals cannot be OH, C₁-C₄ alkoxy or C₁-C₄ haloalkoxy;
and wherein when two radicals R⁵ are attached to the same nitrogen atom, these two radicals together with the nitrogen atom to which they are attached may form a cycle B-1, B-2, B-3, B-4, B-5 wherein the cycle formed is optionally substituted by one or more independently selected from halogen, methyl and halomethyl;
X represents X-3;
Z³ and Z⁵ represent methylene;
Z⁴ represents C=CR⁸R⁹ or CR¹⁰R¹¹;
each R⁸ and R⁹ independently of one another represent hydrogen, halogen, methyl or halomethyl;
each R¹⁰ and R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴, G⁵ and G⁶ represent methylene;
p and q are each independently 1 or 2.

4. A compound according to claim 1, wherein
Y¹, Y², Y³, Y⁴, Y⁵ and Y⁶ independently of one another represent hydrogen, CN, OH, halogen, C₁-C₄ alkyl, C₁-C₄ haloalkyl, C₁-C₄ alkoxy, C₁-C₄ haloalkoxy, C₃-C₆ cycloalkyl, C₁-C₄ alkylthio, N(R⁵)₂, NR⁵COR⁴ or phenyl, wherein phenyl is optionally substituted by one or more groups independently selected selected from halogen, methyl, CN, methoxy, halomethyl and halomethoxy;
R¹ represents hydrogen, C₁-C₄ alkyl, phenyl or pyridin-2-yl wherein phenyl and pyridin-2-yl are optionally substituted by one or more groups independently selected from halogen, CN, methyl, halomethyl, methoxy and halomethoxy;
R² represents hydrogen, C₁-C₈ alkyl, or C₁-C₈ haloalkyl;
each R⁴ independently represents C₁-C₄ alkyl or C₁-C₄ haloalkyl;
each R⁵ independently of one another represents hydrogen, C₁-C₈ alkyl or phenyl, wherein phenyl is optionally substituted by one or more groups independently selected from halogen, CN, C₁-C₄ alkyl, C₁-C₄ haloalkyl, C₁-C₄ alkoxy and C₁-C₄ haloalkoxy;
X represents X-3;
Z³ and Z⁵ represent methylene;
Z⁴ represents C=CR⁸R⁹ or CR¹⁰R¹¹;
each R⁸ and R⁹ independently of one another represent hydrogen, halogen, methyl or halomethyl;
each R¹⁰ and R¹¹ independently of one another represent hydrogen, halogen, OH, C₁-C₄ alkyl, C₁-C₄ haloalkyl, phenyl or CN, wherein the phenyl is optionally substituted by one or more groups independently selected from halogen, CN, methyl, halomethyl, methoxy or halomethoxy;
or R¹⁰ and R¹¹ together with the carbon atom to which they are attached may form a C₃-C₆ cycloalkyl group or a C₃-C₆ halocycloalkyl group;
G¹, G², G³, G⁴, G⁵ and G⁶ represent methylene;
p and q are each independently 1 or 2.

5. A compound according to any one of the preceding claims, wherein G¹, G² and G³ represent methylene.

6. A compound according to any one of the preceding claims, wherein p is 1.

7. A compound according to any one of claims 1-6 wherein R¹ and Y⁵ together represent - CH₂-CH₂-CH₂-.

8. A compound according to any one of claims 1-6 wherein R¹ represents C₁-C₄ alkyl.

9. A compound according to any one of claims 1-8 wherein p is 1 and ―G¹-G²-G³-represents ―CH₂-CH₂-CH₂-.

10. A compound of formula (IV) 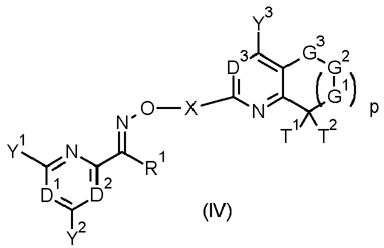 wherein D¹, D², D³, p, G¹, G², G³, X, Y¹, Y², Y³ and R¹ are as defined herein for compounds of formula (I) in any one of claims 1-9;
T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl;
or a salt or N-oxide thereof, or;
a compound of formula (VII) 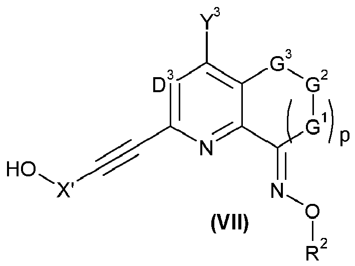 wherein Xʹ represents Xʹ-1, Xʹ-2 or Xʹ-3 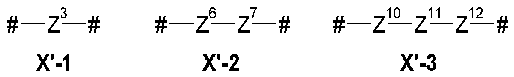 wherein Z³, Z⁴, Z⁶, Z⁷, Z¹⁰, Z¹¹ and Z¹² are as defined herein for a compound of formula (I) in any one of claims 1-9;
D³, Y³, R², G¹, G², G³ and p are as defined herein for compounds of formula (I) in any one of claims 1-9;
or a salt or N-oxide thereof, or;
a compound of formula (XI) 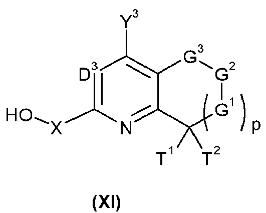 wherein T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl; X, D³, Y³, G¹, G², G³ and p are as defined herein for compounds of formula (I) in any one of claims 1-9;
or a salt or N-oxide thereof, or;
a compound of formula (XII) 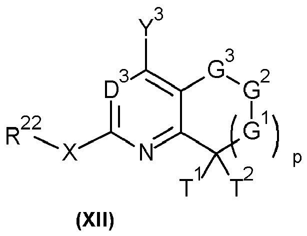 wherein R²² is a halogen or a sulfonic acid ester group;
T¹ and T² are C₁-C₈ alkoxy, or T¹ and T² together with the carbon they are attached to form a carbonyl group or an acetal or ketal function of the form C(O-C₁-C₆-alkylidene-O) whereby the alkylidene fragment may optionally be mono- to tetra-substituted by C₁-C₆ alkyl;
X, D³, Y³, G¹, G², G³ and p are as defined herein for compounds of formula (I) in any one of claims 1-9;
or a salt or N-oxide thereof, or;
a compound of formula (XVI) 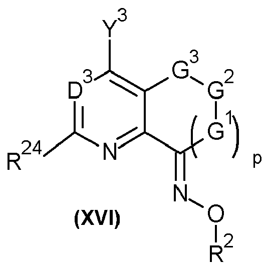 wherein R²⁴ is Cl, Br, I or a sulfonate and R², D³, Y³, G¹, G², G³ and p are as defined herein for compounds of formula (I) in any one of claims 1-9;
or a salt or N-oxide thereof.

11. A fungicidal composition comprising a fungicidally effective amount of a compound of formula (I) as defined in any one of claims 1 to 9, optionally comprising an additional active ingredient.

12. A method of controlling phytopathogenic diseases on useful plants or on propagation material thereof, which comprises applying to the useful plants, the locus thereof or propagation material thereof a fungicidally effective amount of a compound of formula (I) as defined in any one of claims 1 to 9.
